# EUROPEAN PATENT SPECIFICATION

(11) **EP 3 946 435 B1**
(45) Date of publication and mention of the grant of the patent: **27.12.2023**
(21) Application number: 20712382.9
(22) Date of filing: 25.03.2020
(51) Int. Cl.: A61K 39/00, C12N 5/0783, C12N 5/10, A61K 35/17, A61P 35/02, A61P 37/06

(54) **ENHANCEMENT OF CYTOLYTIC T-CELL ACTIVITY BY INHIBITING EBAG9**
ERWEITERUNG DER WIRKUNG ZYTOLYTISCHER T-ZELLEN DURCH HEMMUNG VON EBAG9
AMÉLIORATION DE L'ACTIVITÉ DES LYMPHOCYTES T CYTOLYTIQUES PAR INHIBITION EBAG9

(30) Priority: 25.03.2019 EP 19164822
(43) Date of publication of application: 09.02.2022
(73) Proprietor: Max-Delbrück-Centrum für Molekulare Medizin in der Helmholtz-Gemeinschaft, 13125 Berlin (DE)
(72) Inventor: REHM, Armin, 14057 Berlin (DE); WIRGES, Anthea, 10367 Berlin (DE); BUNSE, Mario, 10961 Berlin (DE); UCKERT, Wolfgang, 13125 Berlin (DE)
(74) Representative: Hertin und Partner Rechts- und Patentanwälte PartG mbB
(86) International application number: PCT/EP2020/058355
(87) International publication number: WO 2020/193628

(56) References cited:
- US-A1- 2017 283 475
- RÜDER C ET AL: "The tumor-associated antigen EBAG9 negatively regulates the cytolytic capacity of mouse CD8+ T cells", JOURNAL OF CLINICAL INVESTIGATION, vol. 119, no. 8, August 2009 (2009-08), pages 2184-2203, XP055625090, ISSN: 0021-9738, DOI: 10.1172/JCI37760 cited in the application
- MIYAZAKI T ET AL: "EBAG9 modulates host immune defense against tumor formation and metastasis by regulating cytotoxic activity of T lymphocytes", ONCOGENESIS, vol. 3, no. 11, E126, 3 November 2014 (2014-11-03), XP055625091, DOI: 10.1038/oncsis.2014.40 cited in the application
- HAN Y ET AL: "Knockdown of RCAS1 expression by RNA interference recovers T cell growth and proliferation", CANCER LETTERS, vol. 257, no. 2, 11 October 2007 (2007-10-11), pages 182-190, XP022294902, ISSN: 0304-3835, DOI: 10.1016/J.CANLET.2007.07.016
- SONODA K ET AL: "Biologic significance of receptor-binding cancer antigen expressed on SiSo cells (RCAS1) as a pivotal regulator of tumor growth through angiogenesis in human uterine cancer", CANCER, vol. 110, no. 9, November 2007 (2007-11), pages 1979-1990, XP055625168, ISSN: 0008-543X, DOI: 10.1002/cncr.23015
- TSOUKALAS N G ET AL: "Clinical significance of RCAS1 and CD3 expression in non-small celllung cancers in immunotherapy era", ANNALS OF ONCOLOGY, vol. 29, no. Suppl. 8, 176P, October 2018 (2018-10), page viii55, XP055625133, 43rd Congress of European Society for Medical Oncology; Munich, 17-23 October 2018 DOI: 10.1093/annonc/mdy269
- BLUHM J ET AL: "CAR T Cells with Enhanced Sensitivity to B Cell Maturation Antigen for the Targeting of B Cell Non-Hodgkin's Lymphoma and Multiple Myeloma", MOLECULAR THERAPY, vol. 26, no. 8, August 2018 (2018-08), pages 1906-1920, XP055625080, ISSN: 1525-0016, DOI: 10.1016/j.ymthe.2018.06.012
- CLAUSS J ET AL: "Efficient Non-Viral T-Cell Engineering by Sleeping Beauty Minicircles Diminishing DNA Toxicity and miRNAs Silencing the Endogenous T-Cell Receptors", HUMAN GENE THERAPY, vol. 29, no. 5, May 2018 (2018-05), pages 569-584, XP055625083, ISSN: 1043-0342, DOI: 10.1089/hum.2017.136
- BUNSE M ET AL: "RNAi-mediated TCR Knockdown Prevents Autoimmunity in Mice Caused by Mixed TCR Dimers Following TCR Gene Transfer", MOLECULAR THERAPY, vol. 22, no. 11, 22 July 2014 (2014-07-22) , pages 1983-1991, XP055295400, ISSN: 1525-0016, DOI: 10.1038/mt.2014.142
- ELLEBRECHT C T ET AL: "Reengineering chimeric antigen receptor T cells for targeted therapy of autoimmune disease", SCIENCE, vol. 353, no. 6295, 8 July 2016 (2016-07-08), pages 179-184, XP055434542, ISSN: 0036-8075, DOI: 10.1126/science.aaf6756 cited in the application
- TOWNSEND M H ET AL: "The expansion of targetable biomarkers for CAR T cell therapy", JOURNAL OF EXPERIMENTAL & CLINICAL CANCER RESEARCH, vol. 37, no. 1, 163, 21 July 2018 (2018-07-21), XP055541235, DOI: 10.1186/s13046-018-0817-0 cited in the application
- HARTMANN J ET AL: "Clinical development of CAR T cells-challenges and opportunities in translating innovative treatment concepts", EMBO MOLECULAR MEDICINE, vol. 9, no. 9, September 2017 (2017-09), pages 1183-1197, XP055487998, ISSN: 1757-4684, DOI: 10.15252/emmm.201607485 cited in the application

## Description

The invention relates to the field of cellular therapeutic agents, in particular to means for enhancing the cytotoxic activity of therapeutic T cells suitable for treating cancer.

The invention relates to a genetically modified cytotoxic T cell comprising one or more exogenous nucleic acid molecules encoding a transgenic antigen-targeting construct, wherein in said cells estrogen receptor-binding fragment-associated antigen 9 (EBAG9) activity is inhibited. The invention relates further to the modified cytotoxic T cell in which the antigen-targeting construct is a chimeric antigen receptor (CAR) or T cell receptor (TCR). The invention further relates to the modified T cell for use as a medicament in the treatment of a proliferative disease, and corresponding methods of treatment, in particular for the treatment of hematologic malignancies. The invention relates further to a pharmaceutical composition comprising the modified T cell, a nucleic acid vector encoding the antigen-targeting construct and means for inhibiting EBAG9, preferably using RNA interference, and to an in vitro method for increasing the cytolytic activity of a cytotoxic T cell.

### BACKGROUND OF THE INVENTION

Hematologic neoplasia are heterogenous and can be distinguished by an aggressive and indolent course. The standard of care is combined antibody/chemotherapy, often in combination with autologous stem cell transplantation, immunomodulatory drugs, irradiation, proteasome inhibitors, signaling pathway inhibitors, and for a very few patients allogeneic stem cell transplantation. Because in many B-NHL entities the median age at diagnosis is >66-72 years, comorbidities also exist that preclude intense and extended chemotherapies or even allogeneic bone marrow transplantations.

Inhibitors of BCR signaling in mature B cell lymphomas, foremost ibrutinib and others, have brought about a tremendous advance in remission rates. Despite initial high sensitivity to this class of kinase inhibitors, it is uncertain whether tumor eradication can be achieved, and secondly, several studies revealed that clonal lymphoma and leukemia evolution led to the occurrence of resistance to BTK inhibition. Thus, the rapid emergence of secondary resistances to targeted therapies urges to find a solution for tolerable salvage therapies, applicable to patients alter several lines of other chemotherapies and thus, with a reduced clinical performance (IPI score).

Adoptive chimeric antigen receptor (CAR)-T cell therapies targeted at the broadly expressed CD19 antigen on leukemia and lymphoma B cells has brought about substantial clinical efficacy. CAR-T approaches based on antigen specificity towards BCMA (WO2017/211900) and CXCR5 (WO2019/038368A1) as tumor associated antigens have also been described.

However, in anti-CD19 antibody or CAR-T cell therapies directed against B-NHL, resistances can occur due to antigen loss or downregulation. A recent study showed that upon anti-CD19 CAR-T cell therapy escape variants emerged that resulted from the selection for alternatively spliced CD19 isoforms and thus, loss of the cognate CD19 CAR epitope. Thus, other CAR-specificities emerge as alternative targets for immunotherapy of B-cell lymphomas besides existing therapeutic mAbs or CAR-T cell therapies. Likewise, for the BCMA antigen associated with multiple myeloma, a shedding from tumor cell membranes was noted, leading to substantial decrease in targetable structures for BCMA CARs.

Efficient adoptive T cell therapy (ATT) is dependent on the generation of high avidity, long-lived tumor antigen-specific CD8+ and/or CD4+ T cells. Functional avidity depends on the structural avidity of a TCR or a CAR for its cognate antigen, but also on a T cells' cytolytic efficiency, which is influenced by synthesis, transport and storage of effector cytokines and cytolytic molecules. In addition, adoptively transferred T cells, either equipped with a TCR or with a CAR, are prone to develop tolerance or a dysfunctional state in the presence of the tumor microenvironment. Thus, T cell functions to break this dysfunctional state or to prevent such a state are warranted.

The function of estrogen receptor-binding fragment-associated antigen 9 (EBAG9) has been assessed in T lymphocytes and been found to regulate cytotoxic activity. Rüder et al (J. Clin. Invest. 2009, 119:2184-2203) teach that EBAG9 negatively regulates the cytolytic capacity of mouse CD8+ T cells. Loss of EBAG9 led to an increase in CTL secretion of granzyme A. Furthermore, Miyazaki et al (Oncogenesis (2014) 3, e126) teach that EBAG9 regulates the cytotoxic activity of mouse T lymphocytes and therefore modulates tumor growth and metastasis by negatively regulating the adaptive immune response. Despite these findings, no suggestions or attempts have been made to incorporate EBAG9 inhibition in a human cytotoxic T cell comprising a transgenic antigen-targeting construct. The findings regarding EBAG9 have to date been considered from the perspective of modulating EBAG9 expression in cancer cells. According to Miyazaki et al, EBAG9 expression is elevated in cancer cells, which subsequently negatively regulates the endogenous host immune response. However, no suggestion has been made in the art to inhibit EBAG9 in therapeutic T cells with engineered antigen specificity for transfer to a subject in order to increase cytolytic capacity.

In light of the disadvantages above and the inherent difficulties in developing cellular therapeutics employing T cells that show high avidity and cytotoxic activity towards malignant cells, the field is in urgent need of novel means for improving the activity and ultimately therapeutic efficacy of cytolytic T cells, in order to overcome difficulties for example in tumor resistance or tumor antigen shedding.

### SUMMARY OF THE INVENTION

In light of the prior art the technical problem underlying the invention was the provision of alternative or improved means for enhancing the cytolytic activity of therapeutic T cells, in particular those with antigen-specific targeting constructs that direct the T cell to particular tumor targets. A further object of the invention was to provide means for improving CAR-T or TCR T cell therapies. Another object of the invention was to provide means for accelerating manufacturing of competent CAR T cells.

This problem is solved by the features of the independent claims. Preferred embodiments of the present invention are provided by the dependent claims. The present invention seeks to solve the problem above whilst avoiding the disadvantages of the prior art.

Therefore, the invention relates to genetically modified cytotoxic T cells comprising one or more exogenous nucleic acid molecules encoding a transgenic antigen-targeting construct, wherein in said cells estrogen receptor-binding fragment-associated antigen 9 (EBAG9) activity is inhibited.

The inventors have engineered modified T cells in which EBAG9 is inhibited to translate the stimulatory effect of EBAG9-deletion or knockdown on cytolytic effector molecule release into the therapeutic context of ATT.

As shown below, in preferred embodiments and the examples, the inventors combined the miRNA targeting of EBAG9 with various CAR constructs, foremost the BCMA CAR, the CD19 CAR and the CXCR5 CAR, as described in more detail herein. Retroviruses were generated, and human T cells were transduced. These T cells were endowed with a substantially improved cytolytic activity, as evidenced by an enhanced cytolytic killing in vitro. In vivo, xenotransplantation experiments into NSG mice using human multiple myeloma cells were performed, followed by the transplantation of low numbers of engineered CAR T cells.

The CAR T cells with a silenced EBAG9 expression were therefore endowed with a high cytolytic efficiency, visible in some examples as complete tumor eradication. The EBAG9 inhibition, preferably RNAi-mediated, leads therefore to surprising effects regarding the increase in efficacy afforded to CAR-T and similar approaches in cellular therapy. This increase represents a surprising effect that could not have been predicted by a skilled person.

The present invention, characterized by an inhibition of EBAG9 in CTLs expressing an antigen targeting construct, is further defined by the advantage of no increased risk of autoimmunity. Alternative cell biological approaches to strengthen T cells cytolytic efficiency are standard cell cultural procedures described in the prior art, including either endowing T cells with a defined maturation status towards effector or memory phenotypes. However, the down regulation of other roadblocks for T cell activation, for example loss of PD-1 or Cbl-b, is associated with substantial risk for autoimmunity. Surprisingly, autoimmunity has not been observed in EBAG9-KO mice, thus there is essentially no risk or a substantially lower risk for developing such a disease subsequent to ATT using the cells of the present invention.

As a further advantage of the invention, EBAG9 targets a posttranslational step in cellular processes and therefore interferes with a defined secretory route which is only effective in T cells, but not in other somatic cells. By selective targeting of EBAG9 using miRNA approaches *in vitro* of mature T cells, the risk profile is very low.

Furthermore, as an additional advantage, EBAG9 silencing is not an oncogenic driver, instead it endows CD8+ T cells - but not NK cells - with a heightened immunological competence. EBAG9 deficiency is also associated with increased antigen-specific memory formation, important for prevention of tumor relapse.

The biological principle of the EBAG9-inhibiting (preferably silencing) approach is novel and associated with unexpected advantages, as described above and in more detail below. For the first time, a posttranslational transport step is targeted in CTLs that helps to enhance the provision of cytolytic effector molecules.

This EBAG9 inhibition results in an enhanced cytolytic capacity and a higher efficiency in tumor eradication, without endangering an exhaustion or dysfunctional state. No obvious side effects have been observed in pre-clinical models, which is in clear contrast to other approaches targeted at signaling processes (PD-1, Cbl-b).

The invention therefore further allows for a more rapid generation of genetically engineered (preferably CD8+ and CD4+) T cells that are endowed with better anti-tumor activity in vitro and in vivo. In order to provide an effective dose of CAR T cells, one typically needs a minimum 12 days before the cells are ready for administration. This time frame can now be accelerated significantly when using EBAG9 inhibition, in some cases reducing preparation time below 10 days to generate an effective dose of CAR T cells. In other words, to achieve the same effect, one requires fewer CAR T cells, and therefore a shorter ex vivo and in vitro expansion phase is required. This will save significant costs in production of the cells and quicker treatments. Improvements are evident therefore at a manufacturing level, a biological level and at a treatment level, providing a suite of advantages that could not have been predicted from the prior art.

In vitro analysis by the inventors revealed further that the engineered T cells were not prone to develop a faster exhaustion stage, indicating that the application of EBAG9-engineered CAR T cells is a feasible strategy to improve ATT for several hematologic malignancies. In some embodiments, the modified CTLs of the invention are characterized by a comparatively slower exhaustion of cytolytic activity compared to unmodified control cells, such as CTLs in which EBAG9 has not been inhibited.

High affinity and high avidity enable CAR-T and TCR-T cells to recognize, be activated against, and kill tumor target cells with high and intermediate cognate antigen surface expression. However, CARs with an avidity-maturation have not yet been described.

The present invention therefore represents a breakthrough concept for CTLs activity improvement, not previously disclosed or suggested in the art. To the best knowledge of the inventors, there is currently no competing biological principle in use where the increase of ATT efficiency is achieved via an improvement in the secretory pathway in T cells.

Despite EBAG9 having a known negative regulatory function on cytolytic activity, no suggestions or attempts have been made in the prior art to incorporate EBAG9 inhibition in a cytotoxic T cell comprising a transgenic antigen-targeting construct. The prior art regarding EBAG9 shows that EBAG9 expression is elevated in cancer cells, which subsequently negatively regulates the endogenous host immune response. The inhibition of EBAG9 in therapeutic T cells, with engineered antigen specificity in order to increase cytolytic capacity, represents an entirely novel approach aimed at manipulating EBAG9 function directly in the cells in which enhanced cytolytic activity is required.

The invention therefore solves a different problem compared to the documents of the prior art regarding EBAG9 and cytolytic activity, namely the present invention seeks to improve CAR-T or TCR-based cell therapies, by not only enhancing cytolytic activity of the cells, but also by improving methods of manufacture for the cells by reducing the required number of cells for therapeutic application. The present invention therefore provides a unique solution to these problems, which could not have been derived from the art.

Furthermore, the combination of an antigen targeting construct and EBAG9 inhibition in cytotoxic T cells leads to a synergistic effect. By directing the cytolytic activity of T cells to the target cells of interest, the enhanced cytolytic activity due to EBAG9 inhibition can be exerted locally and effectively, leading to a cytolytic effect greater than expected and greater than the sum of effects obtained by incorporating either the antigen-targeting construct or the EBAG9 inhibition in isolation.

According to the present invention, EBAG9 inhibition can be obtained via a number of potential mechanisms and using various molecular or chemical tools. The embodiments disclosed herein regarding EBAG9 inhibition are therefore exemplary and not intended as limiting embodiments.

In some embodiments, the inhibition of EBAG9 is preferably determined in comparison to a control cytotoxic T cell, i.e. a cytotoxic T cell obtained from a comparable or the same source, in which EBAG9 production or activity has not been modified, for example by the means described herein.

In some embodiments, the inhibition of EBAG9 activity is associated with an increase in the release of cytolytic granules and/or granzyme-containing secretory lysosomes (preferably compared to a control cytotoxic T cell). Suitable assays are presented below, and are known to a skilled person, with which the release of cytolytic granules and/or granzyme-containing secretory lysosomes can be assessed in two comparative cell populations, without undue effort.

In some embodiments, the measurable effect of EBAG9 inhibition is the enhanced delivery of cytolytic granules. In some embodiments, the measurable effect of EBAG9 inhibition is an increased killing of target cells following antigen-specific CAR-mediated recognition. A skilled person is capable of determining these functional effects using routine and established assays.

One preferred assay is an "in vitro cytotoxicity assay", in which CTLs either with or without EBAG9 inhibition are compared for their efficacy in killing target cells in vitro. Examples for such in vitro assays are presented in more detail in the examples below. In vivo approaches for determining an EBAG9 inhibition based on CTL efficacy are also available and can be carried out by the skilled person without undue effort.

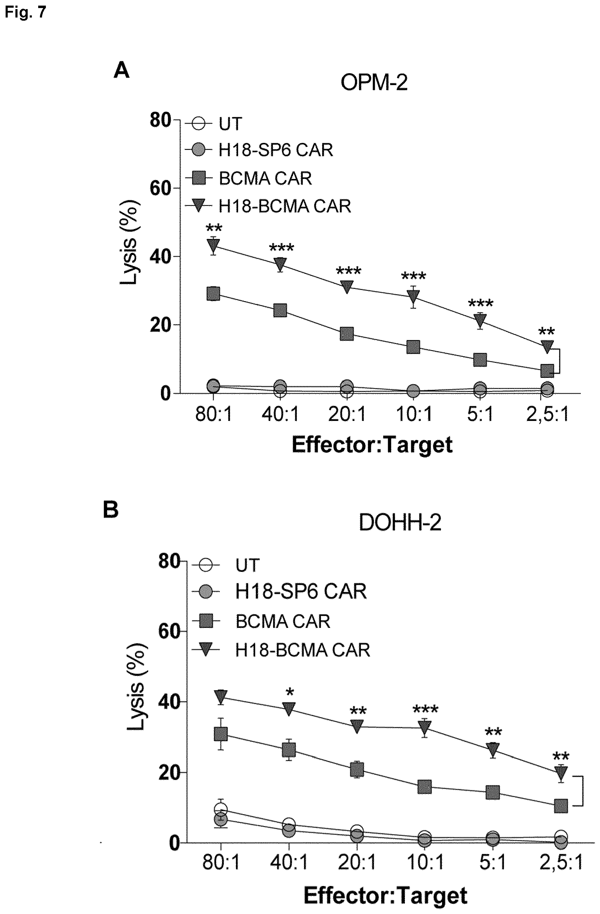

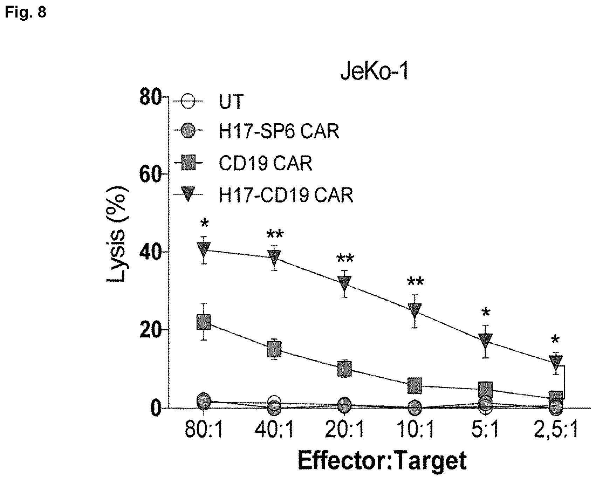

In one embodiment, the isolated T cells modified with a transgenic antigen targeting construct in which EBAG9 is inhibited, are characterized by an increase in cytotoxic activity of 5% or more, 10% or more, 20%, 30%, 40%, 50%, 60%, 70%, 80%, 90%, 100%, 150%, 200%, or 300% or more, when compared to cytolytic T cells without the EBAG9 inhibition. Functional assays for determining quantitatively, or semi-quantitatively the increase in cytolytic activity in the T cells in which EBAG9 is inhibited, are available to a skilled person, some examples of which are described herein. For example, the in vitro assays used to generate Figures 7 and 8 below, or as described in the examples, may be applied in order to determine an increase in cytolytic activity over "unmodified" T cells, i.e. T cells in which no specific modification has been carried out to reduce/inhibit EBAG9 function.

EBAG9 silencing is achieved in some embodiments by miRNA-based retroviruses used in T cell transduction. In several primary cell types of murine and human origin the inventors have shown silencing of EBAG9 transcripts and protein in the range of > 90%.

In some embodiments, the inhibition of EBAG9 in comparison to control CTLs achieves an inhibition of 10% or more, 20%, 30%, 40%, 50%, 60%, 70%, 80%, or 90% or more. In some cases, for example where EBAG9 is deleted or expression is disrupted due to e.g. genomic modifications, a 100% inhibition of EBAG9 may be achieved. In some embodiments, the invention employs miRNAs targeted at either murine or human EBAG9, combined in a retroviral expression construct with various TCR or CAR antigen-specificities.

In a preferred embodiment the genetically modified CTL as described herein is a CD4+ and/or CD8+ T cell, preferably the CTLs are a mixture of CD4+ and CD8+ T cells. These T cell populations, and preferably the composition comprising both CD4+ and CD8+ transformed cells, show particularly effective cytolytic activity against various hematological malignancies, preferably against those cells and/or the associated medical conditions described herein.

In a preferred embodiment the genetically modified CTLs are CD4+ and CD8+ T cells, preferably in a ratio of 1:10 to 10:1, more preferably in a ratio of 5:1 to 1:5, 2:1 to 1:2 or 1:1. Administration of modified CAR-T cells expressing a CAR at the ratios mentioned, preferably at a 1:1 CD4+/CD8+ ratio, lead to beneficial characteristics during treatment of the diseases mentioned herein, for example these ratios lead to improved therapeutic response and reduced toxicity.

In one embodiment, the genetically modified T cell as described herein is characterized in that the transgenic antigen-targeting construct is a chimeric antigen receptor (CAR).

In one embodiment, the genetically modified T cell as described herein is characterized in that the transgenic antigen-targeting construct is a T cell receptor (TCR).

The particular type or form of antigen-targeting construct is not intended as a limiting feature of the invention. The inventive concept is based on an EBAG9-inihibition mediated increase in cytolytic activity. The particular mode of delivering the CTL to any given target cell is therefore not a limiting aspect of the invention. The enhanced activity of the inventive CTL is therefore not dependent on the antigen-targeting construct, which is merely considered as a means for bringing the CTL into proximity with the target (e.g. tumor) cell. The CAR and TCR constructs described in more detail below represent therefore preferred embodiments in which the effect of EBAG9 inhibition can be effectively exploited.

In further embodiments, the CAR constructs to be employed can be exchanged easily, therefore allowing a modular composition of clinically applicable CARs. The antigen-specificity of the CAR is variable and not limiting to the present invention.

In one embodiment, the genetically modified T cell as described herein is characterized in that the inhibition of EBAG9 activity is obtained by knock-down of EBAG9, preferably by RNA interference of EBAG9 expression, such as by small interfering RNA (siRNA), short hairpin RNA (shRNA) and/or micro RNA (miRNA). Gene knockdown is a technique by which the expression of one or more genes are reduced. In some embodiments, the reduction in expression of EBAG9 can occur either through genetic modification or by treatment with a reagent, such as a short DNA or RNA, or other nucleic acid, oligonucleotides that have a sequence complementary to either the gene or an mRNA transcript of EBAG9.

RNA interreference approaches for EBAG9 inhibition (i.e. "silencing" or "knock-down") are preferred for a number of reasons due to their inherent advantages in biological systems. By not interfering with the genome structure or integrity, the RNAi technology has an excellent safety profile compared to manipulated genomes.

In some embodiments, the sequences employed to target EBAG9 are those according to:
H17 (SEQ ID NO 1; AAATAACCGAAACTGGGTGAT) or
H18 (SEQ ID NO 2; TTAAATAACCGAAACTGGGTG).

Other sequences to target alternative sites in EBAG9 are:
TAAATAACCGAAACTGGGTGA (SEQ ID NO 55);
TAGGAATGAGAATACTGTTGC (SEQ ID NO 56);
CTTAATTTCCGTCCTCTGCCA (SEQ ID NO 57);
TTTGGTCTCCACTTAATTTCC (SEQ ID NO 58);
TTCAACATCTGTCTGCTTAGG (SEQ ID NO 59);
AAGTCCACTCTTCAACATCTG (SEQ ID NO 60);
ATCCCAGGAAGTCCACTCTTC (SEQ ID NO 61);
TACTAGAGAAACCTGTGCTCC (SEQ ID NO 62).

In some embodiments, the invention relates to a nucleic acid molecule, either in isolated form or preferably in a vector for CTL transfection, and use of such a molecule for inhibiting EBAG9, selected from the group consisting of:
a) a nucleic acid molecule comprising a nucleotide sequence
   - which comprises a complementary or anti-sense sequence directed to a region of EBAG9 RNA, wherein said sequence is capable of interfering with EBAG9 RNA stability or function, or
   - which comprises or consists of a sequence according to SEQ ID NO 1, 2, or SEQ ID NO 55-62,
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising preferably a sequence identity to a nucleotide sequence according to a) or b) of at least 50%, 60%, 70%, 80%, 90% or 95%;
d) a nucleic acid molecule according to a nucleotide sequence of a) through c) which is modified by deletions, additions, substitutions, translocations, inversions and/or insertions and is functionally analogous/equivalent to a nucleotide sequence according to a) through c).

In some embodiments, the RNAi sequences may be designed to target one or more of the EBAG9 transcripts described herein, preferably according to SEQ ID NO 3-6.

A skilled person is capable of designing effective RNA targeting sequences based on the target sequence and common knowledge in the art. Software for such approaches is commonly available, with which a skilled person can design further sequences used as shRNAs to silence EBAG9. For example, the program BLOCK-iT RNAi from Thermo Fisher may be employed. Alternative software can also be identified and used.

In some embodiments, to generate EBAG9-targeting miRNAs, a miRNA is employed.

miRNAs resemble small interfering RNAs (siRNAs) of the RNA interference (RNAi) pathway, except miRNAs derive from regions of RNA transcripts that fold back on themselves to form short hairpins, whereas siRNAs derive from longer regions of double-stranded RNA.

In some embodiments, an endogenous miRNA can be employed, e.g. the endogenous miRNA-155. The EBAG9-sequence specific targeting sequence, such as the antisense sequences of H17 and H18, can be inserted in the endogenous miRNA, i.e. within the 21-nucleotide containing hairpin structure.

In some embodiments, the EBAG9 knockdown may be achieved by employing an expression vector for the miRNA. miRNA genes are usually transcribed by RNA polymerase II (Pol II), such that appropriate promoters for the expression vector may be elected. The polymerase often binds to a promoter found near the DNA sequence, encoding what will become the hairpin loop of the pre-miRNA. The resulting transcript is capped with a specially modified nucleotide at the 5' end, polyadenylated with multiple adenosines and potentially spliced. The mature miRNA then becomes part of an active RNA-induced silencing complex (RISC) containing Dicer and many associated proteins. Gene silencing may then occur either via EBAG9 mRNA degradation or preventing EBAG9 mRNA from being translated.

In some embodiments, to generate EBAG9-targeting miRNAs, short interfering siRNA are employed. siRNA employ for example short (10-50, preferably 20-25 bp) double stranded or hairpin RNA molecules which comprise a sequence with complementarity to a region of the EBAG9 coding mRNA.

In some embodiments, EBAG9 inhibition is achieved employing siRNA or miRNA directed against a region of any one of SEQ ID NO 3, 4, 5 or 6. A siRNA or miRNA (i.e. the targeting region) may be of 10-50, preferably 15-40, more preferably 18-30, more preferably 20-25 nucleotides, and exhibit sufficient sequence complementarity to the transcript of SEQ ID NO 3, 4, 5 or 6 to induce a reduction in EBAG9 expression over relevant controls. The complementarity between short hairpin RNA and target mRNA may in some embodiments be 50% or more, 60% or more, 70% or more, 80% or more, 85% or more, 90% or more, 95% or more or preferably 100%. Complementarity is preferably calculated analogously to sequence identity, wherein the total number of complementary/identical nucleotides is determined across the entire length of the sequence (e.g. for the length of the short interfering RNA).

In some embodiments, the EBAG9 knockdown may be achieved by employing an expression vector for the siRNA. The siRNA sequence is modified to introduce a short loop between the two strands. The resulting transcript is a short hairpin RNA (shRNA), which can be processed into a functional siRNA by Dicer in its usual fashion. Typical transcription cassettes to express the siRNA in the T cell in which EBAG9 is to be knocked down use an RNA polymerase III promoter (e.g., U6 or H1) to direct the transcription of small nuclear RNAs (snRNAs) (U6 is involved in gene splicing; H1 is the RNase component of human RNase P).

A skilled person is capable of generating further RNA interfering constructs that effectively reduce or inhibit EBAG9 production without undue effort.

In one embodiment, the genetically modified T cell as described herein is characterized in that the inhibition of EBAG9 activity is obtained by genetic modification of the T cell genome with an exogenous nucleic acid molecule, said exogenous nucleic acid molecule preferably comprising a vector that encodes the transgenic antigen-targeting construct and an RNA interfering sequence for knock-down of EBAG9.

In some embodiments, viral vectors are employed, preferably retro- and lentiviral transfers are employed.

In some embodiments, transposons may be employed as a vector encoding a transgenic antigen-targeting construct.

Viral vectors are associated with various advantages, such as they are accepted by regulatory authorities, they show good technical advantages such as reliable modification of cells and they bear a tolerable risk profile.

In some embodiments, the CTL of the present invention employ the MP71 vector, preferably in combination with a gamma retrovirus expression system.

In this combination, an unusually high transduction rate of T cells can be achieved. The transduction system is variable due to a modular design of the CAR construct and the miRNA, meaning that lentiviruses as well as transposons can be employed alternatively.

In some embodiments, the CTL of the present invention employ a retroviral SIN vector (obtainable for example from BIONTECH, Idar-Oberstein), preferably in combination with a gamma retrovirus expression system.

These embodiments of the invention are therefore further advantageous, as due to retroviral, lentiviral or transposon mediated transfer of TCRs or CARs (of any antigen specificity), a single step manipulation of the recipient T cell is sufficient in order to inhibit EBAG9 activity or expression.

In one embodiment, the genetically modified T cell as described herein is characterized in that the inhibition of EBAG9 activity is obtained by genetic modification of the T cell genome by disrupting the expression and/or sequence of the EBAG9 gene. EBAG9 deletions or partial deletions leading to loss of function are envisaged.

As described in more detail below, CRISPR/Cas9 or TALEN technology, or other genetic engineering tools may be employed. The examples of the present application demonstrate that various guide RNAs targeting Exon 4 of EBAG9 together with Cas9 protein are capable of achieving good knockout efficiencies of EBAG9. In one embodiment, without limitation, the CRISPR/Cas9-mediated EBA9 inhibition targets Exon 4 of the EBAG9 gene. This embodiment represents an enabled, but non-limiting preferred target within EBAG9 for CRISPR-mediated knockout; alternative targets suitable for CRISPR-mediated engineering of the EBAG9 gene are known to a skilled person or can be assessed without undue effort.

In other embodiments, genetic engineering tools, such as site directed mutagenesis or recombination-based gene targeting, are known to a skilled person and may also be employed. Genetic (genomic) manipulation however usually requires 2 or several transfections or transductions, which are currently less efficient than viral transfers.

In some embodiments, transfer of the CAR or TCR, and in a second step, transferring the genetic information targeting EBAG9, may be employed to introduce the necessary genetic modifications. In some embodiments, transferring the genetic information targeting EBAG9, and in a second step, transfer of the CAR or TCR, may be employed to introduce the necessary genetic modifications. In some embodiments, the modifications are carried out simultaneously, either in the same vector or exogenous nucleic acid molecule or in separate vectors or exogenous nucleic acid molecules.

In some embodiments, the exogenous nucleic acid molecule encoding the transgenic antigen-targeting construct is positioned in the T cell genome within, adjacent or associated with the EBAG9 gene, thereby disrupting the expression and/or sequence of said EBAG9 gene.

In some embodiments, the CAR sequence may be integrated directly into the EBAG9 genetic locus, and thereby preventing EBAG9 gene expression.

Various alternative strategies for disrupting EBAG9 genetic sequences or mRNA are available to a skilled person and could be established without undue effort. The preferred modes of EBAG9 inhibition disclosed herein are therefore exemplary and not intended to be limiting.

In a further aspect, the invention therefore relates to a genetically modified T cell according to any one of the preceding claims for use as a medicament. Considering the novel T cells defined by EBAG9 inhibition, any given medical use is contemplated. A skilled person is capable of identifying medical conditions that can be treated using CAR-T or TCR T cells and can design a modified T cell accordingly. In preferred embodiments, the medical use is a proliferative disease or an autoimmune disease.

In a further aspect, the invention relates to the genetically modified T cell as described herein for use as a medicament in the treatment of a proliferative disease, wherein the antigen targeted by the transgenic antigen-targeting construct is expressed in a target cell undergoing and/or associated with pathologic cell proliferation. The invention therefore encompasses corresponding methods of treatment employing administering the CTLs as described herein at a therapeutically effective amount to a subject in need thereof.

In one embodiment, the proliferative disease is a hematologic malignancy, and wherein the antigen targeted by the transgenic antigen-targeting construct is expressed in cancerous cells of said hematologic malignancy, for example a tumor-associated antigen (TAA) or tumor-specific antigen (TSA).

Non-limiting examples of hematologic malignancies are non-Hodgkin lymphoma, chronic lymphocytic leukemia, acute myeloid leukemia, acute lymphoblastic leukemia and/or multiple myeloma. Further examples are provided below.

In some embodiments, the antigen targeted by the antigen specific construct is a tumor-associated antigen (TAA). TAAs relate to antigens expressed primarily or preferably in tumor cells, but often these antigens are not expressed exclusively in such cells. CD19 falls under this definition, because it can occur on both transformed and benign B cells. BCMA is also expressed on normal plasma cells and transformed cells, such as multiple myeloma cells.

In some embodiments, the hematologic malignancy to be treated is a CD19-expressing B-cell cancer, and wherein the transgenic antigen-targeting construct binds CD19.

Antibodies directed against CD19 may be used to develop antigen-targeting constructs for CAR constructs targeting cells expressing CD19. In some embodiments, the transgenic antigen-targeting construct comprises an amino acid sequence (antigen binding domain) obtained from an antibody that binds CD19. CD19 antibodies include, without limitation, Blinatumomab, Coltuximabravtansine, MOR208, MEDI-551, Denintuzumabmafodotin, B4, DI-B4 from Merck, Taplitumomabpaptox, XmAb 5871, MDX-1342 or AFM11.

Various CAR constructs targeting CD19 are described in the art. EBAG9 silencing could in principle be applied to any one or more of such CAR-T cell therapeutics. Known CD19 CAR-T cells under clinical investigation are, without limitation, CARs comprising an antigen binding domain with an ScFv of FMC63 or SJ25C1. CAR constructs with either 4-1BB or CD28 costimulatory domains are known, as reviewed in Park et al (Blood, 2016, 127:3312-3320). CAR-T cells under clinical investigation are, without limitation, Tisagenlecleucel form Novartis, axicabtagene ciloleucel from Gilead Sciences, JCAR015 and JCAR017 from Juno Therapeutics, CAR CD19 from Ziopharm Oncology, UCART19 from Cellectis, ALLO-501 from Allogene, BPX-401 from Bellicum Pharmaceuticals, PCAR-019 from PersonGen Biomedicine Suzhou, PBCAR-0191 from Precision Biosciences, SENL-001 from Hebei Senlang Biotechnology, UWC-19 from UWELL Biopharma.

In some embodiments, the hematologic malignancy to be treated is a BCMA-expressing B-cell cancer, and wherein the transgenic antigen-targeting construct binds BCMA.

Antibodies directed against BCMA may be used to develop antigen-targeting constructs for CAR constructs targeting cells expressing BCMA. In some embodiments, the transgenic antigen-targeting construct comprises an amino acid sequence (antigen binding domain) obtained from an antibody that binds BCMA. BCMA antibodies include, without limitation, those described herein, in addition to the antibodies employed in antibody-drug conjugates GSK2857916, HDP-101 or MEDI2228, or in the bi-specific antibodies EM801, Ab-957, AFM26 or TNB383B.

Various CAR constructs targeting BCMA are described in the art. EBAG9 silencing could in principle be applied to any one or more of such CAR-T cell therapeutics. Known BCMA CAR-T cells under clinical investigation are, without limitation, bb2121 from Bluebird Bio, LCAR-B38M from Nanjing Legend Biotech, CART-BCMA from Novartis, KITE-585 from Kite Pharma and BCMA CAR from Pfizer/Cellectis SA, P-BCMA-101 from Poseida Therapeutics, FHVH74-CD828Z, FHVH32-CD828Z, FHVH33-CD828Z, FHVH93-CD828Z from Tenebrioas, Descartes-08 from Cartesian Therapeutics, P-BCMA-ALLO1 from Poseida Therapeutics and EGFRt/BCMA-41BBz from Juno, reviewed in Cho et al (Front Immunol. 2018; 9: 1821).

In some embodiments, the hematologic malignancy to be treated is a CXCR5-expressing cancer, and wherein the transgenic antigen-targeting construct binds CXCR5.

Further tumor associated antigens, capable of targeting via the antigen specific construct, e.g. CAR or TCR construct, may be selected from those below:
The CAR-T or TCR antigen may be selected, without limitation thereto, from those for which CAR constructs have been developed and are in clinical or pre-clinical testing, including hematologic malignancy associated TAA, such as CD30, CD20, CD22, ROR1, CD138, CD70, LeY, CD123, CD16V, CD123, CD33, Ig kappa, Ig lambda, IL-1RAP, NKG2D ligands, Muc1, GPC3, EpCam, CD38, CD5, IL-13Ralpha2, CD133 or CS1 (CD319).

TAAs from solid malignancies may also be targeted and include, without limitation, GPC3, HER2, GD2, EGFR variant III (EGFR vIII), EGFR, CEA, PSMA, FRα, EPCAM, MUC1, ROR1, MUCI16eto, VEGFR2, CD171, PSCA and EphA2, FAP, CAIX, c-MET, CD171, L1-CAM, Mesothelin, Muc1, PD-L1.

Other tumor associated antigens (TAAs) including MART-1, MAGE-A1, MAGE-A3, MAGE-A4, gp100, CEA, TIL 1383I, P53, HPV-16 E6, HPV-16 E7 and HBV may also be selected as the TCR-T or CAR targets.

A detailed summary of the published clinical trials of chimeric antigen receptor T cells (CAR-T) and TCR-transduced T cells (TCR-T) is disclosed in Mo et al (Journal of Cancer, 2017; 8(9): 1690-1703), Hartmann et al. (EMBO Molecular Medicine, 2017; 9 (9): 1183-1197), and Townsend et al. (Journal of Experimental & Clinical Cancer Research, 2018, 37:163).

Although blood cancer (hematological malignancies) appear better suited for a T cell therapy, solid tumors may also be treated, as evidenced by the multiple clinical trials underway investigating a cytolytic effect of CAR-T or TCR-T cells on solid tumor diseases.

For example, for lung cancer, the candidate TAAs may contain mesothelin, EGFR, MUC1, RORI, CEA, WT1, NY-ESO-1 or MAGE-A3/4.

Combined approaches, based on antigen targeting constructs directed to two or more of the above antigens, may also be employed, for example by using one or more targeting constructs targeting CD19 and CD20.

Furthermore, adoptive tandem-CAR-T therapy in glioblastomas appears effective by targeting heterogeneous expression antigens human epidermal growth factor receptor 2 (HER2) and IL13Rα2 on glioblastomas.

Not only are tumor-type specific and diversified TAAs expressed in one kind of cancer targeted, but also one TAA is expressed in multiple kinds of cancers. These TAAs may also be targeted. For example, the NY-ESO-1 is highly expressed in melanoma, multiple myeloma, NSCLC, synovial sarcoma, breast cancer, renal cell cancer, hepatocellular cancer, esophageal cancer, ovarian cancer and bladder cancer. Similarly, mesothelin is highly expressed in mesothelioma and breast cancer, cervical cancer, pancreatic cancer, ovarian cancer, lung cancer and endometrial cancer.

Abbreviations for the above antigens: PD-1: programmed death-1 receptor; NSCLC: non-small cell lung cancer; HLA: histocompatibility leukocyte antigen; ROR1: tyrosine kinase-like orphan receptor 1; BCMA: B-cell maturation antigen; LeY: Lewis (Le)-Y; GPC3: Glypican-3; HER2: human epidermal growth factor receptor-2; GD2: The tumor-associated ganglioside GD2; EGFR: epidermal growth factor receptor; CEA: carcinoembryonic antigen; PSMA: prostate-specific membrane antigen; FRα: folate receptor-alpha; EPCAM: epithelial cell adhesion molecule; MUC1: mucin 1; VEGFR2: vascular Endothelial Growth Factor Receptor-2; PSCA: prostate stem cell antigen; EphA2: erythropoietin-producing hepatocellular carcinoma A2; HPV: human papillomavirus; MAGE: melanoma-associated antigen-encoding gene; TNF-α: tumor necrosis factor-a.

The specific TAAs described herein are of exemplary nature and represent preferred non-limiting embodiments of the invention. The inventive concept of EBAG9 inhibition can be applied to any given modified T cell regardless of antigen specificity of the T cell.

In a further aspect, the invention relates to a pharmaceutical composition comprising a genetically modified T cell as described herein, suitable for the treatment of a proliferative disease, comprising additionally a pharmaceutically acceptable carrier.

In a further aspect, the invention relates to a nucleic acid vector or combination of nucleic acid vectors comprising a sequence that encodes an antigen-targeting construct and an RNA interfering sequence for knock-down of estrogen receptor-binding fragment-associated antigen 9 (EBAG9).

In a further aspect, the invention relates to an in vitro method for increasing the cytolytic activity of a genetically modified cytotoxic T cell, said T cell comprising one or more exogenous nucleic acid molecules encoding a transgenic antigen-targeting construct, the method comprising inhibiting in said T cell the activity of estrogen receptor-binding fragment-associated antigen 9 (EBAG9).

In a further aspect, the invention relates to the product of the in vitro method, namely a set of isolated T cells modified with a transgenic antigen targeting construct in which EBAG9 is inhibited.

In one embodiment, the isolated T cells modified with a transgenic antigen targeting construct in which EBAG9 is inhibited, produced via a method of the invention, are characterized by an inhibition of EBAG9 in comparison to control CTLs of 10% or more, 20%, 30%, 40%, 50%, 60%, 70%, 80%, or 90% or more. In some cases, for example where EBAG9 is deleted or expression is disrupted due to e.g. genomic modifications, a 100% inhibition of EBAG9 may be achieved.

In one embodiment, the isolated T cells modified with a transgenic antigen targeting construct in which EBAG9 is inhibited, as produced by the method of the present invention, are characterized by an increase in cytotoxic activity of 5% or more, 10% or more, 20%, 30%, 40%, 50%, 60%, 70%, 80%, 90%, 100%, 150%, 200%, or 300% or more, when compared to cytolytic T cells without the EBAG9 inhibition. Functional assays for determining quantitatively, or semi-quantitatively the increase in cytolytic activity in the T cells in which EBAG9 is inhibited, are available to a skilled person, some examples of which are described herein. For example, the in vitro assays used to generate Figures 7 and 8 below, or as described in the examples, may be applied in order to determine an increase in cytolytic activity over "unmodified" T cells, i.e. T cells in which no specific modification has been carried out to reduce/inhibit EBAG9 function. In some embodiments, this assay can be used to determine whether the method of the invention has been carried out, i.e. in order to determine any decrease in EBAG9 function.

The in vitro method for increasing the cytolytic activity of the cells represents an important aspect of the invention, as T cell therapeutics require typically in vitro processing prior to administration in a patient. In a typical adoptive T cell therapy (ATT), T cells are isolated from the patient and subsequently genetically modified to express a chimeric antigen receptor (CAR) or T-cell receptor (TCR). During this preparation process the CTLs may be further modified to increase their cytolytic activity.

The present invention therefore enables a method of enhancing the efficacy of the cellular therapeutic without increasing additional burden or difficulty during the process of preparing the cells. The T cells intended for therapeutic application are therefore not subjected to undue stress during preparation, as in some embodiments, the EBAG9 inhibition can be achieved in the same genetic modification step with which the CTL is modified with the antigen targeting construct.

In some embodiments, the in vitro method as described herein is characterized in that inhibiting EBAG9 activity comprises knock-down of EBAG9, preferably by RNA interference of EBAG9 expression, such as by small interfering RNA (siRNA), short hairpin RNA (shRNA) and/or micro RNA (miRNA).

In some embodiments, the in vitro method as described herein is characterized in that inhibiting EBAG9 activity comprises genetic modification of the T cell genome by disrupting the expression and/or sequence of the EBAG9 gene, preferably by CRISPR/Cas9 or TALENs.

In further embodiments of the invention, CRISPR/Cas and TALEN-mediated insertion of the CAR or TCR encoding nucleic acid may be employed. CRISPR/Cas, known to a skilled person, which is adapted from a naturally occurring process in bacteria, may be employed to precisely and efficiently edit DNA to insert the appropriate coding sequences into the immune cell, preferably T cell, of interest. Cas9, a protein that acts as a molecular pair of scissors, is guided to a specific DNA sequence by an associated RNA molecule (a guide RNA). When Cas9 arrives at its target location on the DNA, it facilitates a change in the local genetic code, affecting the function of that gene. CRISPR/Cas9 can deliver the CAR or TCR gene to a very specific site within the T cell genome, which may reduce the risk of gene insertion at incorrect or undesired locations.

As mentioned above, the examples of the present application demonstrate that various guide RNAs targeting Exon 4 of EBAG9 together with Cas9 protein are capable of achieving good knockout efficiencies of EBAG9. In one embodiment of the method for enhancing the efficacy of the cellular therapeutic agent, CRISPR/Cas9-mediated EBA9 inhibition is employed, and preferably targets Exon 4 of the EBAG9 gene. This embodiment represents an enabled, but non-limiting preferred target within EBAG9 for CRISPR-mediated knockout; alternative targets suitable for CRISPR-mediated engineering of the EBAG9 gene are known to a skilled person or can be assessed without undue effort.

The invention relates further to methods of treatment of the medical conditions described herein, comprising typically the administration of a therapeutically effective amount of the modified CTLs to a patient in need of said treatment.

In some embodiments, the treatment described herein can be carried out in a novel or unique patient group due to the increased activity of the CTLs. For example, the CTL described herein is in preferred embodiments applicable to the treatment of patients who are not eligible for other therapies. More specifically, embodiments of the invention relate to the treatment of the following patient collectives:
i) patients with multidrug resistances,
ii) patients not eligible for allogeneic stem cell transplantation,
iii) patients with co-morbidities that preclude further chemotherapies,
iv) aged patients who do not tolerate chemotherapies,
v) the CTL is applicable for salvage therapies even after progressive disease and multiple lines of other standard of care therapies have failed,
vi) it is applicable even at low antigen density on target tumor cells, where antibodies can fail, and/or
vii) it is applicable as a monotherapy which is not the case for antibodies.

In addition, the ability to specifically target B and/or plasma cells would be of great benefit for the treatment of autoimmune diseases. Mild forms of autoimmune disease are usually initially treated with nonsteroidal anti-inflammatory drugs (NSAID) or disease-modifying anti-rheumatic drugs (DMARD). More severe forms of Systemic Lupus Erythematosus (SLE), involving organ dysfunction due to active disease, usually are treated with steroids in conjunction with strong immunosuppressive agents such as cyclophosphamide, a cytotoxic agent that targets cycling cells.

Recently, CAR-T cells were also discussed as a targeted approach to treat autoantibody-mediated diseases (Ellebrecht et al. (2016) Science 353:179-184). Long-lived, sessile plasma cells residing in survival niches in the bone marrow are often resistant to conventional immunosuppressive and cytotoxic drugs as well as to therapies targeting B cells and their activation. This therapeutic challenge could be met by employing CAR-T cell constructs, in particular those with EBAG9 inhibition.

The invention therefore further relates to a genetically modified T cell as described herein for use as a medicament in treating an autoimmune disease.

In a further aspect, the invention therefore relates to a genetically modified T cell as described herein for use as a medicament in the treatment of an autoantibody-dependent autoimmune disease, wherein the antigen targeted by the transgenic antigen-targeting construct is expressed in a target cell associated with autoantibody production, preferably wherein the medical disorder is systemic lupus erythematosus (SLE) or rheumatoid arthritis. Further embodiments of autoimmune-related diseases are provided below. A skilled person in capable of selecting an appropriate target antigen for the antigen targeting construct of the T cells of the present invention. One relevant antigen for targeting autoantibody producing plasma cells is BCMA.

The technical guidance for treating cancer cells provided by the present specification and examples herein also extends to enable the treatment of autoimmune disease. Autoreactive B cells can be effectively targeted by selection of an appropriate antigen to be targeted by the antigen-specific targeting construct of the engineered T cell. The expression of EBAG9 in the engineered T cell is of primary interest; the expression/activity of EBAG9 is reduced/inhibited, therefore enhancing the cytotoxicity of the T cell, independent of EBAG9 expression in the target cell. Therefore, any given target cell can be attacked by the engineered T cell, which has an enhanced cytotoxicity due to EBAG9 inhibition.

The features regarding the modified T cells, their use as a medicament, the related embodiments regarding the vector, pharmaceutical composition and in vitro method, are unified by the novel concept of a modified cytolytic T cell, comprising an antigen specific targeting construct, in which EBAG9 is inhibited. The features disclosed herein with respect to any one aspect of the modified T cells, their use as a medicament, the related embodiments regarding the vector, pharmaceutical composition and in vitro method, are considered to be disclosed in the context of the alternative inventive aspects, such that the features of the cell may also be used to describe e.g. the methods or compositions disclosed herein, and vice versa.

### Embodiments regarding chimeric antigen receptor constructs and CAR T cells:

In one embodiment, the invention relates to a CTL as described herein comprising a chimeric antigen receptor polypeptide (CAR), comprising:
- an extracellular antigen-binding domain, comprising an antibody or antibody fragment that binds a target antigen, wherein said antibody or antibody fragment comprises VH and VL domains of a single chain antibody fragment, wherein preferably a linker polypeptide is positioned between the VH and VL domains, wherein said linker is preferably configured to not interfere with the antibody fragment-antigen interaction;
- a spacer polypeptide (also referred to as a hinge) positioned between the extracellular antigen-binding domain and a transmembrane domain, wherein said spacer polypeptide is preferably configured to not interfere with the antibody fragment-antigen interaction and/or with T cell activation when said CAR is expressed in a T cell expressing said CAR;
- a transmembrane domain, wherein said transmembrane domain is preferably configured to not interfere with the antibody fragment-antigen interaction and/or with T cell activation when said CAR is expressed in a T cell expressing said CAR;
- and an intracellular domain, wherein said intracellular domain comprises a co-stimulatory domain and a signalling domain, wherein said intracellular domain is preferably configured to provide signals to stimulate T cell activation upon binding to the antigen target, for example by increasing cytokine production and/or facilitating T cell replication, thus leading to cytotoxic effect.

The CAR of the present invention may therefore employ various formats, comprising potentially different protein sequences for each of the functional domains described herein. A skilled person can select and testing the desired function of the CARs, for example based on the experimental approaches demonstrated in the examples below. As such, the election of any given specific protein sequence to be used in the CAR of the invention, in any of the functional domains discussed herein, can be assessed by a skilled person using routine methods for functional efficacy. For example, various linker polypeptide sequences positioned between the VH and VL domains, various spacer polypeptide sequences (also referred to as a hinge) positioned between the extracellular antigen-binding domain and a transmembrane domain, various transmembrane domains and various intracellular domains, preferably comprising co-stimulatory and signalling domains, may be employed. The various co-stimulatory domains can also be used in tandem (CD28 + 4-1BB for example).

In embodiments of the invention, the CAR, and each of the elements or domains mentioned herein, are configured to not detrimentally interfere with the antibody fragment-antigen interaction, to not detrimentally interfere with T cell activation when said CAR is expressed in a T cell expressing said CAR, and to not detrimentally interfere with the CAR providing signals to stimulate T cell activation upon binding to the target. In preferred embodiments, the elements of the CAR are selected not to interfere with the EBAG9 inhibition and concomitant increase in cytolytic activity.

### Embodiments relating to the antigen-binding domain of the CAR:

Provided as non-limiting examples, the antigen binding domain of the CAR may be directed to BCMA, CXCR5 or CD19. In other embodiments, antigen binding fragments may be selected based on any given desired antigen considered as a target of a malignant cell. Several specific, non-limiting embodiments for CXCR5 and BCMA CARs are described below, which have been assessed by the inventors and shown to exhibit enhanced cytolytic activity when present in T cells in combination with EBAG9 inhibition.

### CXCR5-specific antigen binding domains:

In one embodiment, the CTLs of the invention comprise a CXCR5-specific chimeric antigen receptor (CAR) polypeptide as described herein, wherein the antigen-binding domain comprises:
- H-CDR1 according to SEQ ID NO 7 (GFTFSTSG),
- H-CDR2 according to SEQ ID NO 8 (ISSSSGFV),
- H-CDR3 according to SEQ ID NO 9 (ARSEAAF),
- L-CDR1 according to SEQ ID NO 10 (KSRLSRMGITP),
- L-CDR2 according to a sequence comprising or consisting of RMS, and
- L-CDR3 according to SEQ ID NO 11 (AQFLEYPPT).

In one embodiment, the CXCR5-specific CAR polypeptide comprises:
- a VH domain with at least 80% sequence identity to SEQ ID NO 12 
(EVQLVESGGGLVQPGGSLRLSCAASGFTFSTSGMNWFRQAPGKGLEWVSYISSSSGFVY
ADSVKGRFTISRDNAQNSLYLQMNSLRAEDTAVYYCARSEAAFWGQGTLVTVSS),
- or with at least 80% sequence identity to SEQ ID NO 13 
(EVQLVESGGGLVQPGKSLKLSCSASGFTFSTSGMHWFRQAPGKGLDWVAYISSSSGFVY
ADAVKGRFTISRDNAQNTLYLQLNSLKSEDTAIYYCARSEAAFWGQGTLVTVSS)
- and a VL domain with at least 80% sequence identity to SEQ ID NO 14 
(DIVLTQSPRSLPVTPGEPASISCRSSKSRLSRMGITPLNWYLQKPGQSPQLLIYRMSNRAS
GVPDRFSGSGSGTDFTLKISKVETEDVGVYYCAQFLEYPPTFGSGTKLEIK),
- or with at least 80% sequence identity to SEQ ID NO 15

(DIVLTQAPRSVSVTPGESASISCRSNKSRLSRMGITPLNWYLQKPGKSPQLLIYRMSNLAS
GVPDRFSGSGSETDFTLKISKVETEDVGVYYCAQFLEYPPTFGSGTKLEIK).

### BCMA-specific antigen binding domains:

In one embodiment, the CTLs of the invention comprise a BCMA-specific chimeric antigen receptor (CAR) polypeptide as described herein, wherein the antigen-binding domain comprises:
- H-CDR1: GFTFSRYW (SEQ ID NO. 16),
- H-CDR2: INPSSSTI (SEQ ID NO. 17),
- H-CDR3: ASLYYDYGDAYDY (SEQ ID NO. 18),
- L-CDR1: QSVESN (SEQ ID NO. 19),
- L-CDR2: SAS, and
- L-CDR3: QQYNNYPLT (SEQ ID NO. 20).
or wherein the antigen-binding domain comprises:
- H-CDR1: RYWFS (SEQ ID NO. 21),
- H-CDR2: EINPSSSTINYAPSLKDK (SEQ ID NO. 22),
- H-CDR3: SLYYDYGDAYDYW (SEQ ID NO. 23),
- L-CDR1: KASQSVESNVA (SEQ ID NO. 24),
- L-CDR2: SASLRFS (SEQ ID NO. 25), and
- L-CDR3: QQYNNYPLTFG (SEQ ID NO. 26),

In one embodiment, the BCMA-specific CAR polypeptide comprises a VH domain with at least 80% sequence identity to:
SEQ ID NO 27 
(EVQLVESGGGLVQPGGSLRLSCAASGFTFSRYWFSWVRQAPGKGLVWVGEINPSSSTINYAPSLK
DKFTISRDNAKNTLYLQMNSLRAEDTAVYYCASLYYDYGDAYDYWGQGTLVTVSS);
and a VL domain with at least 80% sequence identity to SEQ ID NO 28

(EIVMTQSPATLSVSPGERATLSCKASQSVESNVAWYQQKPGQAPRALIYSASLRFSGIPARFSGSG
SGTEFTLTISSLQSEDFAVYYCQQYNNYPLTFGAGTKLELK).

### Embodiments relating to the linker, spacer, transmembrane, and signaling domains:

Below several embodiments are presented for specific non-antigen specific CAR domains employed in the examples.

In further embodiments, the invention relates to a CTL as described herein comprising a chimeric antigen receptor (CAR) polypeptide that comprises one or more linker, spacer, transmembrane, and signaling domains.

In one embodiment, the CAR comprises an intracellular domain, which comprises one or two co-stimulatory domain and a signalling (activation) domain.

In one embodiment, the CAR comprises an extracellular antigen-binding domain with a linker polypeptide positioned between the VH and VL domains, wherein said linker is preferably selected from
- a Whitlow (SEQ ID NO 29; GSTSGSGKPGSGEGSTKG), or
- Gly-Ser (SEQ ID NO 30; SSGGGGSGGGGSGGGGS) linker, or
- linkers with at least 80% sequence identity to SEQ ID NO 29 or 30.

In one embodiment, the CAR comprises additionally a spacer polypeptide positioned between the extracellular antigen-binding domain and the transmembrane domain, wherein said spacer is selected from:
- IgG1 spacer (SEQ ID NO 31; 
PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSLSPGKKDPK),
- IgG1Δ spacer (SEQ ID NO 32; 
PAEPKSPDKTHTCPPCPAPPVAGPSVFLFPPKPKDTLMIARTPEVTCVVVDVSHEDPEVKF
NWYVDGVEVHNAKTKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNGQPENNYKTTPPV
LDSDGSFFLYSKLTVDKSRWQQGNVFSCSVMHEALHNHYTQKSLSSLSPGKK),
- IgG4 (Hi-CH2-CH3) spacer (SEQ ID NO 33; 
ESKYGPPCPPCPAPEFEGGPSVFLFPPKPKDTLMISRTPEVTCVVVDVSQEDPEVQFNWY
VDGVEVHNAKTKPREEQFNSTYRVVSVLTVLHQDWLNGKEYKCKVSNKGLPSSIEKTISKA
KGQPREPQVYTLPPSQEEMTKNQVSLTCLVKGFYPSDIAVEWESNGQPENNYKTTPPVLD
SDGSFFLYSRLTVDKSRWQEGNVFSCSVMHEALHNHYTQKSLSLSLGK),
- IgG4 (Hi-CH3) spacer (SEQ ID NO 34; 
ESKYGPPCPPCPGQPREPQVYTLPPSQEEMTKNQVSLTCLVKGFYPSDIAVEWESNGQPE
NNYKTTPPVLDSDGSFFLYSRLTVDKSRWQEGNVFSCSVMHEALHNHYTQKSLSLSLGK),
- IgG4 (Hi) spacer (SEQ ID NO 35; ESKYGPPCPPCP), or
- a spacer with at least 80% sequence identity to any one of SEQ ID NO 31 to 35.

In one embodiment, the transmembrane domain is selected from:
- a CD8α domain (SEQ ID NO 36; IYIWAPLAGTCGVLLLSLVITLYC), or
- a CD28 domain (SEQ ID NO 37; FWVLVVVGGVLACYSLLVTVAFIIFWV), or
- transmembrane domains with at least 80% sequence identity to SEQ ID NO 36 or 37.

In one embodiment, the intracellular domain comprises:
- a co-stimulatory domain selected from a 4-1BB co-stimulatory domain (SEQ ID NO 38; KRGRKKLLYIFKQPFMRPVQTTQEEDGCSCRFPEEEEGGCEL), and/or
- a CD28 co-stimulatory domain (SEQ ID NO 39; RSKRSRLLHSDYMNMTPRRPGPTRKHYQPYAPPRDFAAYRSL), or
- a co-stimulatory domain comprising both a 4-1BB (SEQ ID NO 38) and a CD28 co-stimulatory domain (SEQ ID NO 39) arranged adjacently, or
- a co-stimulatory domain with at least 80% sequence identity to SEQ ID NO 38 or 39.

In one embodiment, CAR comprises additionally a signaling domain (otherwise known as an activation domain), wherein said signaling domain is
- a CD3zeta (4-1BB or CD28) signaling domain (SEQ ID NO 40; 
LRVKFSRSADAPAYQQGQNQLYNELNLGRREEYDVLDKRRGRDPEMGGKPRRKNPQEGL
YNELQKDKMAEAYSEIGMKGERRRGKGHDGLYQGLSTATKDTYDALHMQALPPR), or
- a signaling domain with at least 80% sequence identity to SEQ ID NO 40.

The invention further relates to (isolated) nucleic acid molecules for use in the CTLs of the present invention, preferably in the form of a vector, such as a viral vector or a transposon vector, preferably a gamma retroviral vector, selected from the group consisting of:
a) a nucleic acid molecule comprising a nucleotide sequence
   - which encodes a chimeric antigen receptor (CAR) polypeptide according to any embodiment of the CAR described herein, or
   - which encodes an extracellular antigen-binding domain, a leader, a transmembrane domain, a spacer, linker or an intracellular domain, preferably according to SEQ ID NO 7-41, or
   - which encodes a leader, transmembrane domain, a spacer, linker or an intracellular domain, the nucleic acid sequence comprising or consisting of one or more of SEQ ID NO 43-54,
b) a nucleic acid molecule which is complementary to a nucleotide sequence in accordance with a);
c) a nucleic acid molecule comprising a nucleotide sequence having sufficient sequence identity to be functionally analogous/equivalent to a nucleotide sequence according to a) or b), comprising a sequence identity to a nucleotide sequence according to a) or b) of at least 80%, preferably 90%, or 95% sequence identity to a nucleotide sequence according to a) or b), wherein functional analogy relates to forming a CAR construct, and when corresponding T cells express said construct, the CAR-T cell product confers T cells with a cytotoxic activity; and/or
d) a nucleic acid molecule which, as a consequence of the genetic code, is degenerate to a nucleotide sequence according to a) through c).

The exchange of signaling domains meets the demands for either a strong and rapid effector phase (CD28 co-stimulatory domain), or a long-lasting relapse control as secured by a T cell memory population (4-1BB signaling domain). The various signaling domains may be exchanged in multiple configuration, providing a CAR with flexibility with respect to its design without loss of the advantageous binding properties.

In preferred embodiments, in combination with the MP71-vector and a gamma-retrovirus expression system, an unusually high transduction rate for human T cells can be achieved. The transduction system is variable due to a modular design of the TCR or CAR construct, meaning that lentiviruses as well as transposons can be employed, depending on the needs and preferences of the skilled person when carrying out the invention. Transfer of the genetic information/nucleic acid molecule for the TCR, CAR and EBAG9 inhibition also includes CRISPR/Cas and TALEN mediated insertion into CTLs.

All suitable methods for transferring the genetic information/nucleic acid molecule for the TCR, CAR and EBAG9 inhibition into the cell expressing said CAR are encompassed by the present invention, and a suitable method may be selected by a skilled person when carrying out the invention. For example, multiple methods of transforming T cells are known in the art, including any given viral-based gene transfer method, such as those based on modified Retroviridae, and non-viral methods such as DNA-based transposons, episomal cDNA vectors and direct transfer of mRNA by electroporation.

Additionally, the signaling components of the CAR construct may be exchanged in a simple three step cloning procedure that allows for a modular composition, and tailor-made construction by a skilled person, of clinically applicable CARs.

A further aspect of the invention relates to a vector comprising a nucleic acid molecule as described herein, preferably a viral vector, more preferably a gamma retroviral vector. In another aspect of the invention, the invention relates to a transposon vector, preferably a sleeping beauty vector, encoding and preferably capable of expressing the inventive TCR, CAR and/or EBAG9 inhibition.

In a preferred embodiment the CTLs intended for administering in treatment of the diseases mentioned herein are genetically modified with a nucleic acid as described herein, encoding and expressing a TCR, CAR and EBAG9 silencing molecule, e.g. miRNA, as described herein, using a "Sleeping beauty" transposon system, in particular a sleeping beauty transposase. The Sleeping Beauty transposon system is a synthetic DNA transposon designed to introduce precisely defined DNA sequences into the chromosomes of vertebrate animals, in the context of the present invention for the purposes of modifying immune cells to express the CAR or TCR as described herein. The sleeping beauty transposons combine the advantages of viruses and naked DNA. Viruses have been evolutionarily selected based on their abilities to infect and replicate in new host cells. Simultaneously, cells have evolved major molecular defense mechanisms to protect themselves against viral infections. Avoiding the use of viruses is also important for social and regulatory reasons. The use of non-viral vectors such as the sleeping beauty system therefore avoids many, but not all, of the defenses that cells employ against vectors. For this reason, the sleeping beauty system enables particularly effective and safe genetic modification of the immune cells for administration to a patient.

Preferred amino acid and nucleotide sequences of the present invention:

| **SEQ ID NO** | **Sequence** | **Description** |
|---|---|---|
| 1 | AAATAACCGAAACTGGGTGAT | H17 EBAG9 targeting 1 |
| 2 | TTAAATAACCGAAACTGGGTG | H18 EBAG9 targeting 2 |
| 3 | AGGCGTGCAGCATTCGCCATGCTCCGCTCACGCGTGGGAGACTGG GCTGTGGGGTACCGGCCCGGAAAGCACGCAGCCTCCAAAGCCGC CTTCCTCAGGGAAATTTGCGTGACCTTACTGCCCTCCGTCTACAGG CCTTGTACCTCTCCAGGCCGATTTTTCCACAATTTAAATCTCAGTTC ACCTGGTATCCAGCTCCAGCAACTTAGAGCGTTTCACGTCACGCCG GGCGCCAGGCGTCGGCTTGTATAACCTGAAAACGCTCCTGTTTTTC TCATCTGTGCAGTGGGTTTTGATTCCCACCATGGCCATCACCCAGT TTCGGTTATTTAAATTTTGTACCTGCCTAGCAACAGTATTCTCATTCC TAAAGAGATTAATATGCAGATCTGGCAGAGGACGGAAATTAAGTGG AGACCAAATAACTTTGCCAACTACAGTTGATTATTCATCAGTTCCTA AGCAGACAGATGTTGAAGAGTGGACTTCCTGGGATGAAGATGCAC CCACCAGTGTAAAGATCGAAGGAGGGAATGGGAATGTGGCAACAC AACAAAATTCTTTGGAACAACTGGAACCTGACTATTTTAAGGACATG ACACCAACTATTAGGAAAACTCAGAAAATTGTTATTAAGAAGAGAGA ACCATTGAATTTTGGCATCCCAGATGGGAGCACAGGTTTCTCTAGT AGATTAGCAGCTACACAAGATCTGCCTTTTATTCATCAGTCTTCTGA ATTAGGTGACTTAGATACCTGGCAGGAAAATACCAATGCATGGGAA GAAGAAGAAGATGCAGCCTGGCAAGCAGAAGAAGTTCTGAGACAG CAGAAACTAGCAGACAGAGAAAAGAGAGCAGCCGAACAACAAAGG AAGAAAATGGAAAAGGAAGCACAACGGCTAATGAAGAAGGAACAAA ACAAAATTGGTGTGAAACTTTCATAACACATGTTCAAATTTTATCATG CCAGTAGGAGAAATCTCAGCTCCACAACCCAAGCAACATTTGTATG GATTTAAGAGTATTTTAAGAAGACATACTGCTTGATTTTAATACATTG ATCAGGCCATCCAGGACACCACGATTCTCCCAAAGTACCTTGAACT CTTAGTGATTGAGACTCAAAAAAACAAAAAAGACTTGAGACAATGTT TTCTTCAACATGCTCCAAATATAAGACATTTGTTTGCTGTACAGAAA GTATCACAAATGGAATATATCAGTACCTCTCAAGCTAGTGTTTCTAG CTAAATAAATGGGTGTATATAATTTTATGGTGGAAAAGAACTGTACT GTCTGTTATGATTTCCTTCAATGTGCATAATGATAAAATAAATAATTT TAATATTCTTTTGTTTCCATGGTTACCTGACCTAAATTAGATAAATTG TAGGGCTTTAGCTTTCTTATTTTTGTCAAAAGTTGGTGTTGACATAC ATTCCCTCTAATTTGAACTGGTATTGTTTACGTTTGATACAACATTAA GGAATTTGATGATTTTCATTTCATGAAAATGACATTAAATGCAATAAT TTTACTTATCATAAACATTTTGTACATCATTATTTTCTTTTGGATTAGT GTTGTCATACATGTAATTTATATCACATGTATAAACATTGAAAATCAA CTAAAATGACATTTGTTCTACATTATAACTTGTGAGTTTCAAGAACTA GTATTAGTAGTTTTTTCCTTTCATTATAGATTGTAAGATGTTGTGGTA TCTTTGAGTGCCTTAGTTCTTCCTTCCTCCCAAAAGCCATTAATTTA CAAATGCTTAAAGCCATCAGGTCAAATATTTCAAAGCCTTTAGATGA TTTCTGTACTAGCTTTAGATGTCTGACGTTATGTAGGTTACCTGTGT TCTTGGCTAGGAAAACATTATTGATTCATTAAATCATAAAGGTGGGA ATAATAATCTTTTATTTATGATGTTGATTGGCTCCAAAATAGTCTTAA GGAAATAAATACTGGGTCTGTAGGGGAAAAGTAGACTTCATAGTTT | EBAG9 transcript variant 1 |
| | AAAATCCCATTAACCTTTTCACCGCAGTTGAAATGCATCCAGCCTGA TTTTCCTATCATTTTGGAATTTTTAAGGATTTTTACTTTCTTAAGTTAC TGCCTAGAATCAACATTCGGTGAGATTTTGAAATGTCATAGATACTG TACAGGCCAAACCTTACTAATTTATTTTACTTAAAGTGATATTTTATA GAAAAATCATAAGTTATACAATGAGAACCCTTTAAGCCCTTAGCCTA AGCTTTCAGACTAAATGTGATTATAGAATAAGATGAAAGTTAACTTT GGTACAGAGCTTTTTATAGCCCCAAATTATATTTCCAGTTATTATATT CATTAGAATTTCTGCTAATAAACTCCCAACTTAAATAGAA | |
| 4 | GCGGGTTTCCCGATGAAGGGGCGGCCATGGCAGCTGCGCAGAGG CAACGCAGGCTGCTACGGAGCGCGCGCCCGGCTTTGAATGAGCG GGGCTGGGAGTGAGCGGGCGGAGCGCGAGCTCGAGGAAGAGACA GGCAGCGCGCGTGAGCGCGCCTTGTGTGCGCGCGCGGCCCGCG GCAGCTCGGAGCCTCCGCCGGGCGGGCGGGGAGGGGGAGGGGC AGGTTTTGATTCCCACCATGGCCATCACCCAGTTTCGGTTATTTAAA TTTTGTACCTGCCTAGCAACAGTATTCTCATTCCTAAAGAGATTAAT ATGCAGATCTGGCAGAGGACGGAAATTAAGTGGAGACCAAATAACT TTGCCAACTACAGTTGATTATTCATCAGTTCCTAAGCAGACAGATGT TGAAGAGTGGACTTCCTGGGATGAAGATGCACCCACCAGTGTAAAG ATCGAAGGAGGGAATGGGAATGTGGCAACACAACAAAATTCTTTGG AACAACTGGAACCTGACTATTTTAAGGACATGACACCAACTATTAGG AAAACTCAGAAAATTGTTATTAAGAAGAGAGAACCATTGAATTTTGG CATCCCAGATGGGAGCACAGGTTTCTCTAGTAGATTAGCAGCTACA CAAGATCTGCCTTTTATTCATCAGTCTTCTGAATTAGGTGACTTAGA TACCTGGCAGGAAAATACCAATGCATGGGAAGAAGAAGATGCA GCCTGGCAAGCAGAAGAAGTTCTGAGACAGCAGAAACTAGCAGAC AGAGAAAAGAGAGCAGCCGAACAACAAAGGAAGAAAATGGAAAAG GAAGCACAACGGCTAATGAAGAAGGAACAAAACAAAATTGGTGTGA AACTTTCATAACACATGTTCAAATTTTATCATGCCAGTAGGAGAAAT CTCAGCTCCACAACCCAAGCAACATTTGTATGGATTTAAGAGTATTT TAAGAAGACATACTGCTTGATTTTAATACATTGATCAGGCCATCCAG GACACCACGATTCTCCCAAAGTACCTTGAACTCTTAGTGATTGAGA CTCAAAAAAACAAAAAAGACTTGAGACAATGTTTTCTTCAACATGCT CCAAATATAAGACATTTGTTTGCTGTACAGAAAGTATCACAAATGGA ATATATCAGTACCTCTCAAGCTAGTGTTTCTAGCTAAATAAATGGGT GTATATAATTTTATGGTGGAAAAGAACTGTACTGTCTGTTATGATTTC CTTCAATGTGCATAATGATAAAATAAATAATTTTAATATTCTTTTGTTT CCATGGTTACCTGACCTAAATTAGATAAATTGTAGGGCTTTAGCTTT CTTATTTTTGTCAAAAGTTGGTGTTGACATACATTCCCTCTAATTTGA ACTGGTATTGTTTACGTTTGATACAACATTAAGGAATTTGATGATTTT CATTTCATGAAAATGACATTAAATGCAATAATTTTACTTATCATAAAC ATTTTGTACATCATTATTTTCTTTTGGATTAGTGTTGTCATACATGTA ATTTATATCACATGTATAAACATTGAAAATCAACTAAAATGACATTTG TTCTACATTATAACTTGTGAGTTTCAAGAACTAGTATTAGTAGTTTTT TCCTTTCATTATAGATTGTAAGATGTTGTGGTATCTTTGAGTGCCTTA GTTCTTCCTTCCTCCCAAAAGCCATTAATTTACAAATGCTTAAAGCC ATCAGGTCAAATATTTCAAAGCCTTTAGATGATTTCTGTACTAGCTTT AGATGTCTGACGTTATGTAGGTTACCTGTGTTCTTGGCTAGGAAAA CATTATTGATTCATTAAATCATAAAGGTGGGAATAATAATCTTTTATT TATGATGTTGATTGGCTCCAAAATAGTCTTAAGGAAATAAATACTGG GTCTGTAGGGGAAAAGTAGACTTCATAGTTTAAAATCCCATTAACCT TTTCACCGCAGTTGAAATGCATCCAGCCTGATTTTCCTATCATTTTG GAATTTTTAAGGATTTTTACTTTCTTAAGTTACTGCCTAGAATCAACA TTCGGTGAGATTTTGAAATGTCATAGATACTGTACAGGCCAAACCTT ACTAATTTATTTTACTTAAAGTGATATTTTATAGAAAAATCATAAGTTA TACAATGAGAACCCTTTAAGCCCTTAGCCTAAGCTTTCAGACTAAAT GTGATTATAGAATAAGATGAAAGTTAACTTTGGTACAGAGCTTTTTA TAGCCCCAAATTATATTTCCAGTTATTATATTCATTAGAATTTCTGCT AATAAACTCCCAACTTAAATAGAAAAAAAAAAAAAAAA | EBAG9 transcript variant 2 |
| 5 | ATGGCACAGAATGTTCGATTTTACTACCAGCTCTCACACTCCGCTTC TGTTTCCCCTTCTCTTACGGGCCGCTGCTCAAACCTTTATTTATCAA ATTTAAGTTTTGATTCCCACCATGGCCATCACCCAGTTTCGGTTATT TAAATTTTGTACCTGCCTAGCAACAGTATTCTCATTCCTAAAGAGAT TAATATGCAGATCTGGCAGAGGACGGAAATTAAGTGGAGACCAAAT AACTTTGCCAACTACAGTTGATTATTCATCAGTTCCTAAGCAGACAG ATGTTGAAGAGTGGACTTCCTGGGATGAAGATGCACCCACCAGTGT AAAGATCGAAGGAGGGAATGGGAATGTGGCAACACAACAAAATTCT TTGGAACAACTGGAACCTGACTATTTTAAGGACATGACACCAACTAT TAGGAAAACTCAGAAAATTGTTATTAAGAAGAGAGAACCATTGAATT TTGGCATCCCAGATGGGAGCACAGGTTTCTCTAGTAGATTAGCAGC TACACAAGATCTGCCTTTTATTCATCAGTCTTCTGAATTAGGTGACT TAGATACCTGGCAGGAAAATACCAATGCATGGGAAGAAGAAGAAGA 6TGCAGCCTGGCAAGCAGAAGAAGTTCTGAGACAGCAGAAACTAGC AGACAGAGAAAAGAGAGCAGCCGAACAACAAAGGAAGAAAATGGA AAAGGAAGCACAACGGCTAATGAAGAAGGAACAAAACAAAATTGGT GTGAAACTTTCATAACACATGTTCAAATTTTATCATGCCAGTAGGAG AAATCTCAGCTCCACAACCCAAGCAACATTTGTATGGATTTAAGAGT ATTTTAAGAAGACATACTGCTTGATTTTAATACATTGATCAGGCCAT CCAGGACACCACGATTCTCCCAAAGTACCTTGAACTCTTAGTGATT GAGACTCAAAAAAACAAAAAAGACTTGAGACAATGTTTTCTTCAACA TGCTCCAAATATAAGACATTTGTTTGCTGTACAGAAAGTATCACAAA TGGAATATATCAGTACCTCTCAAGCTAGTGTTTCTAGCTAAATAAAT GGGTGTATATAATTTTATGGTGGAAAAGAACTGTACTGTCTGTTATG ATTTCCTTCAATGTGCATAATGATAAAATAAATAATTTTAATATTCTTT TGTTTCCATGGTTACCTGACCTAAATTAGATAAATTGTAGGGCTTTA GCTTTCTTATTTTTGTCAAAAGTTGGTGTTGACATACATTCCCTCTAA TTTGAACTGGTATTGTTTACGTTTGATACAACATTAAGGAATTTGATG ATTTTCATTTCATGAAAATGACATTAAATGCAATAATTTTACTTATCAT AAACATTTTGTACATCATTATTTTCTTTTGGATTAGTGTTGTCATACA TGTAATTTATATCACATGTATAAACATTGAAAATCAACTAAAATGACA TTTGTTCTACATTATAACTTGTGAGTTTCAAGAACTAGTATTAGTAGT TTTTTCCTTTCATTATAGATTGTAAGATGTTGTGGTATCTTTGAGTGC CTTAGTTCTTCCTTCCTCCCAAAAGCCATTAATTTACAAATGCTTAAA GCCATCAGGTCAAATATTTCAAAGCCTTTAGATGATTTCTGTACTAG CTTTAGATGTCTGACGTTATGTAGGTTACCTGTGTTCTTGGCTAGGA AAACATTATTGATTCATTAAATCATAAAGGTGGGAATAATAATCTTTT ATTTATGATGTTGATTGGCTCCAAAATAGTCTTAAGGAAATAAATACT GGGTCTGTAGGGGAAAAGTAGACTTCATAGTTTAAAATCCCATTAAC CTTTTCACCGCAGTTGAAATGCATCCAGCCTGATTTTCCTATCATTT TGGAATTTTTAAGGATTTTTACTTTCTTAAGTTACTGCCTAGAATCAA CATTCGGTGAGATTTTGAAATGTCATAGATACTGTACAGGCCAAACC TTACTAATTTATTTTACTTAAAGTGATATTTTATAGAAAAATCATAAGT TATACAATGAGAACCCTTTAAGCCCTTAGCCTAAGCTTTCAGACTAA ATGTGATTATAGAATAAGATGAAAGTTAACTTTGGTACAGAGCTTTT TATAGCCCCAAATTATATTTCCAGTTATTATATTCATTAGAATTTCTG CTAATAAACTCCCAACTTAAATAGAAAAAAAAAAAAAAAA | EBAG9 transcript variant 3 |
| 6 | TGCGACTGGGAGCGGGACCCAGGCGTGCAGCATTCGCCATGCTCC GCTCACGCGTGGGAGACTGGGCTGTGGGGTACCGGCCCGGAAAG CACGCAGCCTCCAAAGCCGCCTTCCTCAGGGAAATTTGCGTGACCT TACTGCCCTCCGTCTACAGGCCTTGTACCTCTCCAGGCCGATTTTT CCACAATTTAAATCTCAGTTCACCTGGTATCCAGCTCCAGCAACTTA GAGCGTTTCACGTCACGCCGGGCGCCAGGCGTCGGCTTGTATAAC CTGAAAACGCTCCTGTTTTTCTCATCTGTGCAGTGGGTATGATTTTT TTTTCATCAACAAATTTCACGTGGGTAATCTGAAATGAAACCACTTA AGTTATGAAACTCTTTCCTTTTGAGTTATTCTGGAGACCTTACTCCG CCTTCGGAACCGCCCCAGTGGTGTCACATACTTGAGGGCCTGACT CTCGGTTGCCAGACATGGCACAGAATGTTCGATTTTACTACCAGCT CTCACACTCCGCTTCTGTTTCCCCTTCTCTTACGGGCCGCTGCTCA AACCTTTATTTATCAAATTTAAGTGAGTTCAGGTTTTGATTCCCACCA TGGCCATCACCCAGTTTCGGTTATTTAAATTTTGTACCTGCCTAGCA | EBAG9 transcript variant X1 |
| | ACAGTATTCTCATTCCTAAAGAGATTAATATGCAGATCTGGCAGAGG ACGGAAATTAAGTGGAGACCAAATAACTTTGCCAACTACAGTTGATT ATTCATCAGTTCCTAAGCAGACAGATGTTGAAGAGTGGACTTCCTG GGATGAAGATGCACCCACCAGTGTAAAGATCGAAGGAGGGAATGG GAATGTGGCAACACAACAAAATTCTTTGGAACAACTGGAACCTGAC TATTTTAAGGACATGACACCAACTATTAGGAAAACTCAGAAAATTGT TATTAAGAAGAGAGAACCATTGAATTTTGGCATCCCAGATGGGAGC ACAGGTTTCTCTAGTAGATTAGCAGCTACACAAGATCTGCCTTTTAT TCATCAGTCTTCTGAATTAGGTGACTTAGATACCTGGCAGGAAAATA CCAATGCATGGGAAGAAGAAGAAGATGCAGCCTGGCAAGCAGAAG AAGTTCTGAGACAGCAGAAACTAGCAGACAGAGAAAAGAGAGCAG CCGAACAACAAAGGAAGAAAATGGAAAAGGAAGCACAACGGCTAAT GAAGAAGGAACAAAACAAAATTGGTGTGAAACTTTCATAACACATGT TCAAATTTTATCATGCCAGTAGGAGAAATCTCAGCTCCACAACCCAA GCAACATTTGTATGGATTTAAGAGTATTTTAAGAAGACATACTGCTT GATTTTAATACATTGATCAGGCCATCCAGGACACCACGATTCTCCCA AAGTACCTTGAACTCTTAGTGATTGAGACTCAAAAAAACAAAAAAGA CTTGAGACAATGTTTTCTTCAACATGCTCCAAATATAAGACATTTGTT TGCTGTACAGAAAGTATCACAAATGGAATATATCAGTACCTCTCAAG CTAGTGTTTCTAGCTAAATAAATGGGTGTATATAATTTTATGGTGGA AAAGAACTGTACTGTCTGTTATGATTTCCTTCAATGTGCATAATGAT AAAATAAATAATTTTAATATTCTTTTGTTTCCATGGTTACCTGACCTA AATTAGATAAATTGTAGGGCTTTAGCTTTCTTATTTTTGTCAAAAGTT GGTGTTGACATACATTCCCTCTAATTTGAACTGGTATTGTTTACGTT TGATACAACATTAAGGAATTTGATGATTTTCATTTCATGAAAATGACA TTAAATGCAATAATTTTACTTATCATAAA | |
| 7 | GFTFSTSG | CXCR5 H-CDR1 |
| 8 | ISSSSGFV | CXCR5 H-CDR2 |
| 9 | ARSEAAF | CXCR5 H-CDR3 |
| 10 | KSRLSRMGITP | CXCR5 L-CDR1 |
| - | RMS | CXCR5 L-CDR2 |
| 11 | AQFLEYPPT | CXCR5 L-CDR3 |
| 12 | EVQLVESGGGLVQPGGSLRLSCAASGFTFSTSGMNWFRQAPGKGLE WVSYISSSSGFVYADSVKGRFTISRDNAQNSLYLQMNSLRAEDTAVYY CARSEAAFWGQGTLVTVSS | CXCR5 Humanized VH |
| 13 | EVQLVESGGGLVQPGKSLKLSCSASGFTFSTSGMHWFRQAPGKGLD WVAYISSSSGFVYADAVKGRFTISRDNAQNTLYLQLNSLKSEDTAIYYC ARSEAAFWGQGTLVTVSS | CXCR5 Rat VH |
| 14 | DIVLTQSPRSLPVTPGEPASISCRSSKSRLSRMGITPLNWYLQKPGQS PQLLIYRMSNRASGVPDRFSGSGSGTDFTLKISKVETEDVGVYYCAQF LEYPPTFGSGTKLEIK | CXCR5 Humanized VL |
| 15 | DIVLTQAPRSVSVTPGESASISCRSNKSRLSRMGITPLNWYLQKPGKS PQLLIYRMSNLASGVPDRFSGSGSETDFTLKISKVETEDVGVYYCAQF LEYPPTFGSGTKLEIK | CXCR5 Rat VL |
| 16 | GFTFSRYW | BCMA H-CDR1a |
| 17 | INPSSSTI | BCMA H-CDR2a |
| 18 | ASLYYDYGDAYDY | BCMA |
| | | H-CDR3a |
| 19 | QSVESN | BCMA L-CDR1a |
| - | SAS | BCMA L-CDR2a |
| 20 | QQYNNYPLT | BCMA L-CDR3a |
| 21 | RYWFS | BCMA H-CDR1b |
| 22 | EINPSSSTINYAPSLKDK | BCMA H-CDR2b |
| 23 | SLYYDYGDAYDYW | BCMA H-CDR3b |
| 24 | KASQSVESNVA | BCMA L-CDR1b |
| 25 | SASLRFS | BCMA L-CDR2b |
| 26 | QQYNNYPLTFG | BCMA L-CDR3b |
| 27 | EVQLVESGGGLVQPGGSLRLSCAASGFTFSRYWFSWVRQAPGKGLV WVGEINPSSSTINYAPSLKDKFTISRDNAKNTLYLQMNSLRAEDTAVYY CASLYYDYGDAYDYWGQGTLVTVSS | BCMA VH |
| 28 | EIVMTQSPATLSVSPGERATLSCKASQSVESNVAWYQQKPGQAPRALI YSASLRFSGIPARFSGSGSGTEFTLTISSLQSEDFAVYYCQQYNNYPLT FGAGTKLELK | BCMA VL |
| 29 | GSTSGSGKPGSGEGSTKG | Whitlow linker |
| 30 | SSGGGGSGGGGSGGGGS | Gly-Ser linker |
| 31 | PAEPKSPDKTHTCPPCPAPPVAGPSVFLFPPKPKDTLMIARTPEVTCV VVDVSHEDPEVKFNWYVDGVEVHNAKTKPREEQYNSTYRVVSVLTVL HQDWLNGKEYKCKVSNKALPAPIEKTISKAKGQPREPQVYTLPPSRDE LTKNQVSLTCLVKGFYPSDIAVEWESNGQPENNYKTTPPVLDSDGSFF LYSKLTVDKSRWQQGNVFSCSVMHEALHNHYTQKSLSLSPGKKDPK | IgG1 spacer |
| 32 | PAEPKSPDKTHTCPPCPAPPVAGPSVFLFPPKPKDTLMIARTPEVTCV VVDVSHEDPEVKFNWYVDGVEVHNAKTKPREEQYNSTYRVVSVLTVL HQDWLNGKEYKCKVSNKALPAPIEKTISKAKGQPREPQVYTLPPSRDE LTKNQVSLTCLVKGFYPSDIAVEWESNGQPENNYKTTPPVLDSDGSFF LYSKLTVDKSRWQQGNVFSCSVMHEALHNHYTQKSLSSLSPGKK | IgG1Δ spacer |
| 33 | ESKYGPPCPPCPAPEFEGGPSVFLFPPKPKDTLMISRTPEVTCVVVDV SQEDPEVQFNWYVDGVEVHNAKTKPREEQFNSTYRVVSVLTVLHQD WLNGKEYKCKVSNKGLPSSIEKTISKAKGQPREPQVYTLPPSQEEMTK NQVSLTCLVKGFYPSDIAVEWESNGQPENNYKTTPPVLDSDGSFFLYS RLTVDKSRWQEGNVFSCSVMHEALHNHYTQKSLSLSLGK | IgG4 (HI-CH2-CH3) spacer |
| 34 | ESKYGPPCPPCPGQPREPQVYTLPPSQEEMTKNQVSLTCLVKGFYPS DIAVEWESNGQPENNYKTTPPVLDSDGSFFLYSRLTVDKSRWQEGNV FSCSVMHEALHNHYTQKSLSLSLGK | IgG4 (HI-CH3) spacer |
| 35 | ESKYGPPCPPCP | IgG4 (HI) spacer |
| 36 | IYIWAPLAGTCGVLLLSLVITLYC | transmembran e domain CD8α |
| 37 | FWVLVVVGGVLACYSLLVTVAFIIFWV | transmembran e domain |
| | | CD28 |
| 38 | KRGRKKLLYIFKQPFMRPVQTTQEEDGCSCRFPEEEEGGCEL | Co-stimulatory domain 4-1BB |
| 39 | RSKRSRLLHSDYMNMTPRRPGPTRKHYQPYAPPRDFAAYRSL | Co-stimulatory domain CD28 |
| 40 | LRVKFSRSADAPAYQQGQNQLYNELNLGRREEYDVLDKRRGRDPEM GGKPRRKNPQEGLYNELQKDKMAEAYSEIGMKGERRRGKGHDGLYQ GLSTATKDTYDALHMQALPPR | Activation domain CD3 zeta (4-1BB) or (CD28) |
| 41 | MDFQVQIFSFLLISASVIMSR | Lkappa Leader |
| 42 | ATGGATTTCCAGGTGCAGATCTTCAGCTTCCTGCTGATCTCCGCCA GCGTGATCATGAGCCGC | Lkappa Leader |
| 43 | GGCAGCACAAGCGGCTCTGGCAAACCTGGATCTGGCGAGGGCAG CACCAAGGGC | Humanized Whitlow |
| 44 | GGCAGCACCAGCGGCTCCGGCAAGCCTGGCTCTGGCGAGGGCAG CACAAAGGGA | Rat Whitlow |
| 45 | CCCGCCGAGCCCAAGAGCCCCGACAAGACCCATACCTGCCCTCCA TGTCCTGCCCCTCCAGTGGCTGGCCCTAGCGTGTTCCTGTTCCCC CCAAAGCCCAAGGACACCCTGATGATCGCCCGGACCCCTGAAGTG ACCTGCGTGGTGGTGGATGTGTCCCACGAGGATCCCGAAGTGAAG TTCAATTGGTACGTGGACGGCGTGGAAGTGCACAACGCCAAGACC AAGCCCAGAGAGGAACAGTACAACAGCACCTACCGGGTGGTGTCT GTGCTGACCGTGCTGCATCAGGACTGGCTGAACGGCAAAGAGTAC AAGTGCAAGGTGTCCAACAAGGCCCTGCCTGCCCCCATCGAGAAA ACCATCTCCAAGGCCAAGGGACAGCCCCGCGAGCCCCAGGTGTAC ACACTGCCTCCAAGCAGGGACGAGCTGACCAAGAACCAGGTCAGC CTGACCTGCCTGGTCAAAGGCTTCTATCCCAGCGACATCGCCGTG GAGTGGGAGAGCAATGGGCAGCCGGAGAACAACTACAAGACCACG CCTCCCGTGCTGGACTCCGACGGCTCCTTCTTCCTCTACAGCAAGC TCACCGTGGACAAGAGCAGGTGGCAGCAGGGGAACGTCTTCTCAT GCTCCGTGATGCATGAGGCTCTGCACAACCACTACACGCAGAAGA GCCTCTCCCTGTCTCCGGGTAAAAAAGATCCCAAA | Humanized IgG1 spacer |
| 46 | CCTGCCGAGCCTAAGAGCCCCGACAAGACCCACACCTGTCCCCCT TGTCCTGCCCCTCCAGTGGCTGGCCCTAGCGTGTTCCTGTTCCCC CCAAAGCCCAAGGATACCCTGATGATCGCCCGGACCCCCGAAGTC ACATGCGTGGTGGTGGACGTGAGCCACGAAGACCCTGAGGTCAAG TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAAA CCATCTCCAAAGCCAAAGGGCAGCCCCGAGAACCACAGGTGTACA CCCTGCCCCCATCCCGGGATGAGCTGACCAAGAACCAGGTCAGCC TGACCTGCCTGGTCAAAGGCTTCTATCCCAGCGACATCGCCGTGG AGTGGGAGAGCAATGGGCAGCCGGAGAACAACTACAAGACCACGC CTCCCGTGCTGGACTCCGACGGCTCCTTCTTCCTCTACAGCAAGCT CACCGTGGACAAGAGCAGGTGGCAGCAGGGGAACGTCTTCTCATG CTCCGTGATGCATGAGGCTCTGCACAACCACTACACGCAGAAGAG CCTCTCCCTGTCTCCGGGTAAAAAAGATCCCAAA | Rat IgG1 spacer |
| 47 | CCAGGTGTCCCTGACCTGCCTCGTGAAGGGCTTCTACCCCTCCGA TATCGCCGTGGAATGGGAGAGCAATGGCCAGCCCGAGAACAACTA CAAGACCACCCCCCCTGTGCTGGACAGCGACGGCTCATTCTTCCT GTACAGCAAGCTGACAGTGGACAAGAGCCGGTGGCAGCAGGGCAA CGTGTTCAGCTGCAGCGTGATGCACGAGGCTCTGCACAACCACTA CACCCAGAAGTCCCTGAGCAGCCTGAGCCCAGGCAAGAAG | Humanized IgG1Δ spacer |
| 48 | CCTGCCGAGCCTAAGAGCCCCGACAAGACCCACACCTGTCCCCCT TGTCCTGCCCCTCCAGTGGCTGGCCCTAGCGTGTTCCTGTTCCCC CCAAAGCCCAAGGATACCCTGATGATCGCCCGGACCCCCGAAGTC ACATGCGTGGTGGTGGACGTGAGCCACGAAGACCCTGAGGTCAAG TTCAACTGGTACGTGGACGGCGTGGAGGTGCATAATGCCAAGACA AAGCCGCGGGAGGAGCAGTACAACAGCACGTACCGTGTGGTCAGC GTCCTCACCGTCCTGCACCAGGACTGGCTGAATGGCAAGGAGTAC AAGTGCAAGGTCTCCAACAAAGCCCTCCCAGCCCCCATCGAGAAAA CCATCTCCAAAGCCAAAGGGCAGCCCCGAGAACCACAGGTGTACA CCCTGCCCCCATCCCGGGATGAGCTGACCAAGAA | Rat IgG1Δ spacer |
| 49 | ATCTACATCTGGGCCCCTCTGGCCGGCACCTGTGGCGTGCTGCTG CTGTCTCTCGTGATCACACTGTACTGC | transmembran e domainCD8α |
| 50 | TTTTGGGTGCTGGTGGTGGTTGGTGGAGTCCTGGCTTGCTATAGCT TGCTAGTAACAGTGGCCTTTATTATTTTCTGGGTG | transmembran e domainCD28 |
| 51 | AAGCGGGGCAGAAAGAAGCTGCTGTACATCTTCAAGCAGCCCTTCA TGCGGCCCGTGCAGACCACCCAGGAAGAGGACGGCTGCTCCTGC AGATTCCCCGAGGAAGAAGAAGGCGGCTGCGAGCTG | Co-stimulatory domain4-1BB |
| 52 | AGGAGTAAGAGGAGCAGGCTCCTGCACAGTGACTACATGAACATG ACTCCCCGCCGCCCCGGGCCCACCCGCAAGCATTACCAGCCCTAT GCCCCACCACGCGACTTCGCAGCCTATCGCTCCCTG | Co-stimulatory domainCD28 |
| 53 | CTGCGCGTGAAGTTTTCTAGAAGCGCCGACGCCCCTGCCTACCAG CAGGGCCAGAACCAGCTGTACAACGAGCTGAACCTGGGCAGACGG GAAGAGTACGACGTGCTGGATAAGCGGAGAGGCCGGGACCCTGA GATGGGCGGCAAGCCTAGAAGAAAGAACCCCCAGGAAGGCCTGTA TAACGAACTGCAGAAAGACAAGATGGCCGAGGCCTACAGCGAGAT CGGAATGAAGGGCGAGCGGAGAAGAGGCAAGGGCCACGATGGAC TGTACCAGGGCCTGAGCACCGCCACCAAGGACACCTATGACGCCC TGCACATGCAGGCTCTGCCCCCCAGATAA | Activation domainCD3 zeta (4-1 BB) |
| 54 | AGAGTGAAGTTCAGCAGGAGCGCAGACGCCCCCGCGTACCAGCAG GGCCAGAACCAGCTCTATAACGAGCTCAATCTAGGACGAAGAGAG GAGTACGATGTTTTGGACAAGAGACGTGGCCGGGACCCTGAGATG GGGGGAAAGCCGAGAAGGAAGAACCCTCAGGAAGGCCTGTACAAT GAACTGCAGAAAGATAAGATGGCGGAGGCCTACAGTGAGATTGGG ATGAAAGGCGAGCGCCGGAGGGGCAAGGGGCACGATGGCCTTTA CCAGGGTCTCAGTACAGCCACCAAGGACACCTACGACGCCCTTCA CATGCAGGCCCTGCCCCCTCGCTGA | Activation domainCD3 zeta (CD28) |
| 55 | TAAATAACCGAAACTGGGTGA | EBAG9 targeting 3 |
| 56 | TAGGAATGAGAATACTGTTGC | EBAG9 targeting 4 |
| 57 | CTTAATTTCCGTCCTCTGCCA | EBAG9 targeting 5 |
| 58 | TTTGGTCTCCACTTAATTTCC | EBAG9 targeting 6 |
| 59 | TTCAACATCTGTCTGCTTAGG | EBAG9 targeting 7 |
| 60 | AAGTCCACTCTTCAACATCTG | EBAG9 targeting 8 |
| 61 | ATCCCAGGAAGTCCACTCTTC | EBAG9 targeting 9 |
| 62 | TACTAGAGAAACCTGTGCTCC | EBAG9 targeting 10 |

### DETAILED DESCRIPTION OF THE INVENTION

The invention relates to engineered T cells, modified by inhibiting EBAG function, thereby enhancing the cytolytic activity of the cells. The enhancement of functional avidity of either TCR-T cells, or T cells equipped with CARs, will lead to a higher cytolytic capacity and thus, to a higher efficiency against tumors. There are specific advantages associated with the modified T cells endowed with a higher cytolytic capacity (engineered T cell) as described herein.

In many cases, tumor patients have received several previous lines of treatment regimens, leading to strong myelosuppression and subsequently, to the inefficiency to mobilize peripheral lymphocytes as starting material for ex vivo cultivation and transduction. This process is referred to as autologous transplantation. Conferring low numbers of T cells with enhanced cytolytic competence helps to bypass quantitative problems, because on a single cell basis engineered T cells perform much stronger. In other words, T cell engineering improves and facilitates the manufacturing process. Another aspect of the manufacturing process is the duration of the ex vivo expansion phase.

Provided that T cells are endowed with a higher cytolytic capacity, in vitro expansion can be terminated earlier leading to a reduction in production costs, an earlier availability of therapy, and a less differentiated T cell population. The latter aspect is of importance because the more advanced the differentiation state of T cells from in vitro cultures is, the shorter is their persistence in vivo.

In allogeneic bone marrow transplantation, a severe risk factor is the development of a graft-versus-host-disease (GvHD). This disease or syndrome strongly correlates with the number of transplanted T cells. Endowing T cells with improved cytolytic capacity alleviates the need for higher T cell numbers and thus, low numbers of engineered T cells confer the same therapeutic efficiency without mediating GvHD.

The threshold level for activation and effector function of engineered T cells is lower. It follows that weak tumor antigens, including neoantigens, self-antigens and minor histocompatibility antigens, and even low numbers of antigens displayed can already trigger effector molecule release from engineered T cells, either carrying a TCR-only, or those equipped with a CAR.

To prevent tumor relapse, it is of substantial advantage to generate memory T cells following a first round of tumor eradication. However, some tumor cells may escape T cell attack and eventually, start to proliferate later. The inventors have however shown that the cytolytic capacity correlates with the formation of antigen-specific memory T cells. Improved antitumor effects of ATT without effectuating more cytokine release which can be harmful due to inducing inflammatory conditions. Considering the above, the inventors have presented novel means for improving T cell cytolytic activity based on the EBAG9 inhibition described in more detail below. The following terms and definitions are provided for greater clarity. All terms maintain their common meaning as understood by a skilled person unless otherwise defined.

### Cytotoxic T cells and cytolytic activity:

A cytotoxic T cell (also known as TC, cytotoxic T lymphocyte, CTL, T-killer cell, cytolytic T cell, T-cell or killer T cell, and as may be used interchangeably herein) is a T cell (a type of white blood cell) that has cytolytic activity against e.g. cancer cells. In some embodiments, the cytolytic activity can be associated with a CD8+ and/or a CD4+ T cell. Both T cell subpopulations can generate and release lytic granule content, that is cytolytic enzymes such as granzymes.

The term "cytolytic" refers to a T cell's capacity to kill target cells by the release of lytic granule content, the latter are also referred to as secretory lysosomes.

As a central element of the adaptive immune response, T cells are capable of eliminating infections and transformed tumor cells. CD8⁺ T cells can mature into cytotoxic T lymphocytes (CTLs) and are primarily involved in the destruction of infected or transformed cells by releasing cytolytic granules into the immunological synapse. These granules include perforin and granzymes that are released in the Ca²⁺-dependent regulated secretion pathway and induce apoptosis within the target cells.

As soon as the CTL recognizes and binds its target cell, secretory lysosomes move and cluster around the microtubule organizing center. After membrane fusion, perforin and granzymes are released into the immunological synapse. Perforin is a pore-forming molecule capable of membrane permeabilization that is important for the entry of granzymes into the target cell cytosol. Within the target cell, programmed cell death pathways are initiated by granzymes. Under some activation conditions, CD4+ T cells equipped with the CAR receptor can acquire cytolytic properties by granzyme release as well. Thus, they can be manipulated by EBAG9 silencing in the same manner as CD8+ cytolytic T cells.

Granzyme A induces a caspase-independent apoptosis characterized by the generation of single-stranded DNA nicks. Due to the action of granzyme A, there is a loss of the mitochondrial inner membrane potential, leading to the release of reactive oxygen species (ROS). As a consequence, DNA single-strand nicks are induced. On the contrary, in addition to activating a caspase-independent cell death program, granzyme B is able to induce caspase-dependent apoptosis by cleaving and activating the caspases 3, 7, 8 and 10 as well as several of their downstream substrates. Furthermore, granzyme B induces ROS production and the release of cytochrome c from the mitochondria.

### EBAG9 and its role in the regulated effector molecule secretion:

Protein transfer from the trans Golgi network (TGN) to secretory lysosomes is highly regulated. The inventors have demonstrated the presence of regulatory proteins such as the Estrogen receptor-binding fragment-associated antigen 9 (EBAG9), which is a negative regulator of the Ca²⁺-dependent regulated secretion of effector molecules.

Human EBAG9 comprises 213 amino acids and exhibits a domain structure. Through a C-terminal coiled-coiled structure, human EBAG9 forms homo-oligomers with an N-terminal located transmembrane domain. EBAG9 is an estrogen-inducible protein that is expressed in most tissues.

The inventors have demonstrated that a loss of EBAG9 enhances the cytolytic activity of CTLs *in vivo* by facilitating the release of granzyme A-containing secretory lysosomes from CD8⁺ T cells.

Mechanistically, EBAG9 interacts with the y2-subunit of AP-1 and inhibits the AP-1 activity clathrin-coated vesicle formation. Furthermore, EBAG9 was also identified as an interaction partner of snapin and BLOS2, which are subunits of the lysosome-related organelles complex-1 (BLOC-1). In the secretory pathway, BLOC-1 regulates protein sorting from the endosome to the secretory lysosomes. Thus, EBAG9 negatively regulates the vesicle transfer from the TGN to the secretory lysosomes and is an attractive target to increase the cytolytic activity of adoptively transferred T cells.

The net effect of the EBAG9-imposed transport regulation is an inhibition of secretory lysosome maturation, involving transport and fusion processes of carriers that contain pro-forms of proteolytic enzymes. Alternatively, the re-uptake of secreted lytic granule content may be inhibited by EBAG9 resulting in less availability of recycled cytolytically active granzymes.

EBAG9 was identified as an estrogen-responsive gene. Regulation of transcription by estrogen is mediated by estrogen receptor, which binds to the estrogen-responsive element found in the 5'-flanking region of this gene. The encoded protein is a tumor-associated antigen that is expressed at high frequency in a variety of cancers. Alternate splicing results in multiple transcript variants. A pseudogene of this gene has been defined on chromosome 10.

In some embodiments, the present invention refers preferably to the estrogen receptor binding site associated antigen 9 (EBAG9) of Homo sapiens (human) according to Gene ID: 9166 of the NCBI database. The gene is located on chromosome 8. EBAG9 may also be known as EB9 or PDAF.

The transcript variants according to SEQ ID NO 3-6 may therefore be targeted using RNAi in order to inhibit EBAG9. A skilled person is capable of designing appropriate means, i.e. antisense, siRNA, miRNA or shRNA based on the target sequence of interest. Example EBAG9 transcripts are selected, without limitation from, NM_004215.5 (EBAG9), transcript variant 1, mRNA, SEQ ID NO 3, NM_198120.2 (EBAG9), transcript variant 2, mRNA, SEQ ID NO 4, NM_001278938.1 (EBAG9), transcript variant 3, mRNA, SEQ ID NO 5, XM_017013960.1 (EBAG9), transcript variant X1, mRNA, SEQ ID NO 6.

Preferred methods for assessing EBAG9 inhibition relate to those disclosed herein and include methods for assessing cytolytic activity of CTLs, either *in vivo* or *in vitro* by assessing quantitatively or semi-quantitatively the release of granzyme A-containing secretory lysosomes from e.g. CD8⁺ T cells. Suitable protocols are disclosed below, as applied in the in vitro examples in the present application.

### RNA interference (RNAi):

RNAi is a post-transcriptionally mediated gene silencing mechanism that is triggered by double-stranded RNA (dsRNA) to induce sequence-specific translational repression or mRNA degradation. Historically, RNAi was known by other names, including co-suppression, post-transcriptional gene silencing (PTGS), and quelling.

In the nucleus, the micro RNA (miRNA) genes are transcribed into 500-3000 nucleotide pri-miRNAs by action of the RNA polymerase II. These pri-miRNAs are capped and polyadenylated. In addition, pri-miRNA contain one or multiple stem-loop sequences and are cleaved by the Drosha-DGCR8 complex to 60-100 nucleotide double-stranded pre-miRNA hairpin structures. Ran GTPase and Exportin-5 mediate the export of pre-miRNAs from the nucleus into the cytoplasm. There, they are further processed by an RNase III enzyme called Dicer to an imperfect duplex structure of 22 nucleotides. One of the strands resembles the mature miRNA that binds to Argonaut (Ago) proteins and is incorporated in the RNA-induced silencing complex (RISC). As a consequence of RISC binding, mRNA degradation or repression of protein translation is induced. The fate of the target mRNA molecules depends on the grade of complementarity between the target mRNA molecule and miRNA but is also affected by the incorporated Ago protein. While incorporation of Ago 2 leads to direct cleavage of the target mRNA, the other Ago proteins negatively impact mRNA stability or attenuate translation.

For the engineered knockdown of specific targets, several dsRNA molecules can be used that enter the RNAi pathway at different points. Transfection with small interfering RNA (siRNA) molecules that enter the RNAi pathway in the cytosol only leads to transient protein knockdown. For long-term manipulation of gene expression, it is necessary to deliver dsRNA molecules by integrating gene transfer vectors. Therefore, short hairpin RNA (shRNA) or miRNA molecules can be applied.

Both enter the RNAi pathway in the nucleus and are then processed to siRNA-like molecules. shRNAs mimic the pre-miRNA stem-loop structure. Their expression is driven by the strong RNA polymerase III promoters that lead to high-level expression and stable gene knockdown. However, shRNA overexpression was shown to mediate toxicity by saturation of the miRNA processing pathways.

Another possibility is the application of artificial miRNAs to mediate a stable knockdown within primary T cells. These artificial miRNAs are analogous to the pri-miRNA and, therefore, are a step further towards mimicking natural miRNA biology. This has several advantages for potential clinical applications. Most importantly, using the endogenous miRNA processing machinery does not trigger cellular self-defense mechanisms such as interferon induction.

In addition, artificial miRNAs are transcribed by RNA polymerase II promoters comparable to most of the natural miRNAs. These promoters mediate regulated and tissue-specific expression and further enable the simultaneous expression of selector or therapeutic transgenes. Moreover, it is possible to combine multiple miRNAs in one expression cassette to target regions in the same or different mRNAs, therefore, gaining an additive effect in target downregulation. Such target down-regulation is referred to herein as e.g. knock-down, silencing, or RNA interference.

### CRISPR engineering:

In some embodiments, inhibiting EBAG9 comprises genetic modification of the T cell genome by disrupting the expression and/or sequence of the EBAG9 gene by CRISPR. In further embodiments of the invention, CRISPR-mediated insertion of the CAR or TCR encoding nucleic acid may be employed.

CRISPR is an abbreviation of Clustered Regularly Interspaced Short Palindromic Repeats and is a family of DNA sequences in bacteria. The sequences contain snippets of DNA from viruses that have attacked the bacterium. These snippets are used by the bacterium to detect and destroy DNA from further attacks by similar viruses. These sequences play a key role in a bacterial defense system and form the basis of a technology known as CRISPR/Cas that effectively and specifically changes genes within organisms.

Sequences of the CRISPR loci are transcribed and processed into CRISPR RNAs (crRNAs) which, together with a trans-activating crRNAs (tracrRNAs), complex with CRISPR-associated (Cas) proteins to dictate specificity of DNA cleavage by Cas nucleases through Watson-Crick base pairing between nucleic acids (Wiedenheft, B et al (2012). Nature 482: 331-338; Horvath, P et al (2010). Science 327: 167-170; Fineran, PC et a. (2012). Virology 434: 202-209).

It was shown that the three components required for the type II CRISPR nuclease system are the Cas9 protein, the mature crRNA and the tracrRNA, which can be reduced to two components by fusion of the crRNA and tracrRNA into a single guide RNA (sgRNA) and that re-targeting of the Cas9/sgRNA complex to new sites could be accomplished by altering the sequence of a short portion of the gRNA (Garneau, JE et al (2010). Nature 468: 67-71; Deltcheva, E et al. (2011). Nature 471: 602-607, Jinek, M et al (2012) Science 337: 816-821).

CRISPR-Cas systems are RNA-guided adaptive immune systems of bacteria and archaea that provide sequence-specific resistance against viruses or other invading genetic material. This immune-like response has been divided into two classes on the basis of the architecture of the effector module responsible for target recognition and the cleavage of the invading nucleic acid (Makarova KS et al. Nat Rev Microbiol. 2015 Nov; 13(11):722-36.). Class 1 comprises multi-subunit Cas protein effectors and Class 2 consists of a single large effector protein. Both Class 1 and 2 use CRISPR RNAs (crRNAs) to guide a Cas nuclease component to its target site where it cleaves the invading nucleic acids. Due to their simplicity, Class 2 CRISPR-Cas systems are the most studied and widely applied for genome editing. The most widely used CRISPR-Cas system is CRISPR-Cas9. It was demonstrated that the CRISPR/Cas9 system could be engineered for efficient genetic modification in mammalian cells.

In some embodiments of the invention, an RNA guided DNA endonuclease is employed. In the context of the present invention, the term "RNA guided DNA endonuclease" refers to DNA endonucleases that interact with at least one RNA-Molecule. DNA endonucleases are enzymes that cleave the phosphodiester bond within a DNA polynucleotide chain. In case of RNA guided DNA endonuclease the interacting RNA-Molecule may guide the RNA guided DNA endonuclease to the site or location in a DNA where the endonuclease becomes active. In particular, the term RNA guided DNA endonuclease refers to naturally occurring or genetically modified Cas nuclease components or CRISPR-Cas systems, which include, without limitation, multi-subunit Cas protein effectors of class 1 CRISPR-Cas systems as well as single large effector Cas proteins of class 2 systems.

Details of the technical application of CRISPR/Cas systems and suitable RNA guided endonuclease are known to the skilled person and have been described in detail in the literature, as for example by Barrangou R et al. (Nat Biotechnol. 2016 Sep 8;34(9):933-941), Maeder ML et al. (Mol Ther. 2016 Mar;24(3):430-46) and Cebrian-Serrano A et al. (Mamm Genome. 2017; 28(7): 247-261). The present invention is not limited to the use of specific RNA guided endonucleases and therefore comprises the use of any given RNA guided endonucleases in the sense of the present invention suitable for use in the method described herein.

Any RNA guided DNA endonuclease known in the art may be employed in accordance with the present invention. RNA guided DNA endonuclease comprise, without limitation, Cas proteins of class 1 CRISPR-Cas systems, such as Cas3, Cas8a, Cas5, Cas8b, Cas8c, Cas10d, Cse1, Cse2, Csy1, Csy2, Csy3, GSU0054, Cas10, Csm2, Cmr5, Csx11, Csx10 and Csf1; Cas proteins of class 2 CRISPR-Cas systems, such as Cas9, Csn2, Cas4, Cpf1, C2c1, C2c3 and C2c2; corresponding orthologous enzymes/CRISPR effectors from various bacterial and archeal species; engineered CRISPR effectors with for example novel PAM specificities, increased fidelity, such as SpCas9-HF1/eSpCas9, or altered functions, such as nickases. Particularly preferred RNA guided DNA endonuclease of the present invention are Streptococcus pyogenes Cas9 (SpCas9), Staphylococcus aureus Cas9, Streptococcus thermophilus Cas9, Neisseria meningitidis Cas9 (NmCas9), Francisella novicida Cas9 (FnCas9), Campylobacter jejuni Cas9 (CjCas9), Cas12a (Cpf1) and Cas13a (C2C2) (Makarova KS et al. (November 2015). Nature Reviews Microbiology. 13 (11): 722-36).

The definition and explanations provided herein are mainly focused on the SpCas9 Crispr/Cas system. However, the person skilled in the art is aware of how to use alternative Crispr/Cas systems as well as tools and methods that provide or allow the gain of information on the details of such alternative systems.

In accordance with the method of the invention, the RNA guided DNA endonuclease may be introduced as a protein, but alternatively the RNA guided DNA endonuclease may also be introduced in form of a nucleic acid molecule encoding said protein. It will be appreciated that the nucleic acid molecule encodes said RNA guided DNA endonuclease in expressible form such that expression in the cell results in a functional RNA guided DNA endonuclease protein such as Cas9 protein. Means and methods to ensure expression of a functional polypeptide are well known in the art. For example, the coding sequences for the endonuclease may be comprised in a vector, such as for example a plasmid, cosmid, virus, bacteriophage or another vector used conventionally e.g. in genetic engineering.

Furthermore, the method of the present invention comprises introducing into the cell at least one guide RNA. In the context of the present invention, a "guide RNA" refers to RNA molecules interacting with RNA guided DNA endonuclease leading to the recognition of the target sequence to be cleaved by the RNA guided DNA endonuclease. According to the present invention, the term "guide RNA" therefore comprises, without limitiation, target sequence specific CRISPR RNAs (crRNA), trans-activating crRNAs (tracrRNA) and chimeric single guide RNAs (sgRNA).

As described herein, the genes encoding the elements of a CRISPR/Cas system, such as for example Cas9, tracrRNA and crRNA, are typically organized in operon(s). DR sequences functioning together with RNA guided endonuclease such as Cas9 proteins of other bacterial species may be identified by bioinformatic analysis of sequence repeats occurring in the respective Crispr/Cas operons and by experimental binding studies of Cas9 protein and tracrRNA together with putative DR sequence flanked target sequences.

In some embodiments, a chimeric single guide RNA sequence comprising such a target sequence specific crRNA and tracrRNA may be employed. Such a chimeric (ch) RNA may be designed by the fusion of a target specific sequence of 20 or more nucleotides (nt) with a part or the entire DR sequence (defined as part of a crRNA) with the entire or part of a tracrRNA, as shown by Jinek et al. (Science 337:816-821). Within the chimeric RNA a segment of the DR and the tracrRNA sequence are complementary able to hybridize and to form a hairpin structure.

Moreover, the at least one guide RNA of the present invention may also be encoded by a nucleic acid molecule, which is introduced into the cell. The definitions and preferred embodiments recited herein with regard to the nucleic acid molecule encoding the endonuclease equally apply to the nucleic acid molecule encoding these RNAs. Regulatory elements for expressing RNAs are known to one skilled in the art, for example a U6 promoter.

### Cell Therapy or Adoptive Cell Transfer (ATT):

The discovery of IL-2 therapy brought forth the hypothesis that T lymphocytes could be extracted from a patient, expanded in vitro, and re-administered as a cancer therapy. This was achieved in humans in 1988, where researchers used an expanded line of tumor-infiltrating lymphocytes to produce regression in patients with metastatic melanoma. ATT works by resecting a tumor specimen and digesting it into a single-cell suspension. In developing these approaches two new methods of ATT surfaced: TCR, and chimeric antigen receptor (CAR) approaches that are now both well established.

In TCR therapy, typically normal circulating T-cells are isolated from the patient's blood and genetically modified via transfection with a retrovirus vector or transposon to express TCRs against a tumor antigen. Specific TCRs are gathered from human patients or mice immunized against the TAA of interest. A limitation of TCR therapy is that the recombinant TCRs still rely upon MHC recognition to achieve cytotoxicity. Chimeric antigen receptor technology was developed and introduced in 2010 to circumvent this issue. This method again utilizes transfection through a virus vector but introduces an antibody variable region to the T-cell, which will be expressed on the membrane and linked to intracellular signaling domains. The first CARs linked to CD3-zeta and the method was later improved to involve costimulatory receptors such as CD28, OX40, and more, as described in more detail below.

### Exogenous nucleic acids:

An "exogenous nucleic acid", "exogenous genetic element", or "transgenic" nucleic acid or construct relates to any nucleic acid introduced into the cell, which is not a component of the cells "original" or "natural" genome or pool of nucleic acids found "naturally" in an unmodified T cell. Exogenous nucleic acids may be integrated or non-integrated in the genetic material of the target T cell or relate to stably transduced nucleic acids. Delivery of an exogenous nucleic acid may lead to genetic modification of the initial cell through permanent integration of the exogenous nucleic acid molecule in the initial cell. However, delivery of the exogenous nucleic acid may also be transient, meaning that the delivered genetic material for provision of the one or more TF disappears from the cell after a certain time. Nucleic acid molecule delivery and potentially genetic modification of a biological cell, i.e. a T cell, can be performed and determined by a skilled person using commonly available techniques. For example, for detecting genetic modification sequencing of the genome or parts thereof of a cell is possible, thereby identifying if exogenous nucleic acids are present. Alternatively, other molecular biological techniques may be applied, such as the polymerase chain reaction (PCR), to identify/amplify exogenous genetic material. Exogenous nucleic acids may be detected by vector sequences, or parts of vector sequences, e.g. those remaining at the site of genetic modification. In cases where vector sequences (for example vector sequences flanking a therapeutic transgene) can be removed from the genome or do not remain after modification, for example by CRISPR technology, the addition of a transgene may still be detected by sequencing efforts by detecting sequences comprising an exogenous sequence at a "non-natural" position in the genome.

Embodiments of the invention relate to genetically modified cytotoxic T cells comprising one or more exogenous nucleic acid molecules encoding a transgenic antigen-targeting construct. In embodiments of the invention, the exogenous nucleic acid represents a nucleic acid sequence not found naturally in a T cell, based on for example comparisons with an unmodified human genome sequence. In embodiments of the invention, the transgenic antigen-targeting construct is a nucleic acid sequence coding an antigen targeting construct, for which the coding sequence is not found naturally in a T cell, based on for example comparisons with an unmodified human genome sequence. In some embodiments, the sequence is present at a "non-natural location" of the genome. In some embodiments, the targeting construct comprises or consists of a non-naturally occurring sequence, i.e. a synthetic sequence designed and created using recombinant or other molecular biological techniques. According to the invention, EBAG9 activity is inhibited (compared to a control cytotoxic T cell). In some embodiments, the presence of an exogenous nucleic acid sequence can be complemented by a functional assessment of EBAG9 in order to further indicate an EBAG9 inhibition.

### Antigen-targeting construct:

As used herein, the term "antigen targeting construct" or "targeting construct" refers to a transgenic molecule (encoded by an exogenous nucleic acid molecule) capable of directing a T cell to a particular antigen, or group of antigens. An antigen target construct is therefore preferably a chimeric antigen receptor (CAR) or a T cell receptor (TCR). Engineered T cells have emerged as a new stage in precision cancer therapy, through forced expression of these antigen targeting molecules on preferably autologous or donor T cells, they result in specifically recognizing tumor antigens and enhance their therapeutic specificity and efficacy.

### Chimeric Antigen Receptors:

According to the present invention, a chimeric antigen receptor polypeptide (CAR), comprises an extracellular antigen-binding domain, comprising an antibody or antibody fragment that binds a target antigen, a transmembrane domain, and an intracellular domain. CARs are typically described as comprising an extracellular ectodomain (antigen-binding domain) derived from an antibody and an endodomain comprising signaling modules derived from T cell signaling proteins.

In a preferred embodiment, the ectodomain preferably comprises variable regions from the heavy and light chains of an immunoglobulin configured as a single-chain variable fragment (scFv). The scFv is preferably attached to a hinge region that provides flexibility and transduces signals through an anchoring transmembrane moiety to an intracellular signaling domain. The transmembrane domains originate preferably from either CD8α or CD28. In the first generation of CARs the signaling domain consists of the zeta chain of the TCR complex. The term "generation" refers to the structure of the intracellular signaling domains. Second generation CARs are equipped with a single costimulatory domain originated from CD28 or 4-1BB. Third generation CARs already include two costimulatory domains, e.g. CD28, 4-1BB, ICOS or OX40, CD3 zeta. The present invention preferably relates to a second or third generation CAR.

In various embodiments, genetically engineered receptors that redirect cytotoxicity of immune effector cells toward B cells are provided. These genetically engineered receptors referred to herein as chimeric antigen receptors (CARs). CARs are molecules that combine antibody-based specificity for a desired antigen with a T cell receptor-activating intracellular domain to generate a chimeric protein that exhibits an antigen specific cellular immune activity. As used herein, the term, "chimeric," describes being composed of parts of different proteins or DNAs from different origins.

CARs contemplated herein, comprise an extracellular domain (also referred to as a binding domain or antigen-binding domain) that binds to a target antigen, a transmembrane domain, and an intracellular domain, or intracellular signaling domain. Engagement of the antigen binding domain of the CAR on the surface of a target cell results in clustering of the CAR and delivers an activation stimulus to the CAR-containing cell. The main characteristic of CARs are their ability to redirect immune effector cell specificity, thereby triggering proliferation, cytokine production, phagocytosis or production of molecules that can mediate cell death of the target antigen expressing cell in a major histocompatibility complex (MHC) independent manner, exploiting the cell specific targeting abilities of monoclonal antibodies, soluble ligands or cell specific co-receptors.

In various embodiments, a CAR comprises an extracellular binding domain that comprises a humanized antigen-specific binding domain; a transmembrane domain; one or more intracellular signaling domains. In particular embodiments, a CAR comprises an extracellular binding domain that comprises a humanized antigen binding fragment thereof; one or more spacer domains; a transmembrane domain; one or more intracellular signaling domains.

The "extracellular antigen-binding domain" or "extracellular binding domain" are used interchangeably and provide a CAR with the ability to specifically bind to the target antigen of interest. The binding domain may be derived either from a natural, synthetic, semi-synthetic, or recombinant source. Preferred are scFv domains.

"Specific binding" is to be understood as via one skilled in the art, whereby the skilled person is clearly aware of various experimental procedures that can be used to test binding and binding specificity. Methods for determining equilibrium association or equilibrium dissociation constants are known in the art. Some cross-reaction or background binding may be inevitable in many protein-protein interactions; this is not to detract from the "specificity" of the binding between CAR and epitope. "Specific binding" describes binding of an antibody or antigen binding fragment thereof (or a CAR comprising the same) to a target antigen at greater binding affinity than background binding. The term "directed against" is also applicable when considering the term "specificity" in understanding the interaction between antibody and epitope.

An "antigen (Ag)" refers to a compound, composition, or substance that can stimulate the production of antibodies or a T cell response in an animal. In particular embodiments, the target antigen is an epitope of a desired polypeptide. An "epitope" refers to the region of an antigen to which a binding agent binds. Epitopes can be formed both from contiguous amino acids or noncontiguous amino acids juxtaposed by tertiary folding of a protein.

"Single-chain Fv" or "scFv" antibody fragments comprise the VH and VL domains of an antibody, wherein these domains are present in a single polypeptide chain and in either orientation {e.g., VL-VH or VH-VL). Generally, the scFv polypeptide further comprises a polypeptide linker between the VH and VL domains which enables the scFv to form the desired structure for antigen binding. In

preferred embodiments, a CAR contemplated herein comprises antigen-specific binding domain that is an scFv and may be a murine, human or humanized scFv. Single chain antibodies may be cloned from the V region genes of a hybridoma specific for a desired target. scFv can be also obtained from phage display libraries, thus bypassing the tradtional hybridoma technology. In particular embodiments, the antigen-specific binding domain that is a humanized scFv that binds a human target antigen polypeptide.

An illustrative example of a variable heavy chain that is suitable for constructing anti-CXCR5 CARs contemplated herein include, but are not limited to the amino acid sequence set forth in SEQ ID NO: 13. An illustrative example of a variable light chain that is suitable for constructing anti-CXCR5 CARs contemplated herein include, but is not limited to the amino acid sequence set forth in SEQ ID NO: 15.

### Antibodies and antibody fragments:

The CAR comprises an extracellular antigen-binding domain, comprising an antibody or antibody fragment that binds a target polypeptide. Antibodies or antibody fragments of the invention therefore include, but are not limited to polyclonal, monoclonal, bispecific, human, humanized or chimeric antibodies, single chain fragments (scFv), single variable fragments (ssFv), single domain antibodies (such as VHH fragments from nanobodies), Fab fragments, F(ab')₂ fragments, fragments produced by a Fab expression library, anti-idiotypic antibodies and epitope-binding fragments or combinations thereof of any of the above, provided that they retain similar binding properties of the CAR described herein, preferably comprising the corresponding CDRs, or VH and VL regions as described herein. Also mini-antibodies and multivalent antibodies such as diabodies, triabodies, tetravalent antibodies and peptabodies can be used in a method of the invention. The immunoglobulin molecules of the invention can be of any class (i.e. IgG, IgE, IgM, IgD and IgA) or subclass of immunoglobulin molecules. Thus, the term antibody, as used herein, also includes antibodies and antibody fragments comprised by the CAR of the invention, either produced by the modification of whole antibodies or synthesized de novo using recombinant DNA methodologies.

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 110 or more amino acids, primarily responsible for antigen recognition. The terms "variable light chain" and "variable heavy chain" refer to these variable regions of the light and heavy chains respectively. Optionally, the antibody or the immunological portion of the antibody, can be chemically conjugated to, or expressed as, a fusion protein with other proteins.

The CARs of the invention are intended to bind against mammalian, in particular human, protein targets. The use of protein names may correspond to either mouse or human versions of a protein.

Affinities of binding domain polypeptides and CAR proteins according to the present disclosure can be readily determined using conventional techniques, e.g., by competitive ELISA (enzyme-linked immunosorbent assay), or by binding association, or displacement assays using labeled ligands, or using a surface-plasmon resonance device such as the Biacore.

Humanized antibodies comprising one or more CDRs of antibodies of the invention or one or more CDRs derived from said antibodies can be made using any methods known in the art. For example, four general steps may be used to humanize a monoclonal antibody. These are: (1) determining the nucleotide and predicted amino acid sequence of the starting antibody light and heavy variable domains (2) designing the humanized antibody, i.e., deciding which antibody framework region to use during the humanizing process (3) the actual humanizing methodologies/techniques and (4) the transfection and expression of the humanized antibody. See, for example, U.S. Pat. Nos. 4,816,567; 5,807,715; 5,866,692; 6,331,415; 5,530,101; 5,693,761; 5,693,762; 5,585,089; 6,180,370; 5,225,539; 6,548,640.

The term humanized antibody means that at least a portion of the framework regions, and optionally a portion of CDR regions or other regions involved in binding, of an immunoglobulin is derived from or adjusted to human immunoglobulin sequences. The humanized, chimeric or partially humanized versions of the mouse monoclonal antibodies can, for example, be made by means of recombinant DNA technology, departing from the mouse and/or human genomic DNA sequences coding for H and L chains or from cDNA clones coding for H and L chains. Humanized forms of mouse antibodies can be generated by linking the CDR regions of non-human antibodies to human constant regions by recombinant DNA techniques (Queen et al., 1989; WO 90/07861). Alternatively the monoclonal antibodies used in the method of the invention may be human monoclonal antibodies. Human antibodies can be obtained, for example, using phage-display methods (WO 91/17271; WO 92/01047).

As used herein, humanized antibodies refer also to forms of non-human (e.g. murine, camel, llama, shark) antibodies that are specific chimeric immunoglobulins, immunoglobulin chains, or fragments thereof (such as Fv, Fab, Fab', F(ab')₂ or other antigen-binding subsequences of antibodies) that contain minimal sequence derived from non-human immunoglobulin.

As used herein, human or humanized antibody or antibody fragment means an antibody having an amino acid sequence corresponding to that of an antibody produced by a human and/or has been made using any of the techniques for making human antibodies known in the art or disclosed herein. Human antibodies or fragments thereof can be selected by competitive binding experiments, or otherwise, to have the same epitope specificity as a particular mouse antibody. The humanized antibodies of the present invention surprisingly share the useful functional properties of the mouse antibodies to a large extent. Human polyclonal antibodies can also be provided in the form of serum from humans immunized with an immunogenic agent. Optionally, such polyclonal antibodies can be concentrated by affinity purification using amyloid fibrillar and/or non-fibrillar polypeptides or fragments thereof as an affinity reagent. Monoclonal antibodies can be obtained from serum according to the technique described in WO 99/60846.

### Variable Regions and CDRs

A variable region of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies.

There are a number of techniques available for determining CDRs, such as an approach based on cross-species sequence variability (i.e., Kabat et al. Sequences of Proteins of Immunological Interest, (5th ed., 1991, National Institutes of Health, Bethesda Md.)); and an approach based on crystallographic studies of antigen-antibody complexes (Al-Lazikani et al. (1997) J. Molec. Biol. 273:927-948). Alternative approaches include the IMGT international ImMunoGeneTics information system, (Marie-Paule Lefranc). The Kabat definition is based on sequence variability and is the most commonly used method. The Chothia definition is based on the location of the structural loop regions, wherein the AbM definition is a compromise between the two used by Oxford Molecular's AbM antibody modelling software (refer www.bioinf.org.uk : Dr. Andrew C.R. Martin's Group). As used herein, a CDR may refer to CDRs defined by one or more approach, or by a combination of these approaches.

In some embodiments, the invention provides an antibody or fragment thereof incorporated into a CAR, wherein said antibody or fragment thereof comprises at least one CDR, at least two, at least three, or more CDRs that are substantially identical to at least one CDR, at least two, at least three, or more CDRs of the antibody of the invention. Other embodiments include antibodies which have at least two, three, four, five, or six CDR(s) that are substantially identical to at least two, three, four, five or six CDRs of the antibodies of the invention or derived from the antibodies of the invention. In some embodiments, the at least one, two, three, four, five, or six CDR(s) are at least about 70%, 75%, 85%, 86%, 87%, 88%, 89%, 90%, 95%, 96%, 97%, 98%, or 99% identical to at least one, two or three CDRs of the antibody of the invention. It is understood that, for purposes of this invention, binding specificity and/or overall activity is generally retained, although the extent of activity may vary compared to said antibody (may be greater or lesser).

### Additional components of the CAR

In certain embodiments, the CARs contemplated herein may comprise linker residues between the various domains, added for appropriate spacing and conformation of the molecule, for example a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. CARs contemplated herein, may comprise one, two, three, four, or five or more linkers. In particular embodiments, the length of a linker is about 1 to about 25 amino acids, about 5 to about 20 amino acids, or about 10 to about 20 amino acids, or any intervening length of amino acids.

Illustrative examples of linkers include glycine polymers; glycine-serine polymers; glycine-alanine polymers; alanine-serine polymers; and other flexible linkers known in the art, such as the Whitlow linker. Glycine and glycine-serine polymers are relatively unstructured, and therefore may be able to serve as a neutral tether between domains of fusion proteins such as the CARs described herein.

In particular embodiments, the binding domain of the CAR is followed by one or more "spacers" or "spacer polypeptides," which refers to the region that moves the antigen binding domain away from the effector cell surface to enable proper cell/cell contact, antigen binding and activation. In certain embodiments, a spacer domain is a portion of an immunoglobulin, including, but not limited to, one or more heavy chain constant regions, e.g., CH2 and CH3. The spacer domain can include the amino acid sequence of a naturally occurring immunoglobulin hinge region or an altered immunoglobulin hinge region. In one embodiment, the spacer domain comprises the CH2 and CH3 domains of IgG1 or IgG4. In one embodiment the Fc-binding domain of such a spacer/hinge region is mutated in a manner that prevents binding of the CAR to Fc-receptors expressed on macrophages and other innate immune cells.

The binding domain of the CAR may in some embodiments be followed by one or more "hinge domains," which play a role in positioning the antigen binding domain away from the effector cell surface to enable proper cell/cell contact, antigen binding and activation. A CAR may comprise one or more hinge domains between the binding domain and the transmembrane domain (TM). The hinge domain may be derived either from a natural, synthetic, semi-synthetic, or recombinant source. The hinge domain can include the amino acid sequence of a naturally occurring immunoglobulin hinge region or an altered immunoglobulin hinge region. Illustrative hinge domains suitable for use in the CARs described herein include the hinge region derived from the extracellular regions of type 1 membrane proteins such as CD8 alpha, CD4, CD28, PD1, CD 152, and CD7, which may be wild-type hinge regions from these molecules or may be altered. In another embodiment, the hinge domain comprises a PD1, CD 152, or CD8 alpha hinge region.

The "transmembrane domain" is the portion of the CAR that fuses the extracellular binding portion and intracellular signaling domain and anchors the CAR to the plasma membrane of the immune effector cell. The TM domain may be derived either from a natural, synthetic, semi-synthetic, or recombinant source. The TM domain may be derived from the alpha, beta or zeta chain of the T-cell receptor, CD3ε, CD3ζ, CD4, CD5, CD8 alpha, CD9, CD 16, CD22, CD27, CD28, CD33, CD37, CD45, CD64, CD80, CD86, CD 134, CD 137, CD 152, CD 154, and PD1. In one embodiment, the CARs contemplated herein comprise a TM domain derived from CD8 alpha or CD28

In particular embodiments, CARs contemplated herein comprise an intracellular signaling domain. An "intracellular signaling domain," refers to the part of a CAR that participates in transducing the message of effective binding to a human target polypeptide into the interior of the immune effector cell to elicit effector cell function, e.g., activation, cytokine production, proliferation and cytotoxic activity, including the release of cytotoxic factors to the CAR-bound target cell, or other cellular responses elicited with antigen binding to the extracellular CAR domain. The term "effector function" refers to a specialized function of an immune effector cell. Effector function of the T cell, for example, may be cytolytic activity or help or activity including the secretion of a cytokine. Thus, the term "intracellular signaling domain" refers to the portion of a protein which transduces the effector function signal and that directs the cell to perform a specialized function. CARs contemplated herein comprise one or more co-stimulatory signaling domains to enhance the efficacy, expansion and/or memory formation of T cells expressing CAR receptors. As used herein, the term, "co-stimulatory signaling domain" refers to an intracellular signaling domain of a co-stimulatory molecule. Co-stimulatory molecules are cell surface molecules other than antigen receptors or Fc receptors that provide a second signal required for efficient activation and function of T lymphocytes upon binding to antigen.

In one embodiment, the CAR comprises an intracellular domain, which comprises a co-stimulatory domain and a signalling (activation) domain. The CAR construct may therefore include an intracellular signaling domain (CD3 zeta) of the native T cell receptor complex and one or more co-stimulatory domains that provide a second signal to stimulate full T cell activation. Co-stimulatory domains are thought to increase CAR T cell cytokine production and facilitate T cell replication and T cell persistence. Co-stimulatory domains have also been shown to potentially prevent CAR T cell exhaustion, increase T cell antitumor activity, and enhance survival of CAR T cells in patients. As a non-limiting example, CAR constructs with the 4-1BB co-stimulatory domain have been associated with gradual, sustained expansion and effector function, increased persistence, and enriched central memory cells (TCM) in the T cell subset composition in preclinical studies. 4-1BB is a member of the tumor necrosis factor (TNF) superfamily, and it is an inducible glycoprotein receptor in vivo that is primarily expressed on antigen-activated CD4 and CD8 T cells. As a non-limiting example, CD28 is member of the immunoglobulin (Ig) superfamily. It is constitutively expressed on resting and activated CD4 and CD8 T cells and plays a critical role in T cell activation by stimulating the PI3K-AKT signal transduction pathway. In one embodiment, the intracellular domain comprises both 4-1BB and CD28 co-stimulatory domains. Other co-stimulatory domains comprise ICOS and OX40 that can be combined with the CD3 zeta signaling (activation) domain.

### T-Cell Receptors:

The T-cell receptor, or TCR, is a molecule typically found on the surface of T cells, or T lymphocytes that is responsible for recognizing fragments of antigen as peptides bound to major histocompatibility complex (MHC) molecules. The TCR is composed of two different protein chains. In humans, in 95% of T cells the TCR consists of an alpha (α) chain and a beta (β) chain (encoded by TRA and TRB, respectively), whereas in 5% of T cells the TCR consists of gamma and delta (γ/δ) chains (encoded by TRG and TRD, respectively). Each chain is composed of two extracellular domains: Variable (V) region and a Constant (C) region, both of Immunoglobulin superfamily (IgSF) domain forming antiparallel β-sheets. The Constant region is proximal to the cell membrane, followed by a transmembrane region and a short cytoplasmic tail, while the Variable region binds to the peptide/MHC complex.

The variable domain of both the TCR α-chain and β-chain each have three hypervariable or complementarity determining regions (CDRs). There is also an additional area of hypervariability on the β-chain (HV4) that does not normally contact antigen and, therefore, is not considered a CDR.

The residues in these variable domains are located in two regions of the TCR, at the interface of the α- and β-chains and in the β-chain framework region that is thought to be in proximity to the CD3 signal-transduction complex. CDR3 is the main CDR responsible for recognizing processed antigen, although CDR1 of the alpha chain has also been shown to interact with the N-terminal part of the antigenic peptide, whereas CDR1 of the β-chain interacts with the C-terminal part of the peptide.

Recombinant TCRs have been previously transfected into therapeutic T cells intended for the treatment of proliferative disease. For example, TCR-T cells are engineered by transducing preferably autologous alpha-beta or gamma-delta cells with a retroviral or lentiviral vector encoding TCR (typically an alpha chain non-covalently bound with a beta chain) that recognizes peptides of interest and CD3z genes. When the engineered T cells recognize peptides bound to the major histocompatibility complex (MHC) on the surface of antigen-presenting or tumor cells, they become activated and start expanding. The first TCR-T cell therapy was used in clinical trial for metastatic melanoma, whose TCR recognizing an HLA-A2-restricted peptide from a melanocytic differentiation antigen, melanoma antigen recognized by T cells 1 (MART-1).

### Polypeptides

"Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full length protein sequence or a fragment of a full length protein, and may include post-translational modifications of the polypeptide, for example, glycosylations, acetylations, phosphorylations and the like, as well as other modifications known in the art, both naturally occurring and non-naturally occurring.

In various embodiments, the CAR polypeptides contemplated herein comprise a signal (or leader) sequence at the N-terminal end of the protein, which co-translationally or post-translationally directs transfer of the protein. Polypeptides can be prepared using any of a variety of well-known recombinant and/or synthetic techniques. Polypeptides contemplated herein specifically encompass the CARs of the present disclosure, or sequences that have deletions from, additions to, and/or substitutions of one or more amino acid of a CAR as disclosed herein.

An "isolated peptide" or an "isolated polypeptide" and the like, as used herein, refer to in vitro isolation and/or purification of a peptide or polypeptide molecule from a cellular environment, and from association with other components of the cell, i.e., it is not significantly associated with in vivo substances. Similarly, an "isolated cell" refers to a cell that has been obtained from an in vivo tissue or organ and is substantially free of extracellular matrix.

### Nucleic acids

As used herein, the terms "polynucleotide" or "nucleic acid molecule" refers to messenger RNA (mRNA), RNA, genomic RNA (gRNA), plus strand RNA (RNA(+)), minus strand RNA (RNA(-)), genomic DNA (gDNA), complementary DNA (cDNA) or recombinant DNA. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence. In various illustrative embodiments, the present invention contemplates, in part, polynucleotides comprising expression vectors, viral vectors, and transfer plasmids, and compositions, and cells comprising the same.

Polynucleotides can be prepared, manipulated and/or expressed using any of a variety of well-established techniques known and available in the art. In order to express a desired polypeptide, a nucleotide sequence encoding the polypeptide, can be inserted into appropriate vector. Examples of vectors are plasmid, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus {e.g., SV40). Examples of expression vectors are pClneo vectors (Promega) for expression in mammalian cells; pLenti4/V5-DEST^{™}, pLenti6/V5-DEST^{™}, and pLenti6.2/V5-GW/lacZ (Invitrogen) for lentivirus-mediated gene transfer and expression in mammalian cells. In particular embodiments, the coding sequences of the chimeric proteins disclosed herein can be ligated into such expression vectors for the expression of the chimeric protein in mammalian cells. The "control elements" or "regulatory sequences" present in an expression vector are those non-translated regions of the vector - origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, any number of suitable transcription and translation elements, including ubiquitous promoters and inducible promoters may be used.

### Vectors

In particular embodiments, a cell (e.g., an immune effector cell, such as a T cell) is transduced with a retroviral vector, e.g., a lentiviral vector, encoding a CAR. For example, a T cell is transduced with a vector encoding a CAR that comprises a humanized antigen specific domain, antibody or antigen binding fragment that binds a target polypeptide, with a transmembrane and intracellular signaling domain, such that these transduced cells can elicit a CAR-mediated cytotoxic response.

Retroviruses are a common tool for gene delivery. In particular embodiments, a retrovirus is used to deliver a polynucleotide encoding a chimeric antigen receptor (CAR) to a cell. As used herein, the term "retrovirus" refers to an RNA virus that reverse transcribes its genomic RNA into a linear double-stranded DNA copy and subsequently covalently integrates its genomic DNA into a host genome. Once the virus is integrated into the host genome, it is referred to as a "provirus." The provirus serves as a template for RNA polymerase II and directs the expression of RNA molecules which encode the structural proteins and enzymes needed to produce new viral particles.

Illustrative retroviruses suitable for use in particular embodiments, include, but are not limited to: Moloney murine leukemia virus (M-MuLV), Moloney murine sarcoma virus (MoMSV), Harvey murine sarcoma virus (HaMuSV), murine mammary tumor virus (MuMTV), gibbon ape leukemia virus (GaLV), feline leukemia virus (FLV), spumavirus, Friend murine leukemia virus, Murine Stem Cell Virus (MSCV) and Rous Sarcoma Virus (RSV)) and lentivirus.

As used herein, the term "lentivirus" refers to a group (or genus) of complex retroviruses. Illustrative lentiviruses include, but are not limited to: HIV (human immunodeficiency virus; including HIV type 1, and HIV type 2); visna-maedi virus (VMV) virus; the caprine arthritis-encephalitis virus (CAEV); equine infectious anemia virus (EIAV); feline immunodeficiency virus (FIV); bovine immune deficiency virus (BIV); and simian immunodeficiency virus (SIV). In one embodiment, HIV based vector backbones (i.e., HIV cis-acting sequence elements) are preferred. In particular embodiments, a lentivirus is used to deliver a polynucleotide comprising a CAR to a cell.

The term "vector" is used herein to refer to a nucleic acid molecule capable transferring or transporting another nucleic acid molecule. The transferred nucleic acid is generally linked to, e.g., inserted into, the vector nucleic acid molecule. A vector may include sequences that direct autonomous replication in a cell, or may include sequences sufficient to allow integration into host cell DNA. Useful vectors include, for example, plasmids (e.g., DNA plasmids or RNA plasmids, DNA plasmids allowing episomal localization and persistence), transposons, cosmids, bacterial artificial chromosomes, and viral vectors. Useful viral vectors include, e.g., replication defective retroviruses and lentiviruses. In further embodiments of the invention, CRISPR/Cas and TALEN-mediated insertion of the CAR or TCR encoding nucleic acid may be employed. Appropriate vectors for CRISPR/Cas and TALEN-mediated insertion are known to a skilled person.

As will be evident to one of skill in the art, the term "viral vector" is widely used to refer either to a nucleic acid molecule (e.g., a transfer plasmid) that includes virus-derived nucleic acid elements that typically facilitate transfer of the nucleic acid molecule or integration into the genome of a cell or to a viral particle that mediates nucleic acid transfer. Viral particles will typically include various viral components and sometimes also host cell components in addition to nucleic acid(s).

The term viral vector may refer either to a virus or viral particle capable of transferring a nucleic acid into a cell or to the transferred nucleic acid itself. Viral vectors and transfer plasmids contain structural and/or functional genetic elements that are primarily derived from a virus. The term "retroviral vector" refers to a viral vector or plasmid containing structural and functional genetic elements, or portions thereof, that are primarily derived from a retrovirus.

In a preferred embodiment the invention therefore relates to a method for transfecting cells with an expression vector encoding a CAR. For example, in some embodiments, the vector comprises additional sequences, such as sequences that facilitate expression of the CAR, such a promoter, enhancer, poly-A signal, and/or one or more introns. In preferred embodiments, the CAR-coding sequence is flanked by transposon sequences, such that the presence of a transposase allows the coding sequence to integrate into the genome of the transfected cell.

In some embodiments, the genetically transformed cells are further transfected with a transposase that facilitates integration of a CAR coding sequence into the genome of the transfected cells. In some embodiments the transposase is provided as DNA expression vector. However, in preferred embodiments, the transposase is provided as an expressible RNA or a protein such that long-term expression of the transposase does not occur in the transgenic cells. For example, in some embodiments, the transposase is provided as an mRNA (e.g., an mRNA comprising a cap and poly-A tail). Any transposase system may be used in accordance with the embodiments of the present invention. However, in some embodiments, the transposase is salmonid-type Tel -like transposase (SB). For example, the transposase can be the so called "Sleeping beauty" transposase, see e.g., U.S. Patent 6,489,458, incorporated herein by reference. In some embodiments, the transposase is an engineered enzyme with increased enzymatic activity. Some specific examples of transposases include, without limitation, SB 10, SB 11 or SB 100X transposase (see, e.g., Mates et al, 2009, Nat Genet. 41(6):753-61, or US9228180).

For example, a method can involve electroporation of cells with an mRNA encoding an SB 10, SB 11 or SB 100X transposase.

### Sequence Variants:

Sequence variants of the claimed nucleic acids, proteins, antibodies, antibody fragments and/or CARs, for example those defined by % sequence identity, that maintain similar binding properties of the invention are also included in the scope of the invention. Such variants, which show alternative sequences, but maintain essentially the same binding properties, such as target specificity, as the specific sequences provided are known as functional analogues, or as functionally analogous. Sequence identity relates to the percentage of identical nucleotides or amino acids when carrying out a sequence alignment.

The recitation "sequence identity" as used herein refers to the extent that sequences are identical on a nucleotide-by-nucleotide basis or an amino acid-by-amino acid basis over a window of comparison. Thus, a "percentage of sequence identity" may be calculated by comparing two optimally aligned sequences over the window of comparison, determining the number of positions at which the identical nucleic acid base (e.g., A, T, C, G, I) or the identical amino acid residue (e.g., Ala, Pro, Ser, Thr, Gly, Val, Leu, He, Phe, Tyr, Trp, Lys, Arg, His, Asp, Glu, Asn, Gin, Cys and Met) occurs in both sequences to yield the number of matched positions, dividing the number of matched positions by the total number of positions in the window of comparison (i.e., the window size), and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Substitution variants have at least one amino acid residue in the antibody molecule removed and a different residue inserted in its place. The sites of greatest interest for substitutional mutagenesis include the hypervariable regions, but FR alterations are also contemplated. If such substitutions result in a change in biological activity, then more substantial changes, denominated "exemplary substitutions" in the table immediately below, or as further described below in reference to amino acid classes, may be introduced and the products screened.

### Potential Amino Acid Substitutions:

| **Original residue** | **Preferred conservative substitutions** | **Examples of exemplary substitutions** |
|---|---|---|
| Ala (A) | Val | Val; Leu; Ile |
| Asg (R) | Lys | Lys; Gln; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogs; citrulline and methionine sulfoxide which are neutral non-polar analogs, phenylglycine which is an aromatic neutral analog; cysteic acid which is a negatively charged analog and ornithine which is a positively charged amino acid analog. Like the naturally occurring amino acids, this list is not exhaustive, but merely exemplary of the substitutions that are well known in the art.

### Genetically modified cells and T cells

The present invention contemplates, in particular embodiments, T cells genetically modified to express the antigen specific targeting constructs contemplated herein, for use in the treatment of cell proliferation diseases. The T cells of the present invention also comprise CD8+ and CD4+ T cells.

As used herein, the term "genetically engineered" or "genetically modified" refers to the addition of extra genetic material in the form of DNA or RNA into the total genetic material in a cell. The terms, "genetically modified cells," "modified cells," and, "redirected cells," are used interchangeably. As used herein, the term "gene therapy" refers to the introduction--permanently or transiently-- of extra genetic material in the form of DNA or RNA into the total genetic material in a cell that restores, corrects, or modifies expression of a gene, or for the purpose of expressing a therapeutic polypeptide, e.g., a CAR. In particular embodiments, the TCRs or CARs contemplated herein are introduced and expressed in CTLs so as to redirect their specificity to a target antigen of interest.

Immune effector cells, such as CTLs of the invention, can be autologous/autogeneic ("self) or non-autologous ("non-self," e.g., allogeneic, syngeneic or xenogeneic). "Autologous," as used herein, refers to cells from the same subject, and represent a preferred embodiment of the invention. "Allogeneic," as used herein, refers to cells of the same species that differ genetically to the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic.

The terms "T cell" or "T lymphocyte" are art-recognized and are intended to include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)-restricted, natural killer (NK)-like T lymphocytes. A T cell can be a T helper (Th; CD4+ T cell) cell, for example a T helper 1 (Th1) or a T helper 2 (Th2) cell. The T cell can be a cytotoxic T cell (CTL; CD8+ T cell), CD4+CD8+ T cell, CD4 CD8 T cell, or any other subset of T cells. Other illustrative populations of T cells suitable for use in particular embodiments include naive T cells and memory T cells and stem cell-like memory cells (TSCM).

For example, when reintroduced back to patients after autologous cell transplantation, the T cells modified with the constructs of the invention as described herein may recognize and kill tumor cells.

The present invention provides methods for making the CTLs which express the contemplated constructs described herein. In one embodiment, the method comprises transfecting or transducing CTLs isolated from an individual such that the immune effector cells express one or more antigen specific constructs (CAR or TCR) as described herein. In certain embodiments, the CTLs are isolated from an individual and genetically modified without further manipulation in vitro. Such cells can then be directly re-administered into the individual. In further embodiments, the CTLs are first activated and stimulated to proliferate in vitro prior to being genetically modified to express a CAR or TCR, potentially together with the EBAG9 silencing agent. In this regard, the CTLs may be cultured before and/or after being genetically modified.

In particular embodiments, prior to in vitro manipulation or genetic modification of the immune effector cells described herein, the source of cells is obtained from a subject. In particular embodiments, T cells can be obtained from a number of sources including, but not limited to, peripheral blood mononuclear cells, bone marrow, lymph nodes tissue, cord blood, thymus issue, induced pluripotent stem cells (iPSC), tissue from a site of infection, ascites, pleural effusion, spleen tissue, and tumors. In certain embodiments, T cells can be obtained from a unit of blood collected from a subject using any number of techniques known to the skilled person, such as sedimentation, e.g., FICOLL^{™} separation, antibody-conjugated bead-based methods such as MACS^{™} separation (Miltenyi). In one embodiment, cells from the circulating blood of an individual are obtained by apheresis. The apheresis product typically contains lymphocytes, including T cells, monocytes, granulocyte, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or with another suitable solution that lacks calcium, magnesium, and most, if not all other, divalent cations. As would be appreciated by those of ordinary skill in the art, a washing step may be accomplished by methods known to those in the art, such as by using a semiautomated flow through centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T cells are isolated from peripheral blood mononuclear cells (PBMCs) by lysing the red blood cells and depleting the monocytes, for example, by centrifugation through a PERCOLL^{™} gradient. A specific subpopulation of T cells can be further isolated by positive or negative selection techniques. One method for use herein is cell sorting and/or selection via negative magnetic immunoadherence or flow cytometry that uses a cocktail of monoclonal antibodies directed to cell surface markers present on the cells negatively selected.

PBMC may be directly genetically modified to express CARs using methods contemplated herein. In certain embodiments, after isolation of PBMC, T lymphocytes are further isolated and in certain embodiments, both cytotoxic and helper T lymphocytes can be sorted into naive, memory, and effector T cell subpopulations either before or after genetic modification and/or expansion. CD8+ cells can be obtained by using standard methods. In some embodiments, CD8+ cells are further sorted into naive, central memory, and effector cells by identifying cell surface antigens that are associated with each of those types of CD8+ cells.

In some embodiments, the T cells described herein, can be obtained from inducible pluripotent stem cells (iPSCs) using methods known to a skilled person.

Accepted approaches for producing CAR T cells rely on the genetic modification and expansion of mature circulating T-cells. Such processes utilize autologous T cells and reduce risk of graft-versus-host (GvHD) disease from allogeneic T cells through endogenous TCR expression as well as rejection through MHC incompatibility. As an alternative, direct in vitro differentiation of engineered T cells from pluripotent stem cells, such as inducible pluripotent stem cells, provides an essentially unlimited source of cells that can be genetically modified to express the CAR of the present invention. In some embodiments, a so-called master iPSC line can be maintained, which represents a renewable source for consistently and repeatedly manufacturing homogeneous cell products. In some embodiments, the transformation of a master iPSC cell line with the CAR encoding nucleic acid is contemplated, prior to expansion and differentiation to the desired T cell. T lymphocytes can for example be generated from iPSCs, such that iPSCs could be modified with the CAR encoding nucleic acids and subsequently expanded and differentiated to T cells for administration to the patient. Differentiation to the appropriate T cell, could also be conducted from the iPSCs before transformation with CAR encoding nucleic acids and expansion prior to administration. All possible combinations of iPSC expansion, genetic modification and expansion to provide suitable numbers of cells for administration are contemplated in the invention.

The T cells can be genetically modified following isolation using known methods, or the T cells can be activated and expanded (or differentiated in the case of progenitors) in vitro prior to being genetically modified. In a particular embodiment, the T cells are genetically modified with the chimeric antigen receptors contemplated herein (e.g., transduced with a viral vector comprising a nucleic acid encoding a CAR) and then are activated and expanded in vitro. In various embodiments, T cells can be activated and expanded before or after genetic modification to express a CAR, using methods as described, for example, in U.S. Patents 6,352,694; 6,534,055; 6,905,680; 6,692,964; 5,858,358; 6,887,466; 6,905,681; 7, 144,575; 7,067,318; 7, 172,869; 7,232,566; 7, 175,843; 5,883,223; 6,905,874; 6,797,514; 6,867,041; and U.S. Patent Application Publication No. 20060121005.

In a further embodiment, a mixture of, e.g., one, two, three, four, five or more, different expression vectors can be used in genetically modifying a donor population of T cells wherein each vector encodes a different antigen targeting construct as contemplated herein.

In one embodiment, the invention provides a method of storing genetically modified T cells which exhibit EBAG9 inhibition, comprising cryopreserving the T cells such that the cells remain viable upon thawing. A fraction of the immune effector cells can be cryopreserved by methods known in the art to provide a permanent source of such cells for the future treatment of patients afflicted with the condition to be treated. When needed, the cryopreserved cells can be thawed, grown and expanded for more such cells.

### Compositions and Formulations

The compositions contemplated herein may comprise one or more polypeptides, polynucleotides, vectors comprising same, genetically modified T cells, etc., as contemplated herein. Compositions include but are not limited to pharmaceutical compositions. A "pharmaceutical composition" refers to a composition formulated in pharmaceutically-acceptable or physiologically-acceptable solutions for administration to a cell or an animal, either alone, or in combination with one or more other modalities of therapy. It will also be understood that, if desired, the compositions of the invention may be administered in combination with other agents as well, such as, e.g., cytokines, growth factors, hormones, small molecules, chemotherapeutics, pro-drugs, drugs, antibodies, or other various pharmaceutically-active agents. There is virtually no limit to other components that may also be included in the compositions, provided that the additional agents do not adversely affect the ability of the composition to deliver the intended therapy.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals. Exemplary pharmaceutically acceptable carriers include, but are not limited to, to sugars, such as lactose, glucose and sucrose; starches, such as corn starch and potato starch; cellulose, and its derivatives, such as sodium carboxymethyl cellulose, ethyl cellulose and cellulose acetate; tragacanth; malt; gelatin; talc; cocoa butter, waxes, animal and vegetable fats, paraffins, silicones, bentonites, silicic acid, zinc oxide; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and any other compatible substances employed in pharmaceutical formulations.

In particular embodiments, compositions of the present invention comprise an amount of T cells contemplated herein. As used herein, the term "amount" refers to "an amount effective" or "an effective amount" of a genetically modified therapeutic cell, e.g., T cell, to achieve a beneficial or desired prophylactic or therapeutic result, including clinical results.

A "prophylactically effective amount" refers to an amount of a genetically modified therapeutic cell effective to achieve the desired prophylactic result. Typically, but not necessarily, since a prophylactic dose is used in subjects prior to or at an earlier stage of disease, the prophylactically effective amount is less than the therapeutically effective amount. The term prophylactic does not necessarily refer to a complete prohibition or prevention of a particular medical disorder. The term prophylactic also refers to the reduction of risk of a certain medical disorder occurring or worsening in its symptoms.

A "therapeutically effective amount" of a genetically modified therapeutic cell may vary according to factors such as the disease state, age, sex, and weight of the individual, and the ability of the stem and progenitor cells to elicit a desired response in the individual. A therapeutically effective amount is also one in which any toxic or detrimental effects of the virus or transduced therapeutic cells are outweighed by the therapeutically beneficial effects. The term "therapeutically effective amount" includes an amount that is effective to "treat" a subject {e.g., a patient). When a therapeutic amount is indicated, the precise amount of the compositions of the present invention to be administered can be determined by a physician with consideration of individual differences in age, weight, tumor size, extent of infection or metastasis, and condition of the patient (subject).

It can generally be stated that a pharmaceutical composition comprising the T cells described herein may be administered at a dosage of 10² to 10¹⁰ cells/kg body weight, preferably 10⁵ to 10⁷ cells/kg body weight, including all integer values within those ranges. The number of cells will depend upon the ultimate use for which the composition is intended as will the type of cells included therein. For uses provided herein, the cells are generally in a volume of a liter or less, can be 500 mls or less, even 250 mls or 100 mls or less. Hence the density of the desired cells is typically greater than 10⁶ cells/ml and generally is greater than 10⁷ cells/ml, generally 10⁸ cells/ml or greater. The clinically relevant number of cells can be apportioned into multiple infusions that cumulatively equal or exceed 10⁵, 10⁶, 10⁷, 10⁸, 10⁹, 10¹⁰, 10¹¹, or 10¹² cells. In some aspects of the present invention, particularly when all the infused cells are redirected to a particular target antigen, lower numbers of cells may be administered. Cell compositions may be administered multiple times at dosages within these ranges. The cells may be allogeneic, syngeneic, xenogeneic, or autologous to the patient undergoing therapy.

Generally, compositions comprising the cells activated and expanded as described herein may be utilized in the treatment and prevention of diseases that arise in individuals who are immunocompromised. In particular, compositions comprising the modified T cells contemplated herein are used in the treatment of hematological malignancies. The modified T cells of the present invention may be administered either alone, or as a pharmaceutical composition in combination with carriers, diluents, excipients, and/or with other components such as IL-2 or other cytokines or cell populations. In particular embodiments, pharmaceutical compositions contemplated herein comprise an amount of genetically modified T cells, in combination with one or more pharmaceutically or physiologically acceptable carriers, diluents or excipients.

Pharmaceutical compositions of the present invention comprising a T cell may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal or intramuscular administration.

The liquid pharmaceutical compositions, whether they be solutions, suspensions or other like form, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes, multiple dose vials or bags made of glass or plastic. An injectable pharmaceutical composition is preferably sterile.

In a particular embodiment, compositions contemplated herein comprise an effective amount of T cells, alone or in combination with one or more therapeutic agents. Thus, the T cell compositions may be administered alone or in combination with other known cancer treatments, such as radiation therapy, chemotherapy, transplantation, immunotherapy, hormone therapy, photodynamic therapy, etc. The compositions may also be administered in combination with antibiotics. Such therapeutic agents may be accepted in the art as a standard treatment for a particular disease state as described herein, such as a particular cancer. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

The T cell product can be stored frozen in dimethyl sulfoxide (DMSO)/human serum albumin (10% / 90% vol/vol) in the gas phase of liquid nitrogen till the conditioning treatment of the patient has been administered. Such storage does not impede the viability and functionality of the T cell product.

### Therapeutic Methods

The genetically modified cells contemplated herein provide improved methods of adoptive immunotherapy for use in the treatment of medical disorders associated with the presence of unwanted cell proliferation, preferably hematological malignancies.

In particular embodiments, compositions comprising modified T cells contemplated herein are used in the treatment of hematologic malignancies, including but not limited to B cell malignancies such as, for example, non-Hodgkin's lymphoma (NHL), such as B cell NHL or T cell non-Hodgkin's lymphoma, with or without a leukemic tumor cell dissemination.

In further embodiments, the method relates to the treatment of a hematological malignancy, selected from non-Hodgkin's lymphoma (NHL), chronic lymphocytic leukemia, acute myeloid leukemia, acute lymphoblastic leukemia and/or multiple myeloma.

Non-Hodgkin lymphoma encompasses a large group of cancers of lymphocytes (white blood cells). Non-Hodgkin lymphomas can occur at any age and are often marked by lymph nodes that are larger than normal, fever, and weight loss. Non-Hodgkin lymphomas can also present on extranodal sites, such as the central nervous system, mucosal tissues including lung, intestine, colon and gut. There are many different types of non-Hodgkin lymphoma. For example, non-Hodgkin's lymphoma can be divided into aggressive (fast-growing) and indolent (slow-growing) types.

Non-Hodgkin lymphomas can be derived from B cells and T-cells. As used herein, the term "non-Hodgkin lymphoma" includes both "B cell" and "T cell" non-Hodgkin lymphoma. B cell non-Hodgkin lymphomas (NHL) include Burkitt lymphoma, chronic lymphocytic leukemia/small lymphocytic lymphoma (CLL/SLL), diffuse large B cell lymphoma, follicular lymphoma, immunoblastic large cell lymphoma, precursor B-lymphoblastic lymphoma, and mantle cell lymphoma. Lymphomas that occur after bone marrow or stem cell transplantation are usually B cell non-Hodgkin lymphomas.

T-cell lymphomas account for approximately 15 percent of all NHLs in the United States. There are many different forms of T-cell lymphomas, such as angioimmunoblastic T-cell lymphoma (AITL), which is a mature T-cell lymphoma of blood or lymph vessel immunoblasts. Further forms of T cell lymphomas relate to cutaneous T cell lymphoma and T cell lymphoma with a leukemic dissemination.

Chronic lymphocytic leukemia (CLL) can also be treated with the present CAR and is an indolent (slow-growing) cancer that causes a slow increase in immature white blood cells (B lymphocytes). Cancer cells spread through the blood and bone marrow and can also affect the lymph nodes or other organs such as the liver and spleen. CLL eventually causes the bone marrow to fail. A different presentation of the disease is called small lymphocytic lymphoma and localizes mostly to secondary lymphoid organs, e.g. lymph nodes and spleen.

Multiple myeloma is a B cell malignancy of mature plasma cell morphology characterized by the neoplastic transformation of a single clone of these types of cells. These plasma cells proliferate in BM and may invade adjacent bone and sometimes the blood. Variant forms of multiple myeloma include overt multiple myeloma, smoldering multiple myeloma, plasma cell leukemia, non-secretory myeloma, IgD myeloma, osteosclerotic myeloma, solitary plasmacytoma of bone, and extramedullary Plasmacytoma.

Acute myeloid leukemia (AML) is a cancer of the myeloid line of blood cells, characterized by the rapid growth of abnormal cells that build up in the bone marrow and blood and interfere with normal blood cells. A hereditary risk for AML appears to exist. The malignant cell in AML is the myeloblast. In normal hematopoiesis, the myeloblast is an immature precursor of myeloid white blood cells. A normal myeloblast will gradually mature into a mature white blood cell. In AML a single myeloblast accumulates genetic changes which hold the cell in its immature state and prevent differentiation. Such a mutation alone does not cause leukemia although when combined with other mutations, which disrupt genes controlling proliferation, the result is the uncontrolled growth of an immature clone of cells, leading to AML.

Acute lymphoblastic leukemia (ALL) is a cancer of the lymphoid line of blood cells characterized by the development of large numbers of immature lymphocytes. In most cases, the cause is unknown. Genetic risk factors may exist, and environmental risk factors may include radiation exposure or chemotherapy. The cancerous cell in ALL is the lymphoblast. Normal lymphoblasts develop into mature, infection-fighting B-cells or T-cells, also called lymphocytes. In ALL both the normal development of lymphocytes and the control over the number of lymphoid cells become defective.

Further hematological malignancies include essentially any other neoplasm in the blood, in a blood cell or precursor blood cell, such as any given leukemia, lymphoma or myeloma.

The genetically modified cells contemplated herein also provide improved methods of adoptive immunotherapy for use in the treatment of medical disorders associated with the presence of unwanted immune cell proliferation, preferably autoimmune diseases associated with the production of autoantibodies.

In one embodiment of the invention the T cell described herein with EBAG9 silencing is intended for use in the treatment of an autoimmune disease, preferably an auto-antibody-dependent autoimmune disease, preferably an autoimmune disease with an inflammatory component, whereby the autoimmune disease is preferably selected from Takayasu Arteritis, Giant-cell arteritis, familial Mediterranean fever, Kawasaki disease, Polyarteritis nodosa, cutanous Polyarteritis nodosa, Hepatitis-associated arteritis, Behcet's syndrome, Wegener's granulomatosis, ANCA-vasculitidies, Churg-Strauss syndrome, microscopic polyangiitis, Vasculitis of connective tissue diseases, Hennoch-Sch6nlein purpura, Cryoglobulinemic vasculitis, Cutaneous leukocytoclastic angiitis, Tropical aortitis, Sarcoidosis, Cogan's syndrome, Wiskott-Aldrich Syndrome, Lepromatous arteritis, Primary angiitis of the CNS, Thromboangiitis obliterans, Paraneoplastic ateritis, Urticaria, Dego's disease, Myelodysplastic syndrome, Eythema elevatum diutinum, Hyperimmunoglobulin D, Allergic Rhinitis, Asthma bronchiale, chronic obstructive pulmonary disease, periodontitis, Rheumatoid Arthritis, atherosclerosis, Amyloidosis, Morbus Chron, Colitis ulcerosa, Autoimmune Myositis, Diabetes mellitus, Guillain-Barre Syndrome, histiocytosis, Osteoarthritis, atopic dermatitis, periodontitis, chronic rhinosinusitis, Psoriasis, psoriatic arthritis, Microscopic colitis, Pulmonary fibrosis, glomerulonephritis, Whipple's disease, Still's disease, erythema nodosum, otitis, cryoglobulinemia, Sjogren's syndrome, Lupus erythematosus, preferably systemic lupus erythematosus (SLE), aplastic anemia, Osteomyelofibrosis, chronic inflammatory demyelinating polyneuropathy, Kimura's disease, systemic sclerosis, chronic periaortitis, chronic prostatitis, idiopathic pulmonary fibrosis, chronic granulomatous disease, Idiopathic achalasia, bleomycin-induced lung inflammation, cytarabine-induced lung inflammation, Autoimmunthrombocytopenia, Autoimmunneutropenia, Autoimmunhemolytic anemia, Autoimmunlymphocytopenia, Chagas' disease, chronic autoimmune thyroiditis, autoimmune hepatitis, Hashimoto's Thyroiditis, atropic thyroiditis, Graves disease, Autoimmune polyglandular syndrome, Autoimmune Addison Syndrome, Pemphigus vulgaris, Pemphigus foliaceus, Dermatitis herpetiformis, Autoimmune alopecia, Vitiligo, Antiphospholipid syndrome, Myasthenia gravis, Stiff-man syndrome, Goodpasture's syndrome, Sympathetic ophthalmia, Folliculitis, Sharp syndrome and/or Evans syndrome, in particular hay fever, periodontitis, atherosclerosis, rheumatoid arthritis, most preferably SLE.

Systemic lupus erythematosus (SLE), also known as lupus, is an autoimmune disease in which the body's immune system attacks healthy tissue in various parts of the body. Symptoms vary between people and may be mild to severe. Common symptoms include painful and swollen joints, fever, chest pain, hair loss, mouth ulcers, swollen lymph nodes, feeling tired, and a red rash which is most commonly on the face.

As used herein, the terms "individual" and "subject" are often used interchangeably and refer to any animal that exhibits a symptom of a disease, disorder, or condition that can be treated with the gene therapy vectors, cell-based therapeutics, and methods disclosed elsewhere herein. In preferred embodiments, a subject includes any animal that exhibits symptoms of a disease, disorder, or condition of the hematopoietic system, e.g., a B cell malignancy, that can be treated with the cell-based therapeutics and methods disclosed herein. Suitable subjects include laboratory animals (such as mouse, rat, rabbit, or guinea pig), farm animals, and domestic animals or pets (such as a cat or dog). Non-human primates and, preferably, human patients, are included. Typical subjects include human patients that have a cancer hematological malignancy, have been diagnosed with a hematological malignancy, or are at risk or having a hematological malignancy.

As used herein "treatment" or "treating," includes any beneficial or desirable effect on the symptoms or pathology of a disease or pathological condition and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

### FIGURES

The invention is demonstrated by way of example by the following figures. The figures are to be considered as providing a further description of potentially preferred embodiments that enhance the support of one or more non-limiting embodiments of the invention.

### Brief description of the figures:

**Figure 1****:** Generating a retroviral MP71 encoding for an EBAG9-targeting miRNA and GFP.
**Figure 2****:** Decreased human EBAG9 expression in Jurkat cells after transduction with -retroviral vectors encoding for different EBAG9-targeting miRNAs.
**Figure 3****:** Experimental timeline for retroviral transduction of human primary T cells prior to functionally in vitro assays.
**Figure 4****:** EBAG9 downregulation facilitates the antigen-independent release of granyzme A from activated human CD8+ T cells.
**Figure 5****:** The MP71 vector is suitable for the simultaneous expression of an EBAG9-targeting miRNA and a CAR.
**Figure 6****:** BCMA and CD19 CAR expression in transduced primary human T cells.
**Figure 7****:** Antigen-specific cytolytic activity of BCMA CAR T cells can be increased by the downregulation of EBAG9.
**Figure 8****:** Increasing cytolytic activity of CAR T cells by silencing EBAG9 is a universally applicable cell biological mechanism.
**Figure 9****:** In vivo engineered BCMA CAR T cells with silenced EBAG9 eradicate multiple myeloma cells more efficiently.
**Figure 10****:** Target site validation for CRISPR-mediated EBAG9 knockout.

### Detailed description of the figures:

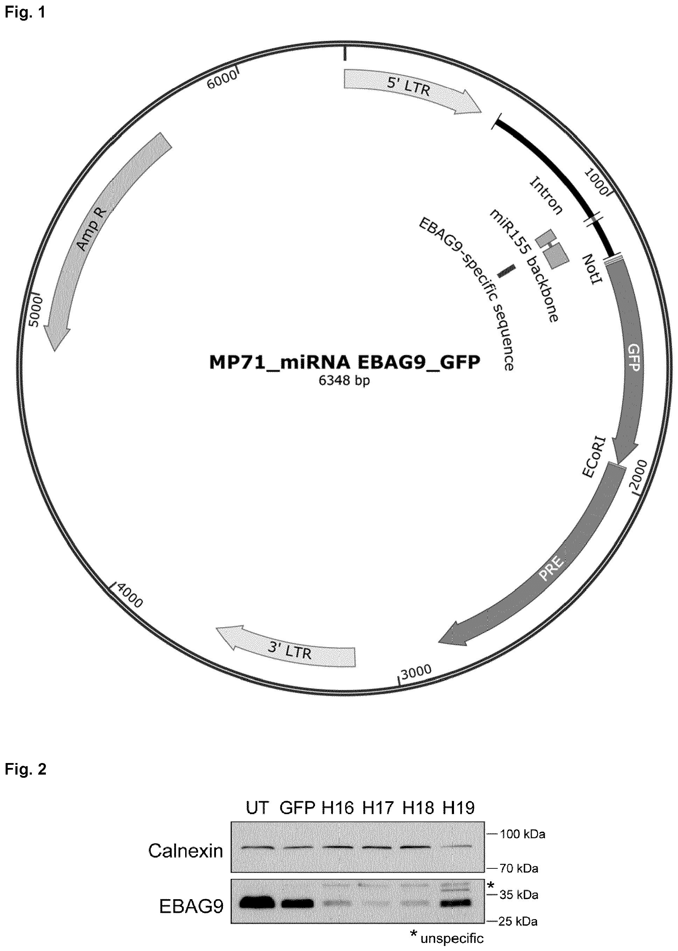

**Figure 1****:** Generating a retroviral MP71 encoding for an EBAG9-targeting miRNA and GFP. EBAG9-targeting miRNAs were generated by exchanging the hairpin antisense sequence of the endogenous miRNA-155 against predicted EBAG9 target site sequences. The resulting miRNA-coding sequences were introduced into a GFP-encoding retroviral MP71 vector at an intronic position. (LTR: long terminal repeat; PRE: post-translational regulatory element; Amp R: ampicillin resistance; GFP: green fluorescence protein).

**Figure 2****:** Decreased human EBAG9 expression in Jurkat cells after transduction with gamma-retroviral vectors encoding for different EBAG9-targeting miRNAs. Retroviral transduction of human Jurkat cells with different GFP-encoding vectors expressing different miRNAs directed against human EBAG9. Positively transduced GFP+ cells were enriched by fluorescence-activated cell sorting (FACS) and analyzed by Western Blot. Calnexin was used as a loading control. Lysates of 1x10e6 Jurkat cells were analyzed. UT, untransduced

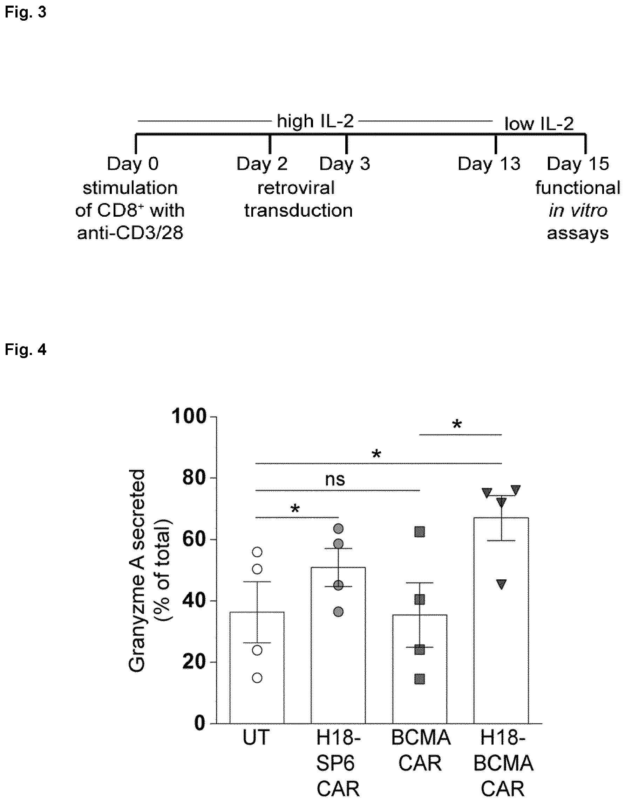

**Figure 3****:** Experimental timeline for retroviral transduction of human primary T cells prior to functionally in vitro assays.

**Figure 4****:** EBAG9 downregulation facilitates the antigen-independent release of granyzme A from activated human CD8+ T cells. Activated human CD8+ T cells were transduced with vectors encoding an SP6 or BCMA CAR in conjunction with the EBAG9-targeting miRNA H18 or the BCMA CAR alone. Enzymatic activities in supernatants were measured on day 15 after CD8+ T cell activation. Granzyme A release was induced by re-stimulation of T cells with anti-human CD3 and anti-CD28 antibodies for 4 h. Values show the release in percentages relative to the total content. Bars represent mean values ± SEM of n=3 experiments with n=4 independent donors per group. *p<0.05, **p<0.01 , *p<0.001 ; ns, not significant. A paired t-test was performed.

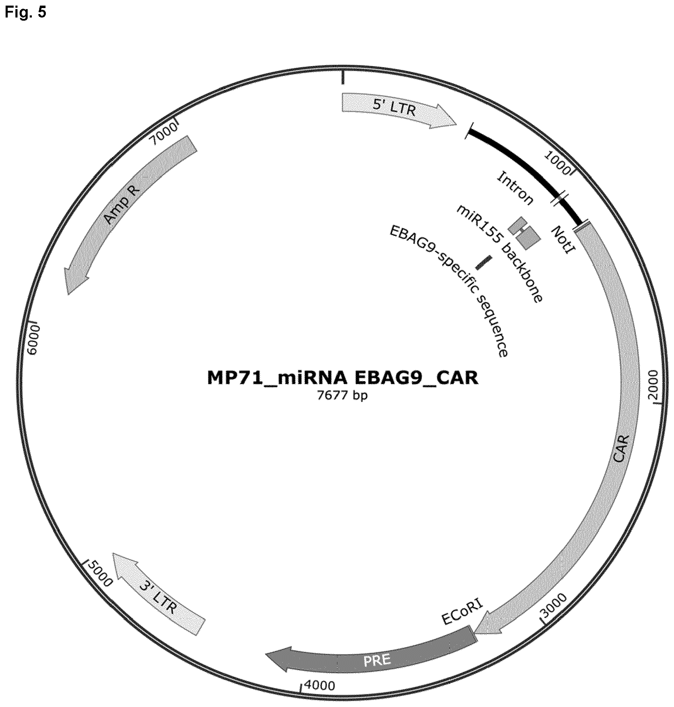

**Figure 5****:** The MP71 vector is suitable for the simultaneous expression of an EBAG9-targeting miRNA and a CAR. Either a SP6, BCMA or CD19 CAR was introduced into a miRNA-containing MP71 vector at the indicated position by using the Notl and EcoRI restriction sites. (LTR - long terminal repeat; PRE - post-translational regulatory element; Amp R - ampicillin resistance; CAR - chimeric antigen receptor)

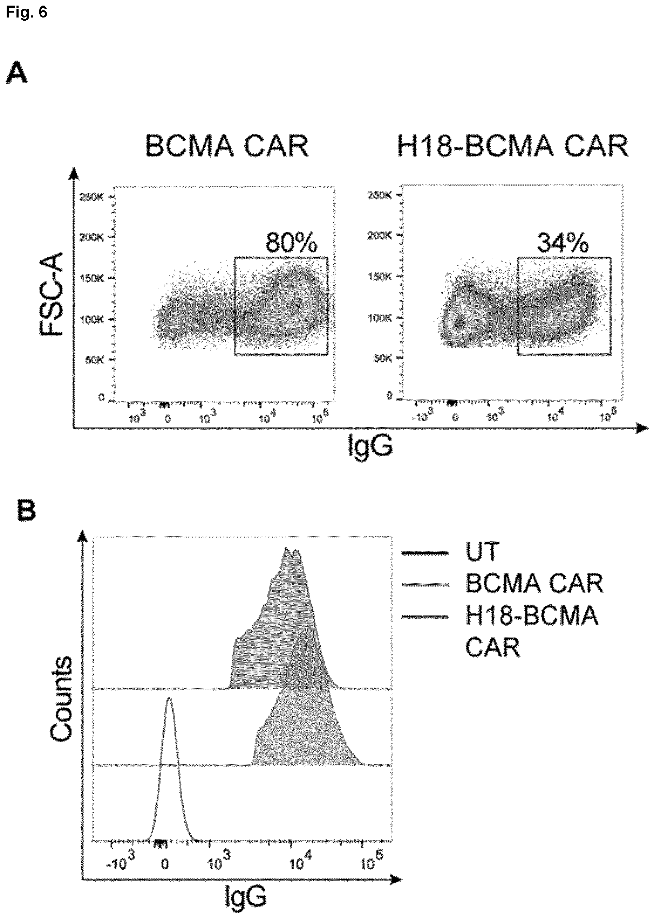

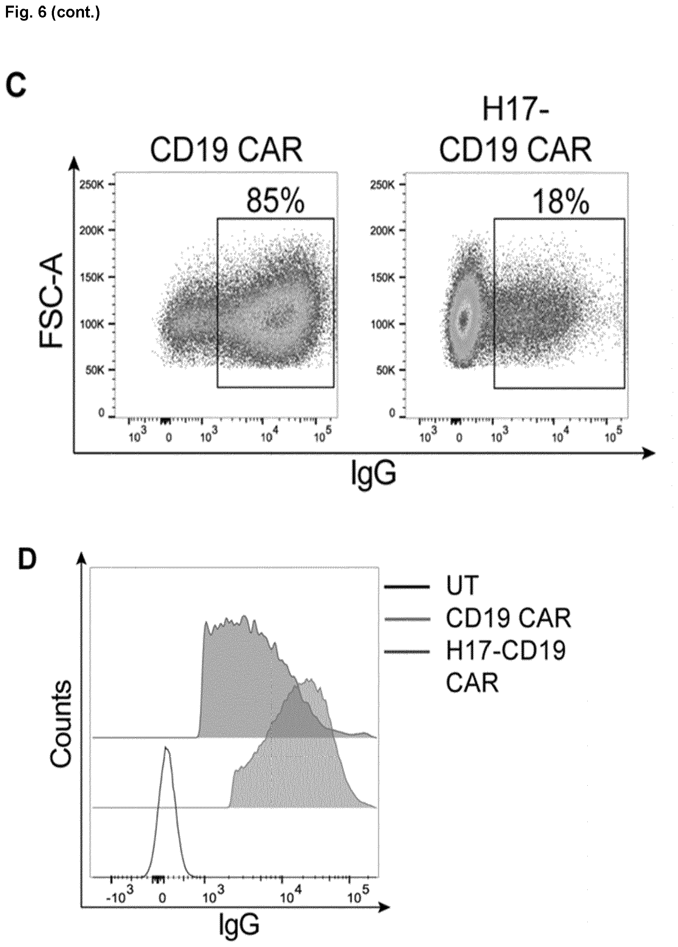

**Figure 6****:** BCMA and CD19 CAR expression in transduced primary human T cells. Primary human T cells were activated by stimulation with anti-human CD3 and anti-human CD28 antibodies for 48 h. Transduction of human T cells with vectors encoding either an BCMA (A-B) or an CD19 (C-D) CAR in conjunction with the EBAG9-targeting miRNA H18 or H17, respectively, was performed twice. Cells were cultured with IL-7/IL-15 supplementation. FACS was performed on day 6 of culture and one representative example per group is shown. Transduction rates are indicated as percentages on the gate.

**Figure 7****:** Antigen-specific cytolytic activity of BCMA CAR T cells can be increased by the downregulation of EBAG9. (A-B) Human CD8+ T cells were activated by stimulation with anti-human CD3 and anti-human CD28 antibodies for 48 h. Transduction of human CD8+ T cells with vectors encoding either an SP6 or BCMA CAR alone or in conjunction with the EBAG9-targeting miRNA H18 was performed twice. Cells were cultured with high IL-2 supplementation for 13 days. Prior to the in vitro cytotoxicity assay, CAR T cells were cultured with low IL-2 supplementation for 48 h. In vitro cytotoxicity assays were performed on day 15 of CAR T cell culture. Transduction rates were adjusted to 20%-30% by addition of UT. [51Cr] chromium-labeled MM (A) and B-NHL (B) cell lines were co-cultured with transduced human T cells at different effector to target ratios for 4 h. Data represent mean ± SEM error bars, n=5 experiments performed in duplicates with 4-8 different donors per group. *p<0,05, **p<0,01, ***p<0,00.1; ns, not significant. A Mann-Whitney U test was employed.

**Figure 8****:** Increasing cytolytic activity of CAR T cells by silencing EBAG9 is a universally applicable cell biological mechanism. Human CD8+ T cells were activated by stimulation with anti-human CD3 and anti-human CD28 antibodies for 48 h. Transduction of human CD8+ T cells with vectors encoding either an SP6 or CD19 CAR alone or in conjunction with the EBAG9-targeting miRNA H17 was performed twice. Cells were cultured with high IL-2 supplementation for 13 days. Prior to the in vitro cytotoxicity assay, CAR T cells were cultured with low IL-2 supplementation for 48 h. In vitro cytotoxicity assays were performed on day 15 of CAR T cell culture. Transduction rates were adjusted to 15% by the addition of UT. [51Cr] chromium-labeled Jeko-1 cell line was co-cultured with transduced human T cells at different effector to target ratios for 4 h. Data represent mean ± SEM error bars n=3 experiments performed in duplicates with 3-6 different donors per group. *p<0,05, **p<0,01, ***p<0,00.1; ns, not significant. A Mann-Whitney U test was employed.

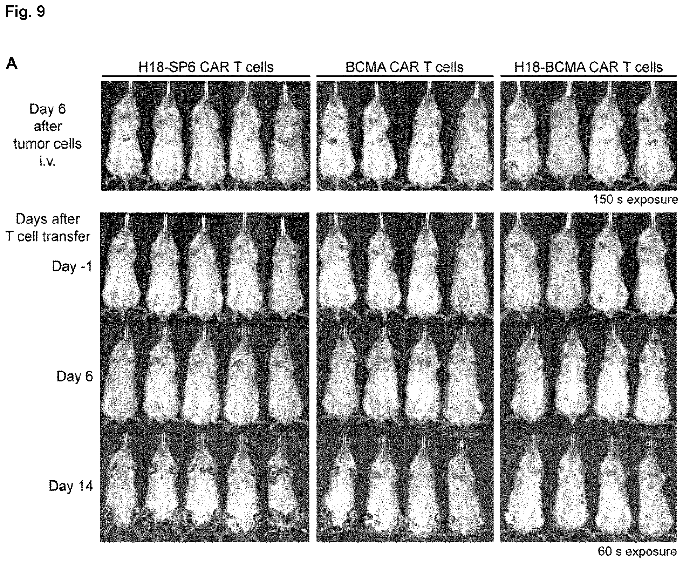

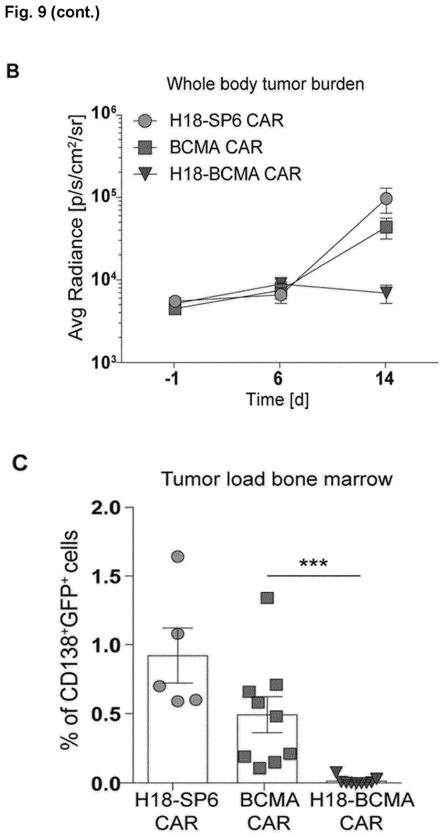

**Figure 9****:** In vivo engineered BCMA CAR T cells with silenced EBAG9 eradicate multiple myeloma cells more efficiently. (A) NSG mice were engrafted with 1×10e7 MM.1S cells stably expressing GFP and a firefly luciferase. On day 6, tumor inoculation was visualized by IVIS with 150 s exposure. One day later, 1x10e6 CAR+ cells (day 10-13 of culture with II-7/IL-15 supplementation) were transferred, and treatment efficiency was observed by IVIS at 60 s exposure. (B) Mean values ± SEM of bioluminescence signal intensities obtained from regions of interest covering the entire body were plotted for each group and timepoint in one experiment. (C) On days 15 and 16, animals were sacrificed and CD138+ GFP+ tumor cells in the bone marrow were quantified by flow cytometry. Mean values ± SEM of n=2 experiments are plotted. *p<0,05, **p<0,01, ***p<0,00.1; ns, not significant. A Mann-Whitney U test was employed.

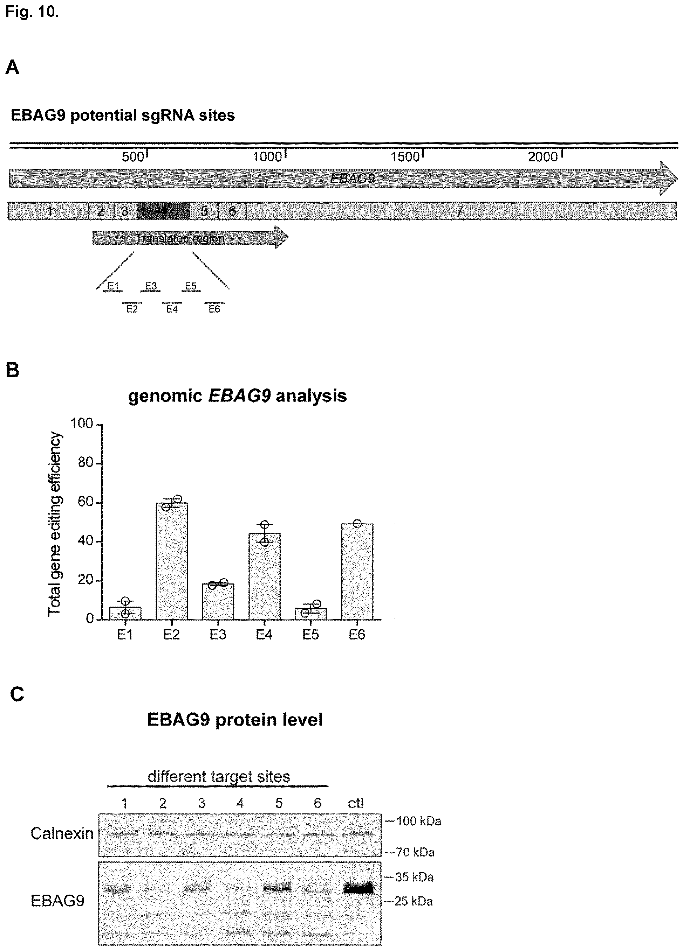

**Figure 10****:** Target site validation for CRISPR-mediated EBAG9 knockout. (A) Schematic overview of the human *EBAG9* gene consisting of 7 exons. Six guide RNAs (E1-E6) were designed targeting different regions in exon 4. (B-C) Primary human T cells were activated by stimulation with human CD3/CD28 Dynabeads for 48 h prior to electroporation with various guide RNAs (E1-E6) and Cas9 protein. At day 9 after activation, genomic DNA and protein samples were taken for analysis of the gene editing efficiency (B) or EBAG9 protein expression level (C), respectively. One representative Western Blot out of three experiments is shown.

### EXAMPLES

The invention is demonstrated by way of the examples disclosed below. The examples provide technical support for a more detailed description of potentially preferred, non-limiting embodiments of the invention.

### Summary of the Examples

1. miRNAs targeted at murine or human EBAG9 were cloned into a retroviral expression vector, followed by transduction of human or murine CD8+ T cells. A knockdown of EBAG9 in human and mouse CD138+ T cells of >90% was achieved, as visualized by Western blot analysis.
2. Transduction of antigen-specific murine T cells (polyclonal) was carried out with γ-retroviruses encoding miRNAs targeted at EBAG9 and subsequently, their adoptive transfer into RAG2-KO mice was accomplished. These mice were challenged with SV40-large T-antigen (Peptide IV) pulsed splenocytes. In an in vivo killing assay, engineered T cells with an EBAG9 knockdown were more efficient in killing than unmodified or control T cells.
3. Human T cells were transduced with γ-retrovirus encoding miRNAs targeted at EBAG9, in combination with various CARs. These cells were used in an in vitro cytotoxicity assay, where target cells expressed the cognate antigens for the CARs chosen. When CAR-positive T cell frequencies were low (<30%), we obtained enhanced cytolysis of either BCMA or CD19 expressing target cells.
4. Human T cells were transduced with γ-retrovirus encoding miRNAs targeted at EBAG9, in combination with an anti-BCMA CAR. NSG mice were transplanted with the multiple myeloma cell line MM.1 S, and tumor onset was measured by IVIS imaging. These mice were then treated with T cells expressing a control CAR, a regular BCMA CAR, and the engineered BCMA CAR that co-expressed a miRNA targeted at human EBAG9. Tumor progression was measured 14 days alter CAR T cell administration. The number of CAR T cells was kept low to better identify the performance of few T cells with enhanced cytolytic strength. Anti-BCMA CAR T cells endowed with a miRNA that silenced EBAG9 were superior in tumor control and led to complete tumor cell eradication from bone marrow.

### Retroviral vector design

### Cloning and miRNA sequences

For silencing of human EBAG9, different miRNAs targeting regions within the open reading frame of the human *EBAG9* gene were generated. Four different target site prediction programs were used for the miRNA design reflecting the requirements of the endogenous RNAi machinery to identify suitable target sites (WlsiRNA, BlockIT, siDESIGN, OligoWalk). The miRNA secondary structure is important for recognition and processing by the RNAi machinery. Characteristic features of the miRNA structure are the rather unstructured backbone and the highly base-paired hairpin that encodes the antisense sequence.

To generate Ebag9-targeting miRNAs, the endogenous miRNA-155 was used. The 21-nucleotide containing antisense sequence within the hairpin structure was exchanged against predicted EBAG9 target site sequences (Table 1).

**Table 1: Hairpin sequences directed against the human EBAG9-gene.**

| miRNA | EBAG9-targeting antisense sequence |
|---|---|
| H17 (SEQ ID NO 1) | 5'- AAATAACCGAAACTGGGTGAT-3' |
| H18 (SEQ ID NO 2) | 5'- TTAAATAACCGAAACTGGGTG-3' |

The resulting miRNA-coding sequences were introduced into a GFP-encoding retroviral MP71 vector at an intronic position using Mlul and Nsil restriction sites. The MP71 vector is known for high transduction efficiency and stable transgene expression in primary T cells. As miRNA transcription is regulated by the polymerase II promoter, the highly active 5' LTR of MP71 can be used to drive miRNA and transgene expression.

### Knockdown efficiency of EBAG9-targeting miRNAs

Efficiency of miRNA-mediated EBAG9 downregulation on the protein level was tested in the human acute T cell leukemia cell line Jurkat J76. Compared to untransduced T cells (UT), an EBAG9 knockdown efficiency of around 80% could be detected for H16, H17, and H18. In contrast, H19 did not effectuate EBAG9 protein downregulation. The MP71-GFP vector without miRNA served as a control. The most efficient miRNAs H17 and H18 were selected for further analysis in primary human T cells.

### Application example:

Human peripheral blood mononuclear cells (PBMCs) were isolated from healthy voluntary donors and CD8+ T cells were enriched by magnetic cell separation (negative selection). CD8+ T cells were activated by stimulation with anti-human CD3 and anti-human CD28 antibodies for 48 hours. Activated human CD8+ T cells were then subjected to two rounds of retroviral transduction and cultured with high IL-2 (100 IU/ml) and IL-15 supplementation (10 ng/ml). On day 13 after CD8+ T cell activation, cytokine supplementation was reduced to 10 IU/ml IL-2 and 1 ng/ml IL-15. Functional assays were performed 48 hours later (Figure 3). To determine the activity of granzyme A, human CAR T cells (day 15 after activation) were restimulated for 4 hours with plate-bound anti-human CD3 and anti-human CD28 antibodies. Supernatants were analyzed for granzyme A activity by incubation with granzyme A substrate solution. Product concentration correlates with enzymatic activity. Supernatants of CAR T cells transferred to non-coated plates were used as a control for the basal secretion of granzyme A.

To prove that EBAG9 silencing increases the release of effector molecules like granzyme A from activated T cells, an in vitro release assay was performed. CD8+ T cells from healthy donors were isolated, transduced twice, and cultivated with high IL-2 supplementation for 13 days. Prior to functional in vitro assays on day 15, IL-2 supplementation was lowered for 48 h. On day 15 of culture, granzyme A release was induced by re-stimulation of T cells with anti-human CD3/CD28 antibodies for 4 h. The granzyme A amount released from BCMA CAR-transduced T cells was similar to those of UT. In contrast, T cells transduced with the H18-BCMA CAR construct released 2-fold higher amounts of granzyme A. Likewise, the H18 miRNA also endowed SP6 CAR T cells with enhanced cytolytic effector molecule secretion (Figure 4).

### Simultaneous expression of EBAG9-targeting miRNAs and CARs

### Cloning

To generate a retroviral vector that allows for the simultaneous expression of an EBAG9-specific miRNA and a CAR, the GFP gene of the miRNA-encoding MP71-GFP vector was exchanged for a CAR cassette using the Notl and EcoRI restriction sites flanking GFP. Using this strategy, a retroviral vector encoding the EBAG9-specific miRNA H18 and the BCMA CAR and a retroviral vector encoding the EBAG9-specific miRNA H17 and the CD19 CAR were cloned (Figure 5). As a negative control for functional assays, the SP6 CAR without any naturally occurring ligand was combined with the EBAG9-targeting miRNAs H17 and H18.

### CAR expression

Retroviral transduction of anti-human CD3/CD28-activated primary human T cells was performed twice using MP71 vectors encoding for the BCMA or the CD19 CAR alone or in conjunction with the EBAG9-specific miRNA H18 or H17, respectively. Cells were cultured with IL-7 and IL-15 supplementation. Staining of the IgG hinge region was performed on day 6 to analyze CAR expression and to determine the transduction efficiency (Figure 6).

### In vitro application example

*In vitro* cytotoxicity assays were performed to investigate the effect of EBAG9 downregulation on the antigen-specific cytolytic capacity of CAR T cells. The *in vitro* cytolytic activity reports on the release of granzymes and perforin, a secretion process that is controlled by EBAG9.

CD8⁺ T cells from healthy donors were isolated, activated with anti-human CD3 and anti-CD28 antibodies for 48 hours, transduced twice, and cultivated with high IL-2 supplementation for 13 days. Prior to *in vitro* cytotoxicity assays on day 15, IL-2 supplementation was lowered for 48 h. On day 15, CAR T cells were co-cultured with target cell lines for 4 hours. Assay supernatants were counted for [⁵¹Cr]-chromium released by lysed target cells. Target cell maximum release was determined by directly counting labeled cells. Spontaneous release was measured by incubating target cells alone.

The BCMA^{high}-expressing multiple myeloma (MM) cell line OPM-2, as well as the BCMA^{low}-expressing B-cell non Hodkin lymphoma (B-NHL) cell line DOHH-2, were used as target cells for BCMA CAR T cells. Prior to co-cultivation with CAR T cells on day 15 of culture, target cells were incubated with [⁵¹Cr]-chromium. After 4 h of co-cultivation with different effector to target ratios, cytolytic activity was observed in BCMA CAR-transduced CD8⁺ T cells, whereas no or little activity could be detected in UT or SP6 CAR T cells. Therefore, no non-specific T cell activation occurred upon EBAG9 downregulation. At the highest effector to target ratio of 80:1, target cell lysis of BCMA CAR T cells was around 30%. The combination of the BCMA CAR with EBAG9 silencing in H18-BCMA CAR T cells led to a significant increase in CAR T cell-mediated cytolytic efficiency in all cell lines tested. For example, in the MM cell line OPM-2, H18-BCMA CAR T cells had a lysis rate approximately 1.5-fold higher than the BCMA CAR only. In a different calculation, the maximal killing rate of BCMA CAR-transduced T cells (E:T 80:1) could be achieved with only one-quarter to one-eighth of EBAG9 knockdown BCMA CAR T cells. Thus, effective dose levels were substantially decreased.

To confirm the RNAi-mediated increase in CAR T cell cytotoxic activity, another miRNA sequence, H17, was used in a CD19 CAR. H17 target the same region within the open reading frame of the EBAG9 gene as H18. As for the BCMA CAR, in vitro cytotoxicity assays were performed. Transduction rates of retrovirally transduced CD8+ T cells were adjusted to around 15% using UT. The CD19^{high}-expressing B-NHL cell line JeKo-1 was used as target cells in a chromium release assay. After 4 h of co-cultivation, almost no lysis activity could be detected for UT and the control H17-SP6 CAR T cells. In Jeko-1 cells, CD19 CAR-transduced T cells effectuated a specific target cell lysis of about 20% (E:T 80:1). Consistent with the previous results, EBAG9 silencing endowed CAR T cells with a substantial gain in killing activity. RNAi-mediated T cell engineering resulted in a cytotoxicity increase of 2-fold. Furthermore, to achieve maximal lysis of JeKo-1 cells by CD19 CAR T cells, only one-fifth to one-eighth of the H17-CD19 CAR T cells were required (Figure 8)

### In vivo application example

To translate the findings of the functional in vitro assays into an in vivo model, a multiple myeloma xenograft model was established. A suitable animal model for the xenotransplantation of human multiple myeloma cell lines and primary human CAR T cells is the immunodeficient NOD scid gamma-chain deficient (NSG) mouse strain. These mice do not have mature T or B cells. In addition, NK cell differentiation is blocked. NSG mice were inoculated with the BCMA-expressing multiple myeloma cell line MM.1S. Tumor progression was monitored by bioluminescence imaging of the MM.1S cell line stably expressing a firefly luciferase-eGFP construct. Tumor cell engraftment was detected by IVIS imaging 6 days after transfer. A single dose of 1×10e6 CAR+ T cells on days 10-13 of culture with IL-7/IL-15 supplementation was injected i.v. one day later. Tumor development was followed by serial IVIS imaging until day 14 after transfer. Mice were sacrificed on days 15-16. Bone marrow was analyzed by flow cytometry for the number of remaining tumor cells and CAR T cells.

Serial IVIS imaging revealed rapid tumor growth between days 6 and 14. The highest specific luciferase signal, which correlates with tumor activity, could be localized to the bone marrow. Treatment with the non-targeting H18-SP6 CAR T cells was unable to control tumor growth. The highest tumor burden was observed in mice from this group. Hence, there was no antigen-independent T cell activation due to EBAG9 silencing and subsequently increased ability of effector molecule release. Mice treated with BCMA CAR T cells showed less tumor progression. However, clinical efficacy at this low number of effector CAR T cells was modest. In contrast, mice that received H18-BCMA CAR T cells showed almost no tumor signal (Figure 9A-B). Accordingly, when analyzing tumor cell numbers (GFP+ CD138+) in bone marrow, the prime niche for myeloma cell homing, tumor cell quantitation revealed 2-fold higher numbers in the H18-SP6 CAR control group compared to the BCMA CAR group. Notably, almost no tumor cells were present in mice treated with RNAi-mediated EBAG9 silencing in BCMA CAR T cells. Altogether, RNAi-mediated downregulation of EBAG9 led to a strongly increased antitumor efficiency even at low effector cell numbers (Fig.9C).

### CRISPR-mediated EBAG9 knockout

In the examples above, EBAG9 downregulation was demonstrated via transducing cells with a retroviral vector that allows for expression of an EBAG9-specific miRNA. Such vectors were also employed for the simultaneous expression of the EBAG9 miRNA together with an antigen-specific CAR construct in transduced T cells. The enhanced cytolytic capacity of CAR T cells with downregulated EBAG9 was demonstrated, both in vitro and in vivo.

To complement the examples above, additional means for downregulating EBAG9 have been assessed. The following example employs CRISPR-mediated EBAG9 knockout, demonstrating that EBAG9 can be effectively removed or reduced from treated T cells via CRISPR, thereby enabling additional means for inhibiting EBAG9 in a cytotoxic T cell.

Different guide RNAs (gRNAs) targeting the exon 4 of the human *EBAG9* gene were generated (E1-E4) in order to knockout EBAG9 in primary human T cells. Human peripheral blood mononuclear cells (PBMCs) were isolated from healthy voluntary donors and CD3⁺ T cells were activated by stimulation with human anti-CD3/CD28 Dynabeads. Activated human CD3⁺ T cells were then subjected to electroporation with gRNAs and Cas9 protein and cultured under IL-2 supplementation. On day 9 after CD3⁺ T cell activation, genomic DNA was isolated and protein lysates were generated. Analysis of gene editing efficiency in the *EBAG9* locus via TIDE analysis (tracking of Indels by decomposition) as well as western blot analysis of EBAG9 protein level revealed a 50% knockout efficiency for the gRNAs E2, E4 and E6. In contrast, gRNAs E1, E3 and E5 show just a minor decrease in the endogenous EBAG9 expression level. CD3⁺ T cells electroporated with a non-targeting gRNA served as control (ctl). Results are shown in Fig. 10. Thus, CRISPR-mediated gene editing can target the human *EBAG9* locus and represents a viable means for EBAG9 inhibition in cytotoxic T cells expressing a transgenic antigen-targeting construct.

*In vitro* cytotoxicity assays to investigate the effect of CRISPR-mediated gene editing of EBAG9 on the antigen-specific cytolytic capacity of CAR T cells are ongoing and are expected to demonstrate an enhanced cytolytic activity of such EBAG9-CRISPR-edited CAR T cells via the release of granzymes and perforin, as shown for miRNA-mediated EBAG9 downregulation, as described above.

## Claims

1. Genetically modified cytotoxic T cell comprising one or more exogenous nucleic acid molecules encoding a transgenic antigen-targeting construct, wherein in said cells estrogen receptor-binding fragment-associated antigen 9 (EBAG9) activity is inhibited.

2. Genetically modified T cell according to the preceding claim, wherein the inhibition of EBAG9 activity is associated with an increase in the release of cytolytic granules and/or granzyme-containing secretory lysosomes (compared to a control cytotoxic T cell).

3. Genetically modified T cell according to any one of the preceding claims, wherein the transgenic antigen-targeting construct is a chimeric antigen receptor (CAR).

4. Genetically modified T cell according to any one of the preceding claims, wherein the transgenic antigen-targeting construct is a T cell receptor (TCR).

5. Genetically modified T cell according to any one of the preceding claims, wherein the inhibition of EBAG9 activity is obtained by knock-down of EBAG9, preferably by RNA interference of EBAG9 expression, such as by small interfering RNA (siRNA), short hairpin RNA (shRNA) and/or micro RNA (miRNA).

6. Genetically modified T cell according to the preceding claim, wherein the inhibition of EBAG9 activity is obtained by genetic modification of the T cell genome with one or more exogenous nucleic acid molecules, said exogenous nucleic acid molecules comprising a vector that encodes the transgenic antigen-targeting construct and an RNA interfering sequence for knock-down of EBAG9.

7. Genetically modified T cell according to any one of the preceding claims, wherein the inhibition of EBAG9 activity is obtained by genetic modification of the T cell genome by disrupting the expression and/or sequence of the EBAG9 gene, wherein preferably an exogenous nucleic acid molecule encoding the transgenic antigen-targeting construct is positioned in the T cell genome within, adjacent or associated with the EBAG9 gene, thereby disrupting the expression and/or sequence of said EBAG9 gene.

8. Genetically modified T cell according to any one of the preceding claims for use as a medicament.

9. Genetically modified T cell according to the preceding claim for use as a medicament in the treatment of a proliferative disease, wherein the antigen targeted by the transgenic antigen-targeting construct is expressed in a target cell undergoing and/or associated with pathologic cell proliferation.

10. Genetically modified T cell for use according to the preceding claim, wherein the proliferative disease is a hematologic malignancy, such as non-Hodgkin lymphoma, chronic lymphocytic leukemia, acute myeloid leukemia, acute lymphoblastic leukemia and/or multiple myeloma, and wherein the antigen targeted by the transgenic antigen-targeting construct is expressed in cancerous cells of said hematologic malignancy, for example a tumor-associated antigen (TAA).

11. Genetically modified T cell for use according to the preceding claim, wherein the hematologic malignancy is a CD19-expressing B-cell cancer, and wherein the transgenic antigen-targeting construct binds CD19, or wherein the hematologic malignancy is a BCMA-expressing B-cell cancer, and wherein the transgenic antigen-targeting construct binds BCMA, or wherein the hematologic malignancy is a CXCR5-expressing cancer, and wherein the transgenic antigen-targeting construct binds CXCR5.

12. Genetically modified T cell according to claim 9 for use as a medicament in the treatment of an autoantibody-dependent autoimmune disease, wherein the antigen targeted by the transgenic antigen-targeting construct is expressed in a target cell associated with autoantibody production, preferably wherein the medical disorder is systemic lupus erythematosus (SLE) or rheumatoid arthritis.

13. Pharmaceutical composition comprising a genetically modified T cell according to any one of the preceding claims suitable for the treatment of a proliferative disease, comprising additionally a pharmaceutically acceptable carrier.

14. A nucleic acid vector or combination of nucleic acid vectors comprising a sequence that encodes an antigen-targeting construct and an RNA interfering sequence for knock-down of estrogen receptor-binding fragment-associated antigen 9 (EBAG9).

15. In vitro method for increasing the cytolytic activity of a genetically modified cytotoxic T cell, said T cell comprising one or more exogenous nucleic acid molecules encoding a transgenic antigen-targeting construct, the method comprising inhibiting in said T cell the activity of estrogen receptor-binding fragment-associated antigen 9 (EBAG9), wherein inhibiting EBAG9 activity preferably comprises:
a. knock-down of EBAG9, preferably by RNA interference of EBAG9 expression, such as by small interfering RNA (siRNA), short hairpin RNA (shRNA) and/or micro RNA (miRNA), or
b. genetic modification of the T cell genome by disrupting the expression and/or sequence of the EBAG9 gene, preferably by CRISPR/Cas9 or TALENs.

## Patentansprüche

1. Genetisch modifizierte zytotoxische T-Zelle, die ein oder mehrere exogene Nukleinsäuremoleküle umfasst, die für ein transgenes Antigen-Targeting-Konstrukt codieren, wobei in den Zellen die EBAG9-Aktivität (Estrogen receptor-Binding fragment-Associated Antigen 9 - EBAG9) gehemmt ist.

2. Genetisch modifizierte T-Zelle nach dem vorhergehenden Anspruch, wobei die Hemmung der EBAG9-Aktivität mit einer Erhöhung der Freisetzung von zytolytischen Granula und/oder Granzym enthaltenden sekretorischen Lysosomen (im Vergleich zu einer zytotoxischen Kontroll-T-Zelle) verbunden ist.

3. Genetisch modifizierte T-Zelle nach einem der vorhergehenden Ansprüche, wobei das transgene Antigen-Targeting-Konstrukt ein chimärer Antigenrezeptor (CAR) ist.

4. Genetisch modifizierte T-Zelle nach einem der vorhergehenden Ansprüche, wobei das transgene Antigen-Targeting-Konstrukt ein T-Zell-Rezeptor (TCR) ist.

5. Genetisch modifizierte T-Zelle nach einem der vorhergehenden Ansprüche, wobei die Hemmung der EBAG9-Aktivität durch Knockdown von EBAG9, vorzugsweise durch RNA-Interferenz der EBAG9-Expression, wie etwa durch Small-Interfering-RNA (siRNA), Short-Hairpin-RNA (shRNA) und/oder Mikro-RNA (miRNA) erreicht wird.

6. Genetisch modifizierte T-Zelle nach dem vorhergehenden Anspruch, wobei die Hemmung der EBAG9-Aktivität durch genetische Modifizierung des T-Zell-Genoms mit einem oder mehreren exogenen Nukleinsäuremolekülen erreicht wird, wobei die exogenen Nukleinsäuremoleküle einen Vektor umfassen, der für das transgene Antigen-Targeting-Konstrukt und eine RNA-Interferenzsequenz zum Knockdown von EBAG9 codiert.

7. Genetisch modifizierte T-Zelle nach einem der vorhergehenden Ansprüche, wobei die Hemmung der EBAG9-Aktivität durch genetische Modifizierung des T-Zell-Genoms durch Störung der Expression und/oder Sequenz des EBAG9-Gens erreicht wird, wobei vorzugsweise ein exogenes Nukleinsäuremolekül, das für das transgene Antigen-Targeting-Konstrukt codiert, im T-Zell-Genom innerhalb des, benachbart zu dem oder assoziiert mit dem EBAG9-Gen(s) positioniert ist, wodurch die Expression und/oder Sequenz des EBAG9-Gens gestört wird.

8. Genetisch modifizierte T-Zelle nach einem der vorhergehenden Ansprüche zur Verwendung als Medikament.

9. Genetisch modifizierte T-Zelle nach dem vorhergehenden Anspruch zur Verwendung als Medikament bei der Behandlung einer proliferativen Erkrankung, wobei das Antigen, auf das das transgene Antigen-Targeting-Konstrukt abzielt, in einer Zielzelle exprimiert wird, die eine pathologische Zellproliferation durchläuft und/oder mit einer solchen assoziiert ist.

10. Genetisch modifizierte T-Zellen zur Verwendung nach dem vorhergehenden Anspruch, wobei die proliferative Erkrankung eine hämatologische Malignität wie etwa Non-Hodgkin-Lymphom, chronische lymphatische Leukämie, akute myeloische Leukämie, akute lymphoblastische Leukämie und/oder multiples Myelom ist und wobei das Antigen, das auf das transgene Antigen-Targeting-Konstrukt abzielt, in Krebszellen der hämatologischen Malignität exprimiert wird, beispielsweise ein Tumor-assoziiertes Antigen (TAA) .

11. Genetisch modifizierte T-Zellen zur Verwendung nach dem vorhergehenden Anspruch, wobei die hämatologische Malignität ein CD19-exprimierender B-Zellkrebs ist und wobei das transgene Antigen-Targeting-Konstrukt CD19 bindet, oder wobei die hämatologische Malignität ein BCMA-exprimierender B-Zellkrebs ist und wobei das transgene Antigen-Targeting-Konstrukt BCMA bindet, oder wobei die hämatologische Malignität ein CXCR5-exprimierender Krebs ist und wobei das transgene Antigen-Targeting-Konstrukt CXCR5 bindet.

12. Genetisch modifizierte T-Zelle nach Anspruch 9 zur Verwendung als Medikament bei der Behandlung einer Autoantikörper-abhängigen Autoimmunerkrankung, wobei das Antigen, auf das das transgene Antigen-Targeting-Konstrukt abzielt, in einer Zielzelle exprimiert wird, die mit der Autoantikörperproduktion verbunden ist, vorzugsweise wobei die medizinische Erkrankung systemischer Lupus erythematodes (SLE) oder rheumatoide Arthritis ist.

13. Pharmazeutische Zusammensetzung, umfassend eine genetisch modifizierte T-Zelle nach einem der vorhergehenden Ansprüche, die für die Behandlung einer proliferativen Erkrankung geeignet ist, zusätzlich umfassend einen pharmazeutisch verträglichen Träger.

14. Nukleinsäurevektor oder eine Kombination von Nukleinsäurevektoren, umfassend eine Sequenz, die für ein Antigen-Targeting-Konstrukt und eine RNA-Interferenzsequenz für den Knockdown von EBAG9 (Estrogen receptor-Binding fragment-Associated Antigen 9 - EBAG9) codiert.

15. In-vitro-Verfahren zur Steigerung der zytolytischen Aktivität einer genetisch modifizierten zytotoxischen T-Zelle, wobei die T-Zelle ein oder mehrere exogene Nukleinsäuremoleküle umfasst, die für ein transgenes Antigen-Targeting-Konstrukt codieren, wobei das Verfahren Hemmen der EBAG9-Aktivität (Estrogen receptor-Binding fragment-Associated Antigen 9 - EBAG9) umfasst, wobei das Hemmen der EBAG9-Aktivität vorzugsweise Folgendes umfasst:
A. Knockdown von EBAG9, vorzugsweise durch RNA-Interferenz der EBAG9-Expression, wie etwa durch Small-Interfering-RNA (siRNA), Short-Hairpin-RNA (shRNA) und/oder Mikro-RNA (miRNA), oder
B. genetische Modifizierung des T-Zell-Genoms durch Störung der Expression und/oder Sequenz des EBAG9-Gens, vorzugsweise durch CRISPR/Cas9 oder TALENs.

## Revendications

1. Lymphocyte T cytotoxique génétiquement modifié comprenant une ou plusieurs molécules d'acide nucléique exogènes codant une construction de ciblage d'antigène transgénique, dans lequel, dans lesdits lymphocytes, l'activité de l'antigène 9 associé au fragment de liaison au récepteur d'oestrogène (EBAG9) est inhibée.

2. Lymphocyte T génétiquement modifié selon la revendication précédente, dans lequel l'inhibition de l'activité d'EBAG9 est associée à une augmentation de la libération de granules cytolytiques et/ou de lysosomes sécrétoires contenant des granzymes (par rapport au lymphocyte T cytotoxique témoin).

3. Lymphocyte T génétiquement modifié selon l'une quelconque des revendications précédentes, dans lequel la construction de ciblage d'antigène transgénique est un récepteur d'antigène chimérique (CAR).

4. Lymphocyte T génétiquement modifié selon l'une quelconque des revendications précédentes, dans lequel la construction de ciblage d'antigène transgénique est un récepteur de lymphocyte T (TCR).

5. Lymphocyte T génétiquement modifié selon l'une quelconque des revendications précédentes, dans lequel l'inhibition de l'activité d'EBAG9 est obtenue par l'inactivation d'EBAG9, de préférence par interférence d'ARN de l'expression d'EBAG9, comme par exemple par un petit ARN interférent (siARN), un ARN en épingle à cheveux court (shARN) et/ou un micro ARN (miARN).

6. Lymphocyte T génétiquement modifié selon la revendication précédente, dans lequel l'inhibition de l'activité d'EBAG9 est obtenue par modification génétique du génome du lymphocyte T avec une ou plusieurs molécules d'acide nucléique exogènes, lesdites molécules d'acide nucléique exogènes comprenant un vecteur codant pour la construction de ciblage d'antigène transgénique et une séquence interférente d'ARN pour l'inactivation d'EBAG9.

7. Lymphocyte T génétiquement modifié selon l'une quelconque des revendications précédentes, dans lequel l'inhibition de l'activité d'EBAG9 est obtenue par modification génétique du génome du lymphocyte T en perturbant l'expression et/ou la séquence du gène d'EBAG9, dans lequel de préférence une molécule d'acide nucléique exogène codant pour la construction de ciblage d'antigène transgénique est positionnée dans le génome des lymphocytes T à l'intérieur, adjacent ou associé au gène d'EBAG9, perturbant ainsi l'expression et/ou la séquence dudit gène d'EBAG9.

8. Lymphocyte T génétiquement modifié selon l'une quelconque des revendications précédentes, destiné à être utilisé comme médicament.

9. Lymphocyte T génétiquement modifié selon la revendication précédente destiné à être utilisé comme médicament dans le traitement d'une maladie proliférative, dans lequel l'antigène ciblé par la construction de ciblage d'antigène transgénique est exprimé dans une cellule cible subissant et/ou associée à une prolifération cellulaire pathologique.

10. Lymphocyte T génétiquement modifié destiné à être utilisé selon la revendication précédente, dans lequel la maladie proliférative est une hémopathie maligne, telle qu'un lymphome non hodgkinien, une leucémie lymphoïde chronique, une leucémie myéloïde aiguë, une leucémie lymphoblastique aiguë et/ou un myélome multiple, et dans lequel l'antigène ciblé par la construction de ciblage d'antigène transgénique est exprimé dans des cellules cancéreuses de ladite hémopathie maligne, par exemple un antigène associé à une tumeur (TAA).

11. Lymphocyte T génétiquement modifié destiné à être utilisé selon la revendication précédente, dans lequel l'hémopathie maligne est un cancer à cellules B exprimant CD19, et dans lequel la construction de ciblage d'antigène transgénique se lie à CD19, ou dans lequel l'hémopathie maligne est un cancer à cellules B exprimant BCMA, et dans lequel la construction de ciblage d'antigène transgénique se lie au BCMA, ou dans lequel l'hémopathie maligne est un cancer exprimant CXCR5, et dans lequel la construction de ciblage d'antigène transgénique se lie à CXCR5.

12. Lymphocyte T génétiquement modifié selon la revendication 9 destiné à être utilisé comme médicament dans le traitement d'une maladie auto-immune dépendante des auto-anticorps, dans lequel l'antigène ciblé par la construction de ciblage d'antigène transgénique est exprimé dans une cellule cible associée à la production d'auto-anticorps, de préférence dans lequel le trouble médical est le lupus érythémateux systémique (SLE) ou la polyarthrite rhumatoïde.

13. Composition pharmaceutique comprenant un lymphocyte T génétiquement modifié selon l'une quelconque des revendications précédentes, adaptée au traitement d'une maladie proliférative, comprenant par ailleurs un support pharmaceutiquement acceptable.

14. Vecteur d'acide nucléique ou combinaison de vecteurs d'acide nucléique comprenant une séquence qui code pour une construction de ciblage d'antigène et une séquence interférant avec l'ARN pour l'inactivation de l'antigène 9 associé au fragment de liaison au récepteur d'oestrogène (EBAG9).

15. Procédé in vitro pour augmenter l'activité cytolytique d'un lymphocyte T cytotoxique génétiquement modifié, ledit lymphocyte T comprenant une ou plusieurs molécules d'acide nucléique exogènes codant pour une construction de ciblage d'antigène transgénique, le procédé comprenant l'inhibition dans ledit lymphocyte T de l'activité de l'antigène 9 associé au fragment de liaison au récepteur d'oestrogène (EBAG9), dans lequel l'inhibition de l'activité d'EBAG9 comprend de préférence :
a. l'inactivation d'EBAG9, de préférence par interférence d'ARN sur l'expression d'EBAG9, par exemple par un petit ARN interférent (siARN), un ARN en épingle à cheveux court (shARN) et/ou un micro-ARN (miARN), ou
b. la modification génétique du génome des lymphocytes T en perturbant l'expression et/ou la séquence du gène d'EBAG9, de préférence par CRISPR/Cas9 ou TALENs.
